# EUROPEAN PATENT APPLICATION

(11) **EP 4 729 075 A1**
(43) Date of publication of application: **22.04.2026**
(21) Application number: 24822771.2
(22) Date of filing: 14.06.2024
(51) Int. Cl.: A61K 48/00, C12N 9/22, C12N 15/113

(54) **EPIGENETIC EDITING TOOL FOR TARGETING HEPATITIS B VIRUS GENE**

(30) Priority: 16.06.2023 CN 202310720942; 06.12.2023 CN 202311667186
(71) Applicant: Epigenic Therapeutics Pte. Ltd., Singapore 068908 (SG)
(72) Inventor: PENG, Wenbo, Shanghai 200131 (CN); ZHAO, Junzheng, Shanghai 200131 (CN); MAO, Shaoshuai, Shanghai 200131 (CN); WU, Leilei, Shanghai 200131 (CN); SUN, Di, Shanghai 200131 (CN); LV, Ruimin, Shanghai 200131 (CN); ZANG, Ying, Shanghai 200131 (CN); LIU, Junjian, Shanghai 200131 (CN)
(74) Representative: Weickmann & Weickmann PartmbB
(86) International application number: PCT/CN2024/099136
(87) International publication number: WO 2024/255823

(57) **Abstract**

The present application relates to the field of biomedicine, and provides an epigenetic editing tool for targeting a hepatitis B virus gene and a use thereof.

## Description

### TECHNICAL FIELD

The present application relates to the field of biomedicine, and in particular to a target-specific epigenetic editing tool and a use thereof.

### BACKGROUND

Hepatitis B virus (HBV) is a hepatotropic DNA virus. The chronic hepatitis B infection caused by HBV can lead to lasting liver inflammation, thus significantly increasing the probability of liver cirrhosis and liver cancer. According to the World Health Organization (WTO) estimates, there are currently more than 250 million cases of chronic hepatitis B caused by Hepatitis B virus worldwide, of which more than 800,000 patients die every year, causing a huge burden on global public health. As a country with a large population, China accounts for about one-third of the world's hepatitis B carriers. Since the 1980s, with the increase of hepatitis B vaccination rate, new hepatitis B cases in China have been effectively controlled, but there is still a huge number of hepatitis B infected people in China.

At present, treatments for HBV, such as nucleoside (acid) analogs and IFN, can effectively inhibit virus replication, but it is difficult to achieve a functional cure, that is, reducing surface antigen (HBsAg) to an undetectable level, and long-term medication is required. Once treatment is stopped, HBV replication will rebound. The main reason for this phenomenon is that covalently closed circular DNA (cccDNA) will be formed after HBV infected hepatocytes, and the HBV genome can be integrated into the hepatocyte genome of the host. cccDNA and integrated DNA can serve as templates for viral replication and protein expression in a long-term and stable manner and can not eliminated via current conventional treatments. Therefore, eliminating or silencing cccDNA and integrated DNA may effectively achieve the treatment of hepatitis B infection. Since cccDNA can assemble with histones to form a chromosome-like structure in the host liver cell nucleus, its transcriptional regulation can also be affected by epigenetic modifications. There are three CpG islands in the HBV genome, hereinafter referred to as CG I, CG II and CG III, respectively, and CpG islands are key regions for achieving epigenetic regulation. The transcription of the HBV genome is regulated by four promoters (Xp, Cp, Sp1, Sp2) and two enhancers (Enh I and Enh II). Among them, Xp, Cp, Enh I and Enh II are located in the CG II region, and Sp1 and Sp2 are located in the vicinity of CG I and CG III. Therefore, epigenetic editing of CpG islands may affect multiple regulatory elements, thereby affecting the transcription of the viral genome.

Introducing epigenetic modifications into specific regulatory regions or specific sites of the HBV genome can change the chromatin structure, thereby adjusting the target gene to a transcriptional repression state and realizing the silent regulation of the target gene. In this process, DNA will not be cleavaged, avoiding the possibility of double-strand breaks in the genome, and fundamentally eliminating the risk of activating unpredictable DNA repair mechanisms and potentially producing immunogenic truncated or mutant proteins. However, at present, most of the research on epigenetic editing tools that specifically target the HBV genome is still in the early stage, and the development of epigenetic editing technologies that can sustainably cure or eliminate HBV virus still faces many challenges such as unknowns and limitations.

### SUMMARY

The present application aims to develop a safe and effective therapy for silent regulation of HBV target genes, and provides a method for introducing an inhibitory epigenetic modification into a specific regulatory region of HBV genome by an epigenetic editing tool, thereby changing the transcription activity of hepatitis B virus cccDNA and integrated genomic DNA, realizing the reduction of HBV replication and protein expression, and achieving the purpose of treating hepatitis B.

In one aspect, the present application provides a composition comprising: (1) a fusion molecule or a nucleic acid sequence encoding the fusion molecule, wherein the fusion molecule comprises at least one DNA binding protein and at least one gene expression modulator, and (2) at least one single guide RNA (sgRNA), or a nucleic acid sequence encoding the sgRNA, wherein the sgRNA is complementary to a target DNA sequence in the vicinity of a hepatitis B virus (HBV) gene and/or within a HBV gene regulatory element, and the HBV gene is a type B HBV gene comprising a nucleotide sequence as set forth in SEQ ID NO: 1, a type C HBV gene comprising a nucleotide sequence as set forth in SEQ ID NO: 2, or a type D HBV gene comprising a nucleotide sequence as set forth in SEQ ID NO: 3; the HBV gene regulatory element comprises a transcription initiation site, a core promoter, a promoter, an enhancer, a silencer, an insulator element, a boundary element, and/or a locus control region, and the target DNA sequence is located between nucleotide at position 1056 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

In certain embodiments, the target DNA sequence is located between nucleotide at position 1056 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

In certain embodiments, the target DNA sequence is located between nucleotide at position 1060 and nucleotide at position 1079, between nucleotide at position 1149 and nucleotide at position 1612, between nucleotide at position 1693 and nucleotide at position 1852, and/or between nucleotide at position 2863 and nucleotide at position 2882 of the HBV gene.

In certain embodiments, the target DNA sequence is located in one or more of following regions of the type B HBV gene: between nucleotide at position 1149 and nucleotide at position 1190, between nucleotide at position 1210 and nucleotide at position 1310, between nucleotide at position 1350 and nucleotide at position 1400, between nucleotide at position 1420 and nucleotide at position 1450, and between nucleotide at position 1470 and nucleotide at position 1592.

In certain embodiments, the target DNA sequence is located in one or more of following regions of the type C HBV gene: between nucleotide at position 1150 and nucleotide at position 1180, between nucleotide at position 1200 and nucleotide at position 1310, between nucleotide at position 1350 and nucleotide at position 1390, between nucleotide at position 1420 and nucleotide at position 1460, and between nucleotide at position 1480 and nucleotide at position 1593.

In certain embodiments, the target DNA sequence is located in one or more of following regions of the type D HBV gene: between nucleotide at position 1056 and nucleotide at position 1079, between nucleotide at position 1101 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2882, between nucleotide at position 2911 and nucleotide at position 2930, and between nucleotide at position 3048 and nucleotide at position 3067.

In certain embodiments, the target DNA sequence is located in one or more of following regions of the type D HBV gene: between nucleotide at position 1060 and nucleotide at position 1079, between nucleotide at position 1160 and nucleotide at position 1310, between nucleotide at position 1253 and nucleotide at position 1284, between nucleotide at position 1370 and nucleotide at position 1470, between nucleotide at position 1490 and nucleotide at position 1612, between nucleotide at position 1693 and nucleotide at position 1852, and between nucleotide at position 2863 and nucleotide at position 2882.

For example, the sequence of the complete genome of type B HBV (serotype adw) is shown below:

CTCCACCACTTTCCACCAAACTCTTCAAGATCCCAGAGTCAGGGCCCTGTACTTTC
CTGCTGGTGGCTCCAGTTCAGGAACAGTGAGCCCTGCTCAGAATACTGTCTCTGCCAT
ATCGTCAATCTTATCGAAGACTGGGGACCCTGTACCGAACATGGAGAACATCGCATCA
GGACTCCTAGGACCCCTGCTCGTGTTACAGGCGGGGTTTTTCTTGTTGACAAAAATCC
TCACAATACCACAGAGTCTAGACTCGTGGTGGACTTCTCTCAATTTTCTAGGGGGAAC
ACCCGTGTGTCTTGGCCAAAATTCGCAGTCCCAAATCTCCAGTCACTCACCAACCTGT
TGTCCTCCAATTTGTCCTGGTTATCGCTGGATGTGTCTGCGGCGTTTTATCATCTTCCTC
TGCATCCTGCTGCTATGCCTCATCTTCTTGTTGGTTCTTCTGGACTATCAAGGTATGTTG
CCCGTTTGTCCTCTAATTCCAGGATCATCAACAACCAGCACCGGACCATGCAAAACCT
GCACAACTCCTGCTCAAGGAACCTCTATGTTTCCCTCATGTTGCTGTACAAAACCTAC
GGACGGAAACTGCACCTGTATTCCCATCCCATCATCTTGGGCTTTCGCAAAATACCTAT
GGGAGTGGGCCTCAGTCCGTTTCTCTTGGCTCAGTTTACTAGTGCCATTTGTTCAGTG
GTTCGTAGGGCTTTCCCCCACTGTCTGGCTTTCAGTTATATGGATGATGTGGTTTTGGG
GGCCAAGTCTGTACAACATCTTGAGTCCCTTTATGCCGCTGTTACCAATTTTCTTTTGT
CTTTGGGTATACATTTAAACCCTCACAAAACAAAAAGATGGGGATATTCCCTTAACTTC
ATGGGATATGTAATTGGGAGTTGGGGCACATTGCCACAGGAACATATTGTACAAAAAA
TCAAAATGTGTTTTAGGAAACTTCCTGTAAACAGGCCTATTGATTGGAAAGTATGTCA
ACGAATTGTGGGTCTTTTGGGGTTTGCCGCCCCTTTCACGCAATGTGGATATCCTGCTT
TAATGCCTTTATATGCATGTATACAAGCGAAACAGGCTTTTACTTTCTCGCCAACTTAC
AAGGCCTTTCTAAGTAAACAGTATCTGAACCTTTACCCCGTTGCTCGGCAACGGCCTG
GTCTGTGCCAAGTGTTTGCTGACGCAACCCCCACTGGTTGGGGCTTGGCCATAGGCC
ATCAGCGCATGCGTGGAACCTTTGTGTCTCCTCTACCGATCCATACTGCGGAACTCCTA
GCCGCTTGTTTTGCTCGCAGCAGGTCTGGGGCAAACCTCATCGGGACTGACAATTCTG
TCGTGCTCTCCCGCAAGTATACATCATTTCCATGGCTGCTAGGCTGTGCTGCCAACTGG
ATCCTGCGCGGGACGTCCTTTGTTTACGTCCCGTCGGCGCTGAATCCCGCGGACGACC
CCTCTCGGGGCCGCTTGGGACTCTACCGCCCGCTTCTCCGCCTATTGTACCGGCCGAC

CACGGGGCGCACCTCTCTTTACGCGGACTCCCCGTCTGTGCCTTCTCATCTGCCGGAC
CGTGTGCACTTCGCTTCACCTCTGCACGTCGCATGGAGACCACCGTGAACGCCCACA
GGAACCTGCCCAAGGTCTTGCATAAGAGGACTCTTGGACTTTCAGCAATGTCAACGA
CCGACCTTGAGGCATACTTCAAAGACTGTGTGTTTACTGAGTGGGAGGAGTTGGGGG
AGGAGGTTAGGTTAAAGGTCTTTGTACTAGGAGGCTGTAGGCATAAATTGGTGTGTTC
ACCAGCACCATGCAACTTTTTCACCTCTGCCTAATCATCTCATGTTCATGTCCTACTGT
TCAAGCCTCCAAGCTGTGCCTTGGGTGGCTTTGGGGCATGGACATTGACCCGTATAAA
GAATTTGGAGCTTCTGTGGAGTTACTCTCTTTTTTGCCTTCTGACTTCTTTCCTTCTATT
CGAGATCTCCTCGACACCGCCTCTGCTCTGTATCGGGAGGCCTTAGAGTCTCCGGAAC
ATTGTTCACCTCACCATACGGCACTCAGGCAAGCTATCCTGTGTTGGGGTGAGTTGAT
GAATCTAGCTACCTGGGTGGGAAGTAATTTGGAAGATCCAGCATCCAGGGAATTAGTA
GTCAGCTATGTCAACGTTAACATGGGCCTAAAAATCAGACAACTCTTGTGGTTTCACA
TTTCCTGTCTTACTTTTGGGAGAGAAACTGTCCTTGAATATTTGGTGTCTTTTGGAGTG
TGGATTCGCACTCCTCCTGCATATAGACCACCAAATGCCCCTATCTTATCAACTCTTCC
GGAAACTACTGTTGTTAGACGACGAGGCAGGTCCCCTAGAAGAAGAACTCCCTCGCC
TCGCAGACGAAGGTCTCAATCGCCGCGTCGCAGAAGATATCAATCTCGGGAATCTCA
ATGTTAGTATTCCTTGGACACATAAGGTGGGAAACTTTACGGGGCTTTATTCTTCTACG
GTACCTTGCTTTAATCCTAAATGGCAAACTCCTTCTTTTCCTGACATTCATTTGCAGGA
GGACATTGTTGATAGATGTAAGCAATTTGTGGGGCCCCTTACAGTAAATGAAAACAGG
AGACTGAAATTAATTATGCCTGCTAGGTTTTATCCCAATGTTACTAAATATTTGCCCTTA
GATAAAGGGATCAAACCGTATTATCCAGAGTATGTAGTTAATCATTACTTCCAGACGCG
ACATTATTTACACACTCTTTGGAAGGCGGGGATCTTATATAAAAGAGAGTCCACACGT
AGCGCCTCATTTTGCGGGTCACCATATTCTTGGGAACAAGATCTACAGCATGGGAGGT
TGGTCTTCCAAACCTCGAAAAGGCATGGGGACAAATCTTTCTGTCCCCAATCCCCTGG
GATTCTTCCCCGATCATCAGTTGGACCCTGCATTCAAAGCCAACTCAGAAAGTCCAGA
TTGGGACCTCAACCCGCACAAGGACAACTGGCCGGACGCCAACAAGGTGGGAGTGG

GAGCATTCGGGCCAGGGTTCACCCCTCCCCATGGGGGACTGTTGGGGTGGAGCCCTC
AGGCTCAGGGCCTACTCACAACTGTGCCAGCAGCTCCTCCTGCCTCCACCAATCG
GCAGTCAGGAAGGCAGCCTACTCCCTTATCTCCACCTCTAAGGGACACTCATCCTCAG
GCCATGCAGTGGAA (SEQ ID NO: 1).

For example, the sequence of the complete genome of type C HBV (serotype adr) is shown below:

CTCCACAACATTCCACCAAGCTCTGCTAGATCCCAGAGTGAGGGGCCTATATTTTC
CTGCTGGTGGCTCCAGTTCCGGAACAGTAAACCCTGTTCCGACTACTGCCTCACCCAT
ATCGTCAATCTTCTCGAGGACTGGGGACCCTGCACCGAACATGGAGAACACAACATC
AGGATTCCTAGGACCCCTGCTCGTGTTACAGGCGGGGTTTTTCTTGTTGACAAGAATC
CTCACAATACCACAGAGTCTAGACTCGTGGTGGACTTCTCTCAATTTTCTAGGGGGAG
CACCCACGTGTCCTGGCCAAAATTCGCAGTCCCCAACCTCCAATCACTCACCAACCTC
TTGTCCTCCAATTTGTCCTGGCTATCGCTGGATGTGTCTGCGGCGTTTTATCATATTCCT
CTTCATCCTGCTGCTATGCCTCATCTTCTTGTTGGTTCTTCTGGACTACCAAGGTATGTT
GCCCGTTTGTCCTCTACTTCCAGGAACATCAACCACCAGCACGGGGCCATGCAAGAC
CTGCACGATTCCTGCTCAAGGAACCTCTATGTTTCCCTCTTGTTGCTGTACAAAACCTT
CGGACGGAAACTGCACTTGTATTCCCATCCCATCATCCTGGGCTTTCGCAAGATTCCTA
TGGGAGTGGGCCTCAGTCCGTTTCTCCTGGCTCAGTTTACTAGTGCCATTTGTTCAGT
GGTTCGTAGGGCTTTCCCCCACTGTTTGGCTTTCAGTTATATGGATGATGTGGTATTGG
GGGCCAAGTCTGTACAACATCTTGAGTCCCTTTTTACCTCTATTACCAATTTTCTTTTGT
CTTTGGGTATACATTTAAACCCTAATAAAACCAAACGTTGGGGCTACTCCCTTAACTTC
ATGGGATATGTAATTGGAAGTTGGGGTACTTTACCGCAGGAACATATTGTACTAAAACT
CAAGCAATGTTTTCGAAAATTACCTGTAAATAGACCTATTGATTGGAAAGTCTGTCAA
AGAATTGTGGGTCTTTTGGGCTTTGCTGCCCCTTTTACACAATGTGGCTATCCTGCCTT
GATGCCTTTATATGCATGTATACAATCTAAGCAGGCTTTCACTTTCTCGCCAACTTACA
AGGCCTTTCTGTGTAAACAATATCTGAACCTTTACCCCGTTGCCCGGCAACGGTCAGG

TCTCTGCCAAGTGTTTGCTGACGCAACCCCCACTGGATGGGGCTTGGCCATAGGCCAT
CGGCGCATGCGTGGAACCTTTGTGGCTCCTCTGCCGATCCATACTGCGGAACTCCTAG
CAGCTTGTTTCGCTCGCAGCCGGTCTGGAGCGAAACTTATCGGCACCGACAACTCTG
TTGTCCTCTCTCGGAAATACACCTCCTTTCCATGGCTGCTAGGGTGTGCTGCCAACTG
GATCCTGCGCGGGACGTCCTTTGTCTACGTCCCGTCGGCGCTGAATCCCGCGGACGAC
CCGTCTCGGGGCCGTTTGGGGCTCTACCGTCCCCTTCTTCATCTGCCGTTCCGGCCGA
CCACGGGGCGCACCTCTCTTTACGCGGTCTCCCCGTCTGTGCCTTCTCATCTGCCGGA
CCGTGTGCACTTCGCTTCACCTCTGCACGTCGCATGGAGACCACCGTGAACGCCCAC
CAGGTCTTGCCCAAGGTCTTACATAAGAGGACTCTTGGACTCTCAGCAATGTCAACGA
CCGACCTTGAGGCATACTTCAAAGACTGTGTGTTTAAAGACTGGGAGGAGTTGGGGG
AGGAGATTAGGTTAAAGGTCTTTGTACTAGGAGGCTGTAGGCATAAATTGGTCTGTTC
ACCAGCACCATGCAACTTTTTCACCTCTGCCTAATCATCTCATGTTCATGTCCTACTGT
TCAAGCCTCCAAGCTGTGCCTTGGGTGGCTTTGGGGCATGGACATTGACCCGTATAAA
GAATTTGGAGCATCTGTGGAGTTACTCTCTTTTTTGCCTTCTGACTTCTTTCCTTCTATT
CGAGATCTCCTCGACACCGCTTCTGCTCTGTATCGGGAGGCCTTAGAGTCTCCGGAAC
ATTGTTCACCTCACCATACAGCACTCAGGCAAGCTATTCTGTGTTGGGGTGAGTTGAT
GAATCTGGCCACCTGGGTGGGAAGTAATTTGGAAGACCCAGCATCCAGGGAATTAGT
AGTCAGCTATGTCAATGTTAATATGGGCCTAAAAATCAGACAACTATTGTGGTTTCACA
TTTCCTGTCTTACTTTTGGAAGAGAAACTGTTCTTGAGTATTTGGTATCTTTTGGAGTG
TGGATTCGCACTCCTCCCGCTTACAGACCACCAAATGCCCCTATCTTATCAACACTTCC
GGAAACTACTGTTGTTAGACGACGAGGCAGGTCCCCTAGAAGAAGAACTCCCTCGCC
TCGCAGACGAAGATCTCAATCGCCGCGTCGCAGAAGATCTCAATCTCGGGAATCTCA
ATGTTAGTATCCCTTGGACTCATAAGGTGGGAAACTTTACTGGGCTTTATTCTTCTACT
GTACCTGTCTTTAATCCCGAGTGGCAAACTCCCTCCTTTCCTCACATTCATTTACAGGA
GGACATTATTAATAGATGTCAACAATATGTGGGCCCTCTTACAGTTAATGAAAAAAGG
AGATTAAAATTAATTATGCCTGCTAGGTTCTATCCTAACCTTACCAAATATTTGCCCTTA

GACAAAGGCATTAAACCGTATTATCCTGAACATGCAGTTAATCATTACTTCAAAACTAG
GCATTATTTACATACTCTGTGGAAGGCTGGCATTCTATATAAGAGAGAAACTACACGCA
GCGCCTCATTTTGTGGGTCACCATATTCTTGGGAACAAGAGCTACAGCATGGGAGGTT
GGTCTTCCAAACCTCGACAAGGCATGGGGACGAATCTTTCTGTTCCCAATCCTCTGGG
ATTCTTTCCCGATCACCAGTTGGACCCTGCGTTCGGAGCCAACTCAAACAATCCAGAT
TGGGACTTCAACCCCAACAAGGATCACTGGCCAGAGGCAAATCAGGTAGGAGCGGG
AGCATTCGGGCCAGGGTTCACCCCACCACACGGCGGTCTTTTGGGGTGGAGCCCTCA
GGCTCAGGGCATATTGACAACAGTGCCAGCAGCACCTCCTCCTGCCTCCACCAATCG
GCAGTCAGGAAGACAGCCTACTCCCATCTCTCCACCTCTAAGAGACAGTCATCCTCAG
GCCATGCAGTGGAA (SEQ ID NO: 2).

For example, the sequence of the complete genome of type D HBV (serotype ayw) is shown below:

TTCCACAACCTTTCACCAAACTCTGCAAGATCCCAGAGTGAGAGGCCTGTATTTCC
CTGCTGGTGGCTCCAGTTCAGGAGCAGTAAACCCTGTTCCGACTACTGCCTCTCCCTT
ATCGTCAATCTTCTCGAGGATTGGGGACCCTGCGCTGAACATGGAGAACATCACATCA
GGATTCCTAGGACCCCTTCTCGTGTTACAGGCGGGGTTTTTCTTGTTGACAAGAATCC
TCACAATACCGCAGAGTCTAGACTCGTGGTGGACTTCTCTCAATTTTCTAGGGGGAAC
TACCGTGTGTCTTGGCCAAAATTCGCAGTCCCCAACCTCCAATCACTCACCAACCTCC
TGTCCTCCAACTTGTCCTGGTTATCGCTGGATGTGTCTGCGGCGTTTTATCATCTTCCTC
TTCATCCTGCTGCTATGCCTCATCTTCTTGTTGGTTCTTCTGGACTATCAAGGTATGTTG
CCCGTTTGTCCTCTAATTCCAGGATCCTCAACCACCAGCACGGGACCATGCCGAACCT
GCATGACTACTGCTCAAGGAACCTCTATGTATCCCTCCTGTTGCTGTACCAAACCTTCG
GACGGAAATTGCACCTGTATTCCCATCCCATCATCCTGGGCTTTCGGAAAATTCCTATG
GGAGTGGGCCTCAGCCCGTTTCTCCTGGCTCAGTTTACTAGTGCCATTTGTTCAGTGG
TTCGTAGGGCTTTCCCCCACTGTTTGGCTTTCAGTTATATGGATGATGTGGTATTGGGG
GCCAAGTCTGTACAGCATCTTGAGTCCCTTTTTACCGCTGTTACCAATTTTCTTTTGTC

TTTGGGTATACATTTAAACCCTAACAAAACAAAGAGATGGGGTTACTCTCTGAATTTTA
TGGGTTATGTCATTGGAAGTTATGGGTCCTTGCCACAAGAACACATCATACAAAAAAT
CAAAGAATGTTTTAGAAAACTTCCTATTAACAGGCCTATTGATTGGAAAGTATGTCAA
CGAATTGTGGGTCTTTTGGGTTTTGCTGCCCCATTTACACAATGTGGTTATCCTGCGTT
AATGCCCTTGTATGCATGTATTCAATCTAAGCAGGCTTTCACTTTCTCGCCAACTTACA
AGGCCTTTCTGTGTAAACAATACCTGAACCTTTACCCCGTTGCCCGGCAACGGCCAGG
TCTGTGCCAAGTGTTTGCTGACGCAACCCCCACTGGCTGGGGCTTGGTCATGGGCCAT
CAGCGCGTGCGTGGAACCTTTTCGGCTCCTCTGCCGATCCATACTGCGGAACTCCTAG
CCGCTTGTTTTGCTCGCAGCAGGTCTGGAGCAAACATTATCGGGACTGATAACTCTGT
TGTCCTCTCCCGCAAATATACATCGTATCCATGGCTGCTAGGCTGTGCTGCCAACTGGA
TCCTGCGCGGGACGTCCTTTGTTTACGTCCCGTCGGCGCTGAATCCTGCGGACGACCC
TTCTCGGGGTCGCTTGGGACTCTCTCGTCCCCTTCTCCGTCTGCCGTTCCGACCGACC
ACGGGGCGCACCTCTCTTTACGCGGACTCCCCGTCTGTGCCTTCTCATCTGCCGGACC
GTGTGCACTTCGCTTCACCTCTGCACGTCGCATGGAGACCACCGTGAACGCCCACCG
AATGTTGCCCAAGGTCTTACATAAGAGGACTCTTGGACTCTCTGCAATGTCAACGACC
GACCTTGAGGCATACTTCAAAGACTGTTTGTTTAAAGACTGGGAGGAGTTGGGGGAG
GAGATTAGATTAAAGGTCTTTGTACTAGGAGGCTGTAGGCATAAATTGGTCTGCGCAC
CAGCACCATGCAACTTTTTCACCTCTGCCTAATCATCTCTTGTTCATGTCCTACTGTTC
AAGCCTCCAAGCTGTGCCTTGGGTGGCTTTGGGGCATGGACATCGACCCTTATAAAGA
ATTTGGAGCTACTGTGGAGTTACTCTCGTTTTTGCCTTCTGACTTCTTTCCTTCAGTAC
GAGATCTTCTAGATACCGCCTCAGCTCTGTATCGGGAAGCCTTAGAGTCTCCTGAGCA
TTGTTCACCTCACCATACTGCACTCAGGCAAGCAATTCTTTGCTGGGGGGAACTAATG
ACTCTAGCTACCTGGGTGGGTGTTAATTTGGAAGATCCAGCATCTAGAGACCTAGTAG
TCAGTTATGTCAACACTAATATGGGCCTAAAGTTCAGGCAACTCTTGTGGTTTCACATT
TCTTGTCTCACTTTTGGAAGAGAAACCGTTATAGAGTATTTGGTGTCTTTCGGAGTGT
GGATTCGCACTCCTCCAGCTTATAGACCACCAAATGCCCCTATCCTATCAACACTTCCG

GAAACTACTGTTGTTAGACGACGAGGCAGGTCCCCTAGAAGAAGAACTCCCTCGCCT
CGCAGACGAAGGTCTCAATCGCCGCGTCGCAGAAGATCTCAATCTCGGGAACCTCAA
TGTTAGTATTCCTTGGACTCATAAGGTGGGGAACTTTACTGGTCTTTATTCTTCTACTGT
ACCTGTCTTTAATCCTCATTGGAAAACACCATCTTTTCCTAATATACATTTACACCAAG
ACATTATCAAAAAATGTGAACAGTTTGTAGGCCCACTTACAGTTAATGAGAAAAGAAG
ATTGCAATTGATTATGCCTGCTAGGTTTTATCCAAAGGTTACCAAATATTTACCATTGGA
TAAGGGTATTAAACCTTATTATCCAGAACATCTAGTTAATCATTACTTCCAAACTAGAC
ACTATTTACACACTCTATGGAAGGCGGGTATATTATATAAGAGAGAAACAACACATAGC
GCCTCATTTTGTGGGTCACCATATTCTTGGGAACAAGATCTACAGCATGGGGCAGAAT
CTTTCCACCAGCAATCCTCTGGGATTCTTTCCCGACCACCAGTTGGATCCAGCCTTCA
GAGCAAACACAGCAAATCCAGATTGGGACTTCAATCCCAACAAGGACACCTGGCCAG
ACGCCAACAAGGTAGGAGCTGGAGCATTCGGGCTGGGTTTCACCCCACCGCACGGA
GGCCTTTTGGGGTGGAGCCCTCAGGCTCAGGGCATACTACAAACTTTGCCAGCAAAT
CCGCCTCCTGCCTCCACCAATCGCCAGACAGGAAGGCAGCCTACCCCGCTGTCTCCA
CCTTTGAGAAACACTCATCCTCAGGCCATGCAGTGGAA (SEQ ID NO: 3).

In certain embodiments, the sgRNA comprises a nucleotide sequence as set forth in any one of SEQ ID NOs: 4-1165.

In certain embodiments, the sgRNA comprises a partial sequence of the nucleotide sequence as set forth in any one of SEQ ID NOs: 4-1165, and the partial sequence has a length of 15-20 base pairs.

In certain embodiments, the at least one DNA binding protein is a CRISPR enzyme, a zinc finger nuclease (ZNF), a transcription activator-like effector nuclease (TALEN), a homing endonuclease, a dCas9-FokI nuclease, or a MegaTal nuclease.

In certain embodiments, the CRISPR enzyme is a class 2 Cas protein and/or a mutant thereof.

In certain embodiments, the CRISPR enzyme is one or more of the following Cas proteins: class 2 type II-A Cas protein, class 2 type II-B Cas protein, class 2 type II-C Cas protein, class 2 type V-A Cas protein, class 2 type V-B Cas protein, class 2 type V-C Cas protein, class 2 type V-U Cas protein, and mutants of the above.

In certain embodiments, the CRISPR enzyme is a Cas9 protein and/or a mutant thereof.

In certain embodiments, the at least one DNA binding protein is dCas9.

In certain embodiments, the dCas9 comprises Staphylococcus aureus dCas9, Streptococcus pyogenes dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (e.g., strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (e.g., strain DSM 16511) dCas9, Campylobacter lari (e.g., strain CF89-12) dCas9, Streptococcus thermophilus (e.g., strain LMD-9) dCas9.

In certain embodiments, the dCas9 comprises an amino acid sequence as set forth in SEQ ID NOs: 1170-1187.

In certain embodiments, the at least one gene expression modulator provides a modification of at least one nucleotide in the vicinity of the HBV gene and/or within the HBV gene regulatory element.

In certain embodiments, the at least one gene expression modulator comprises one or more selected from a DNA methyltransferase, a DNA hydroxymethylase, a DNA demethylase, a histone methyltransferase, a histone demethylase, a histone acetyltransferase, a histone deacetylase, a phosphatase, a kinase, a transcriptional activator, a transcriptional repressor, a portion of the above, and any combination of the above.

In certain embodiments, the modification of the at least one nucleotide is DNA methylation.

In certain embodiments, the at least one gene expression modulator comprises one or more selected from a DNA methyltransferase (DNMT), a zinc finger protein-based transcription factor, a portion of the above, and any combination of the above.

In certain embodiments, the at least one gene expression modulator comprises a DNA methyltransferase (DNMT) or a portion thereof, and a zinc finger protein-based transcription factor or a portion thereof.

In certain embodiments, the DNA methyltransferase is DNMT3A, DNMT3B, DNMT3L, DNMT1, or DNMT2.

In certain embodiments, the DNMT3A comprises an amino acid sequence as set forth in SEQ ID NO: 1166, and the DNMT3L comprises an amino acid sequence as set forth in SEQ ID NO: 1167 or 1195.

In certain embodiments, the zinc finger protein-based transcription factor is a Krüppel-associated inhibitor (KRAB) or a KRAB domain derived from ZIM3 (ZIM3 KRAB).

In certain embodiments, the zinc finger protein-based transcription factor comprises an amino acid sequence as set forth in SEQ ID NO: 1168 or 1196.

In certain embodiments, the DNA methyltransferase is selected from DNMT3A and DNMT3L, and a combination thereof, and the zinc finger protein-based transcription factor is KRAB or ZIM3 KRAB.

In certain embodiments, the fusion molecule comprises the at least one gene expression modulator fused to the C-terminus, the N-terminus or both termini of the at least one DNA binding protein.

In certain embodiments, the at least one gene expression modulator is directly fused to the at least one DNA binding protein.

In certain embodiments, the at least one gene expression modulator is indirectly fused to the at least one DNA binding protein via a non-regulatory agent, a second modulator or a linker.

In certain embodiments, the fusion molecule comprises domains DNMT3A-DNMT3L-dCas9-KRAB or domains DNMT3A-DNMT3L-ZIM3 KRAB-dCas9, wherein "-" means that the individual domains of the fusion molecule are directly and/or indirectly linked, and the individual domains are linked in order from N-terminus to C-terminus.

In certain embodiments, the fusion molecule comprises an amino acid sequence as set forth in SEQ ID NO: 1169 or 1194.

In certain embodiments, the fusion molecule further comprises at least one nuclear localization sequence (NLS).

In certain embodiments, the at least one nuclear localization sequence is directly or indirectly fused to the C-terminus, the N-terminus or both termini of the at least one DNA binding protein.

In certain embodiments, the nucleic acid sequence encoding the fusion molecule is deoxyribonucleic acid (DNA) or messenger ribonucleic acid (mRNA).

In certain embodiments, the fusion molecule is packaged in liposomes or lipid nanoparticles.

In certain embodiments, the fusion molecule and the sgRNA are packaged in liposomes or lipid nanoparticles.

In certain embodiments, the fusion molecule and the sgRNA are packaged in the same liposome or lipid nanoparticle, or in different liposomes or lipid nanoparticles.

In certain embodiments, the liposome or the lipid nanoparticle comprises an ionizable lipid (20%-70%, molar ratio), a PEGylated lipid (0%-30%, molar ratio), a supporting lipid (30%-50%, molar ratio), and cholesterol (10%-50%, molar ratio).

In certain embodiments, the ionizable lipid is selected from a pH-responsive ionizable lipid, a thermo-responsive ionizable lipid and a photo-responsive ionizable lipid.

In certain embodiments, the fusion molecule is packaged in AAV vectors.

In certain embodiments, the fusion molecule and the sgRNA are packaged in AAV vectors.

In certain embodiments, the fusion molecule and the sgRNA are packaged in the same AAV vector or different AAV vectors.

In certain embodiments, the composition is a pharmaceutical composition comprising a pharmaceutically acceptable carrier.

In another aspect, the present application provides a single guide RNA (sgRNA), wherein the sgRNA comprises a sequence complementary to a target DNA sequence, and the target DNA sequence is located in the vicinity of a hepatitis B virus (HBV) gene and/or within an HBV gene regulatory element; the HBV gene is a type B HBV gene comprising a nucleotide sequence as set forth in SEQ ID NO: 1, a type C HBV gene comprising a nucleotide sequence as set forth in SEQ ID NO: 2, or a type D HBV gene comprising a nucleotide sequence as set forth in SEQ ID NO: 3; the HBV gene regulatory element comprises a transcription initiation site, a core promoter, a promoter, an enhancer, a silencer, an insulator element, a boundary element, and/or a locus control region, and the target DNA sequence is located between nucleotide at position 1056 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

In certain embodiments, the target DNA sequence is located between nucleotide at position 1056 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

In certain embodiments, the target DNA sequence is located between nucleotide at position 1060 and nucleotide at position 1079, between nucleotide at position 1149 and nucleotide at position 1612, between nucleotide at position 1693 and nucleotide at position 1852, and/or between nucleotide at position 2863 and nucleotide at position 2882 of the HBV gene.

In certain embodiments, the target DNA sequence is located in one or more of following regions of the type B HBV gene: between nucleotide at position 1149 and nucleotide at position 1190, between nucleotide at position 1210 and nucleotide at position 1310, between nucleotide at position 1350 and nucleotide at position 1400, between nucleotide at position 1420 and nucleotide at position 1450, and between nucleotide at position 1470 and nucleotide at position 1592.

In certain embodiments, the target DNA sequence is located in one or more of following regions of the type C HBV gene: between nucleotide at position 1150 and nucleotide at position 1180, between nucleotide at position 1200 and nucleotide at position 1310, between nucleotide at position 1350 and nucleotide at position 1390, between nucleotide at position 1420 and nucleotide at position 1460, and between nucleotide at position 1480 and nucleotide at position 1593.

In certain embodiments, the target DNA sequence is located in one or more of following regions of the type D HBV gene: between nucleotide at position 1056 and nucleotide at position 1079, between nucleotide at position 1101 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2882, between nucleotide at position 2911 and nucleotide at position 2930, and between nucleotide at position 3048 and nucleotide at position 3067.

In certain embodiments, the target DNA sequence is located in one or more of following regions of the type D HBV gene: between nucleotide at position 1060 and nucleotide at position 1079, between nucleotide at position 1160 and nucleotide at position 1310, between nucleotide at position 1253 and nucleotide at position 1284, between nucleotide at position 1370 and nucleotide at position 1470, between nucleotide at position 1490 and nucleotide at position 1612, between nucleotide at position 1693 and nucleotide at position 1852, and between nucleotide at position 2863 and nucleotide at position 2882.

In certain embodiments, the sgRNA comprises a nucleotide sequence as set forth in any one of SEQ ID NOs: 4-1165.

In certain embodiments, the sgRNA comprises a partial sequence of a nucleotide sequence as set forth in any one of SEQ ID NOs: 4-1165, and the partial sequence has a length of 15 to 20 base pairs.

In another aspect, the present application provides a nucleic acid molecule encoding the sgRNA described herein.

In another aspect, the present application provides a method for reducing or eliminating the expression of a hepatitis B virus (HBV) gene product in a cell, wherein the method comprises a step of introducing the composition of the present application into the cell, thereby reducing or eliminating the expression of the HBV gene product in the cell.

In another aspect, the present application provides a method for reducing or eliminating the expression of a hepatitis B virus (HBV) gene product in a subject in vivo, wherein the method comprises a step of introducing the composition of the present application into a cell of the subject, thereby reducing or eliminating the expression of the HBV gene product in the subject.

In another aspect, the present application provides a method for treating a hepatitis B virus (HBV) infection-related disease in a subject or alleviating a symptom of a HBV infection-related disease in a subject, wherein the method comprises a step of introducing an effective amount of the composition of the present application into a cell of the subject.

In certain embodiments, the subject is a mammal, such as a human, a monkey, a mouse, a rat, a rabbit, a pig, a horse, a cat, and a dog.

In certain embodiments, the method comprises administering the composition to the subject one or more times.

In certain embodiments, the method comprises administering the composition to the subject at least twice.

In certain embodiments, an interval between administering the composition more than once or administering the composition at least twice is 1 day, 2 days, 3 days, 4 days, 5 days, 6 days, 7 days, 8 days, 9 days, 10 days, 11 days, 12 days, 13 days, 14 days or 15 days. For example, the method comprises administering the composition to the subject once a day, once every 2 days, once every 3 days, once every 4 days, once every 5 days, once every 6 days, once every 7 days, once every 8 days, once every 9 days, once every 10 days, once every 11 days, once every 12 days, once every 13 days, once every 14 days, or once every 15 days until the subject achieves a clinical cure.

In certain embodiments, the HBV infection-related disease comprises hepatitis, cirrhosis, liver fibrosis, and hepatocellular carcinoma caused by HBV infection.

In certain embodiments, the composition is for use in treating a hepatitis B virus (HBV) infection-related disease in a subject or alleviating a symptom of an HBV infection-related disease in a subject.

In another aspect, the present application provides a kit comprising a composition of the present application and a container for placing the composition and/or an instruction for use.

Those skilled in the art may easily discern other aspects and advantages of the present application from the following detailed description. Only exemplary embodiments of the present application are shown and described in the following detailed description. As those skilled in the art will realize, the contents of the present application enable those skilled in the art to make modifications to the specific embodiments disclosed without departing from the spirit and scope of the invention to which the present application relates. Accordingly, the drawings and descriptions in the specification of the present application are illustrative only and not limited.

### BRIEF DESCRIPTION OF DRAWINGS

The specific features of the invention to which the present application relates are as shown in the appended claims. The features and advantages of the invention to which the present application relates may be better understood by reference to the exemplary embodiments and the drawings described in detail below. The accompanying drawings are briefly described as follows:
FIG. 1A shows a schematic diagram of the reporter system for HBV viral protein expression described in the present application.
FIG. 1B shows a schematic diagram of the screening protocol for sgRNA functional validation using the reporter cell line shown in FIG. 1A.

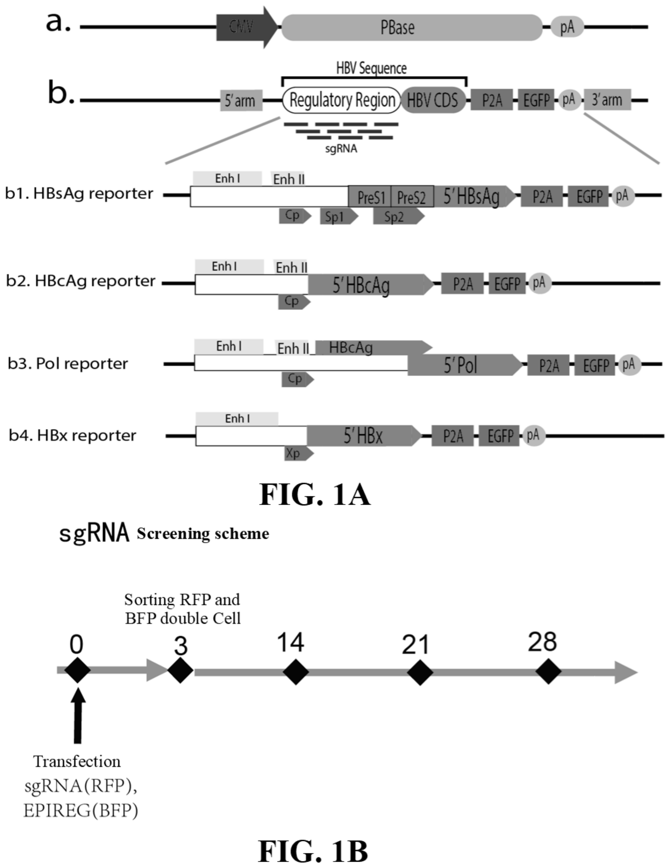

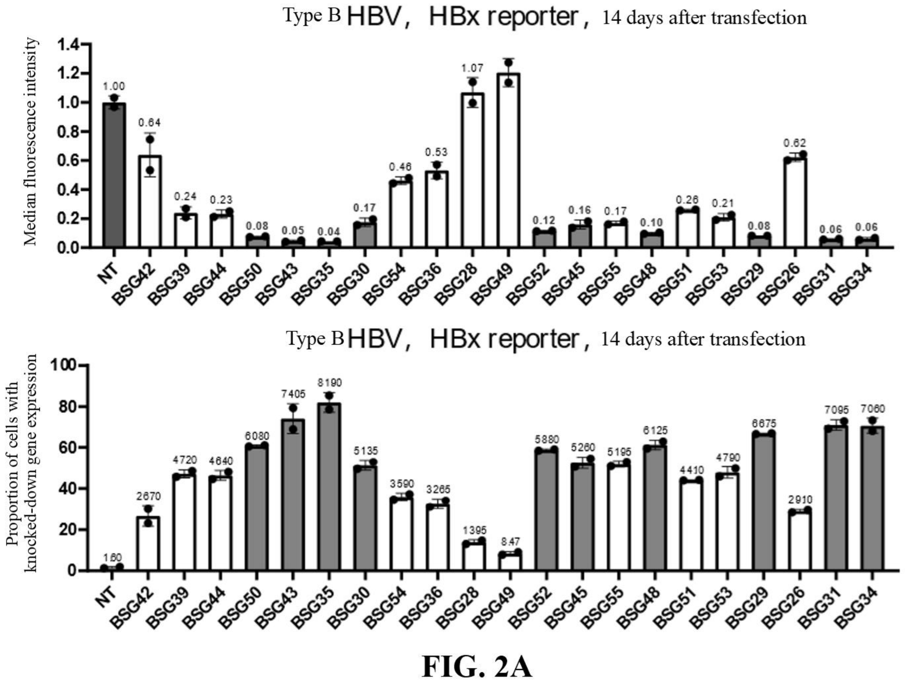

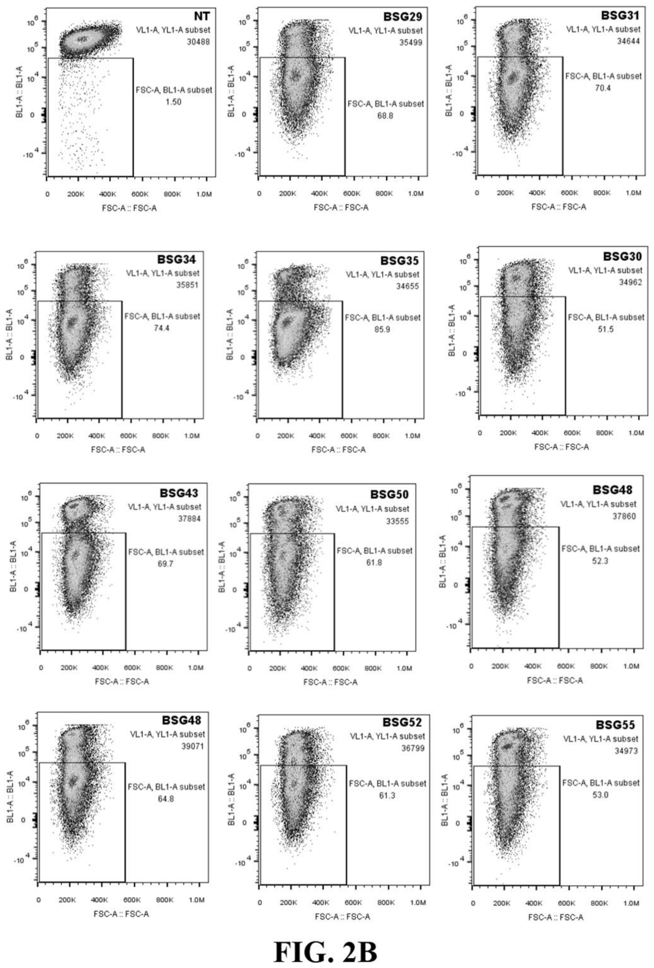

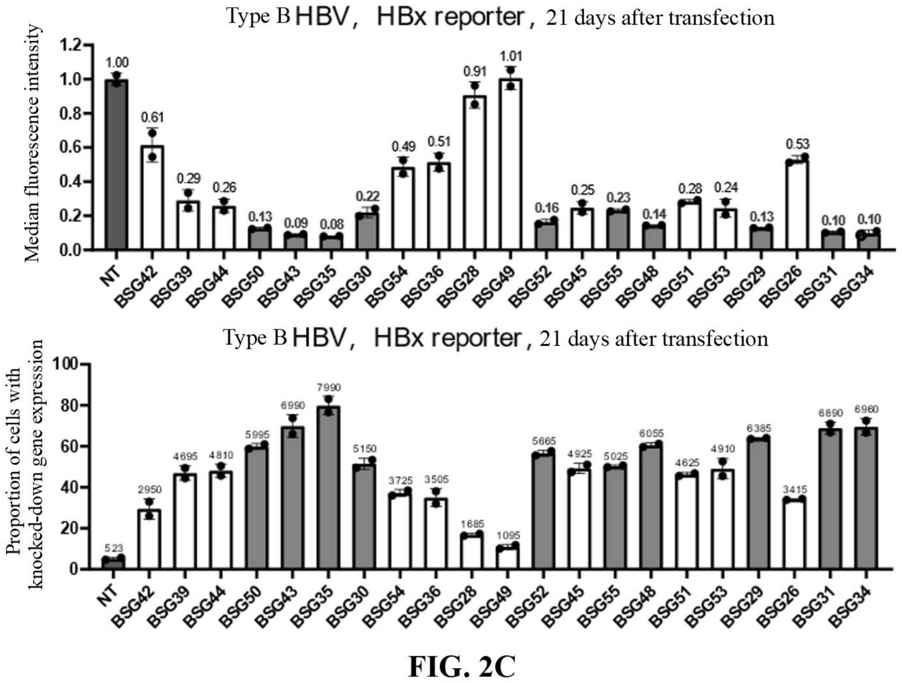

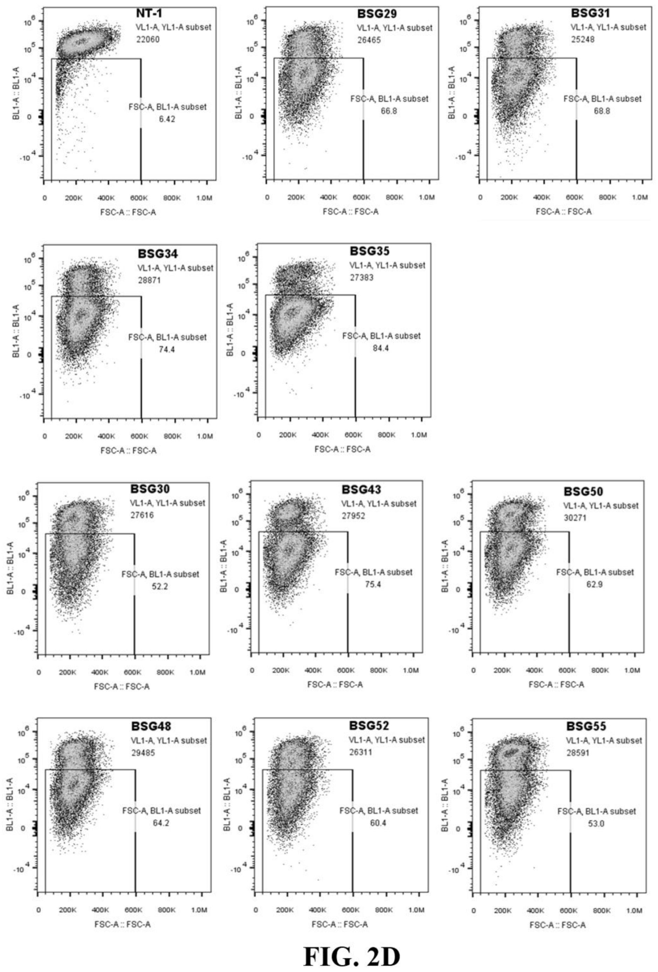

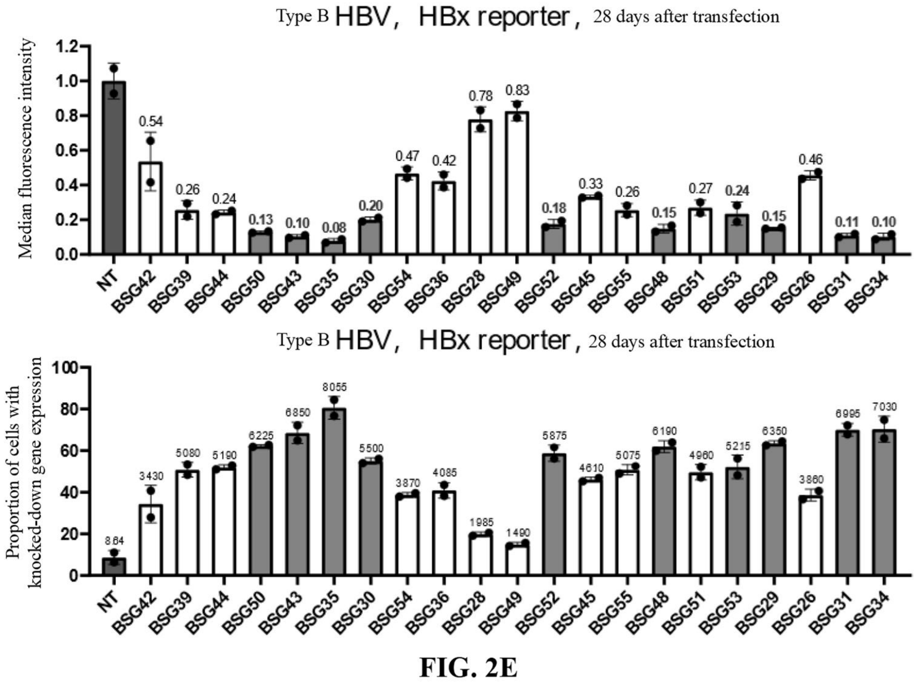

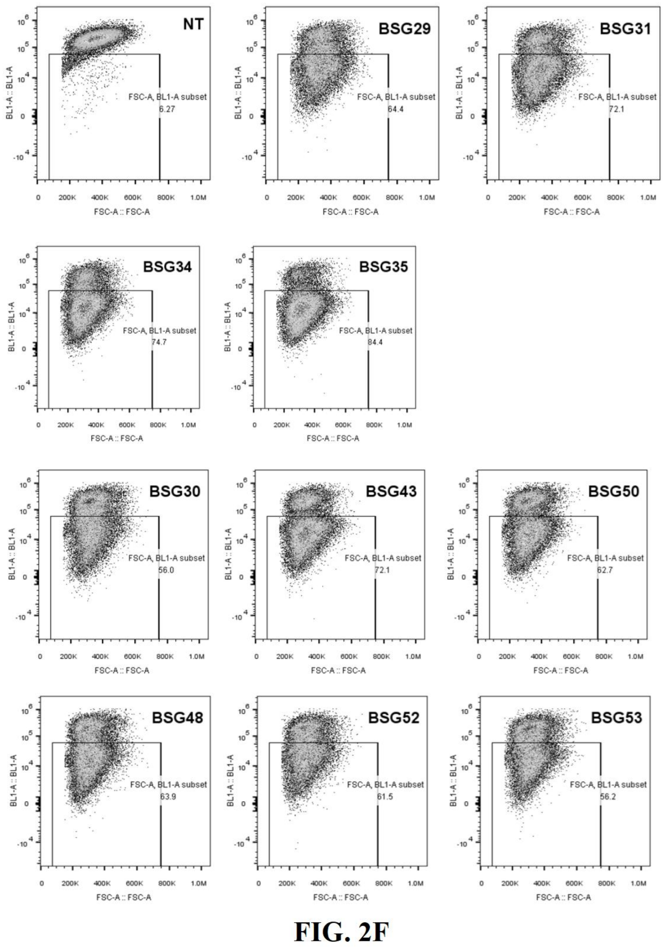

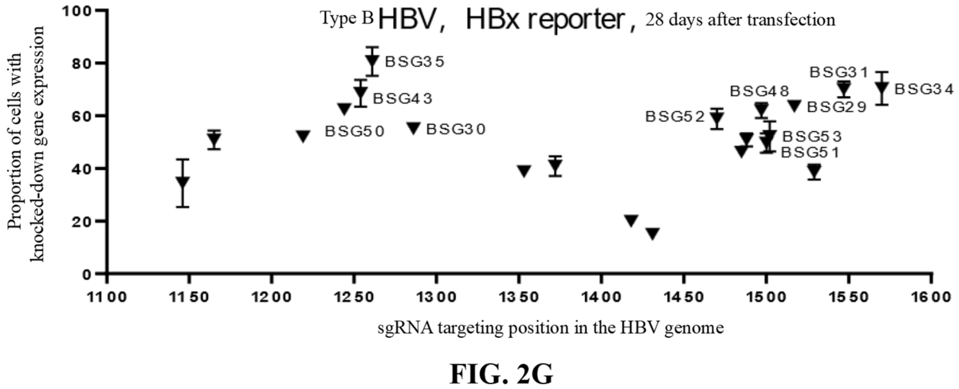

FIGs. 2A-2F show the changes in fluorescence intensity 14, 21, and 28 days after epigenetic editing of the type B HBV gene using the HBx reporter cell line shown in FIG. 1A. Figure 2G shows the relationship between the target site of sgRNA in the type B HBV genome and the knockdown ability of the sgRNA (the vertical axis represents the proportion of cells with knocked-down gene expression by different sgRNAs 28 days after transfection, which can represent the editing ability of sgRNA; the horizontal axis represents the starting position of the target sequence of each sgRNA in the type B HBV genome).
FIGs. 3A-3B show the changes in fluorescence intensity 14 days after epigenetic editing of the type C HBV gene using the HBx reporter cell line shown in FIG. 1A. FIG. 3C shows the relationship between the target site of sgRNA in the type C HBV genome and the knockdown ability of the sgRNA (the vertical axis represents the proportion of cells with knocked-down gene expression by different sgRNAs 14 days after transfection, which can represent the editing ability of sgRNA; the horizontal axis represents the starting position of the target sequence of each sgRNA in the type C HBV genome).

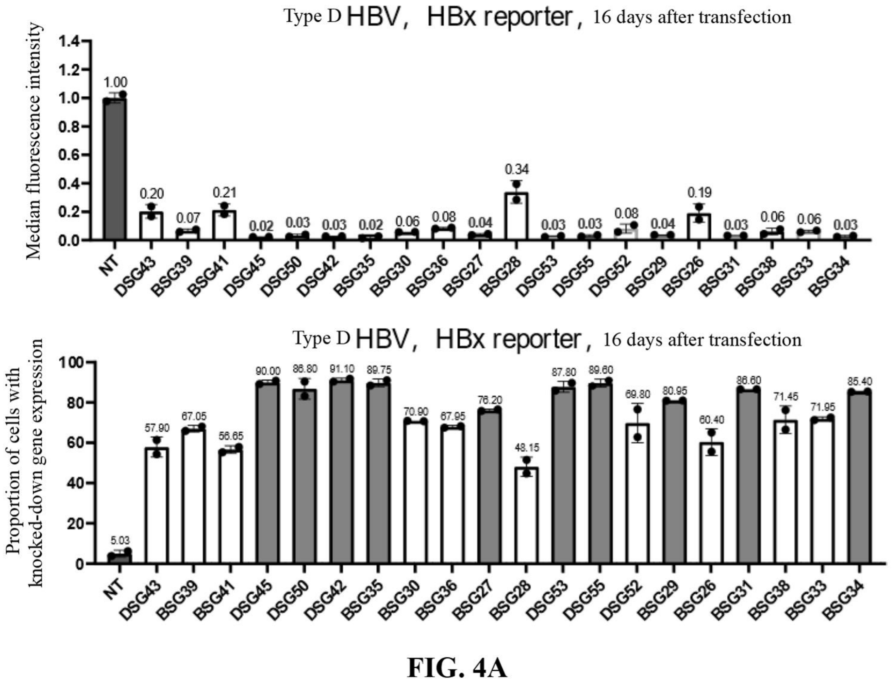

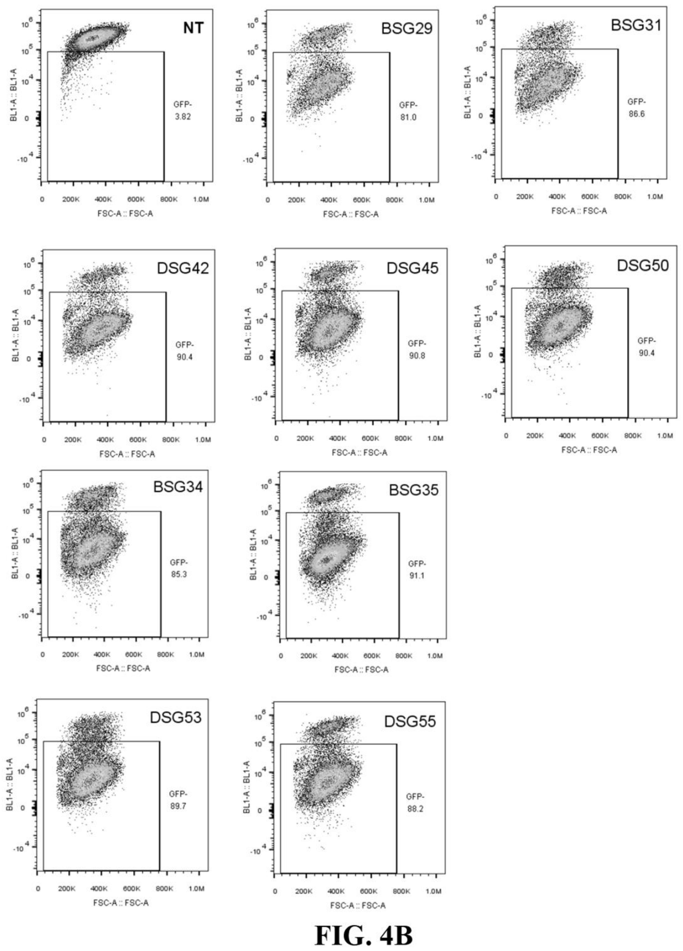

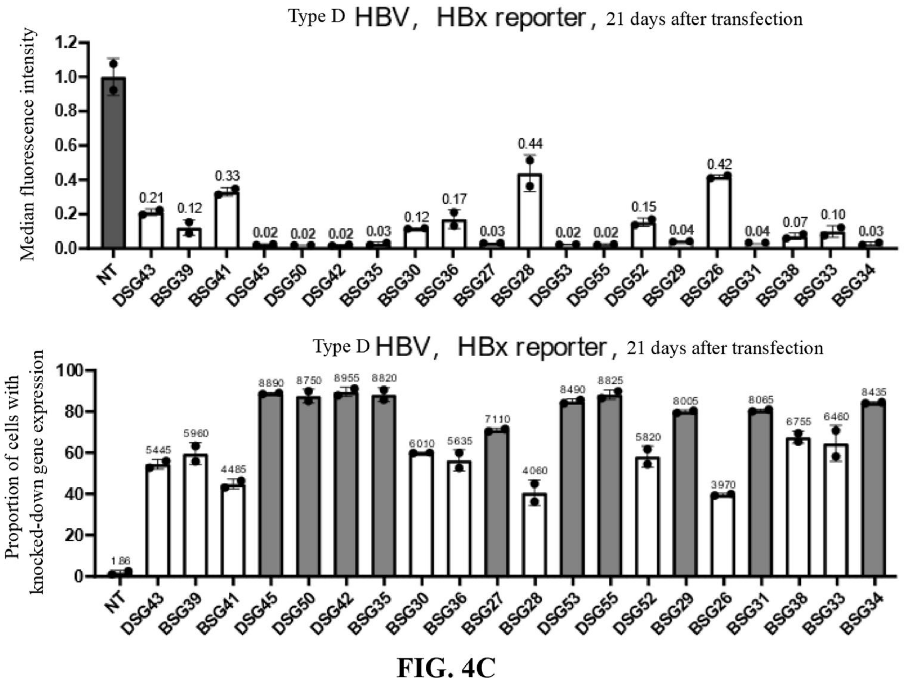

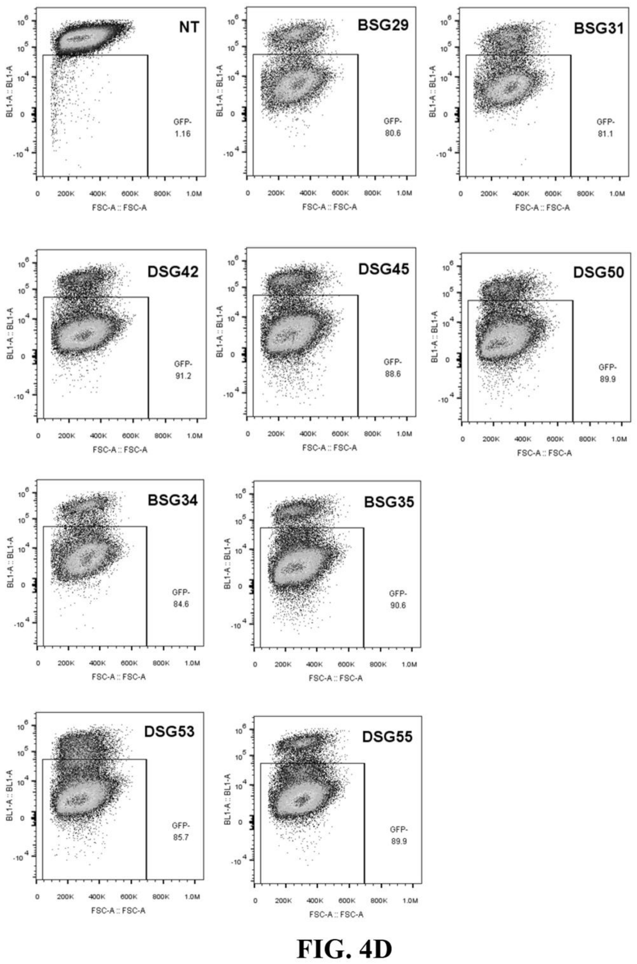

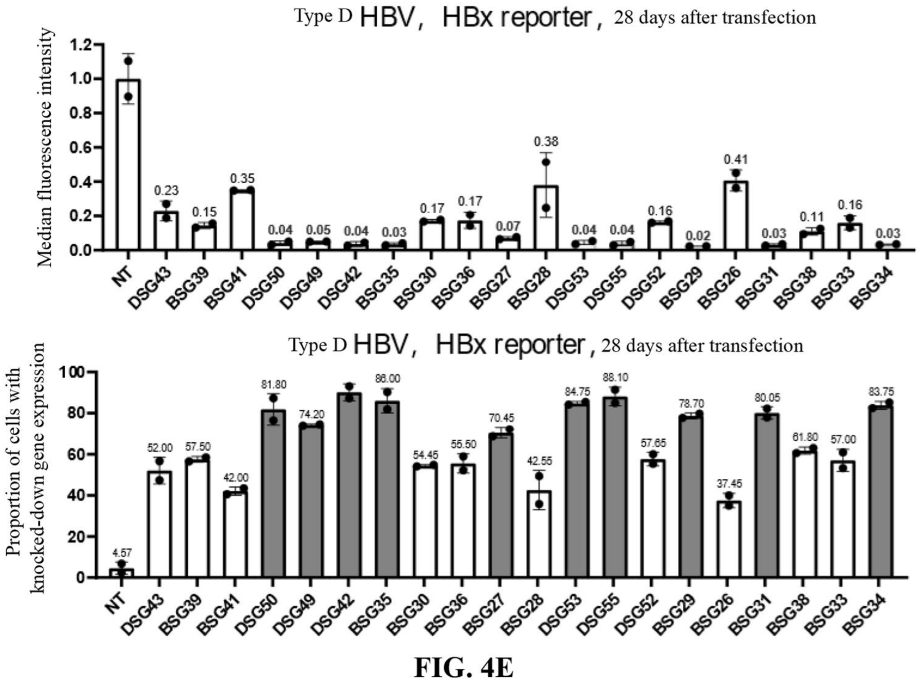

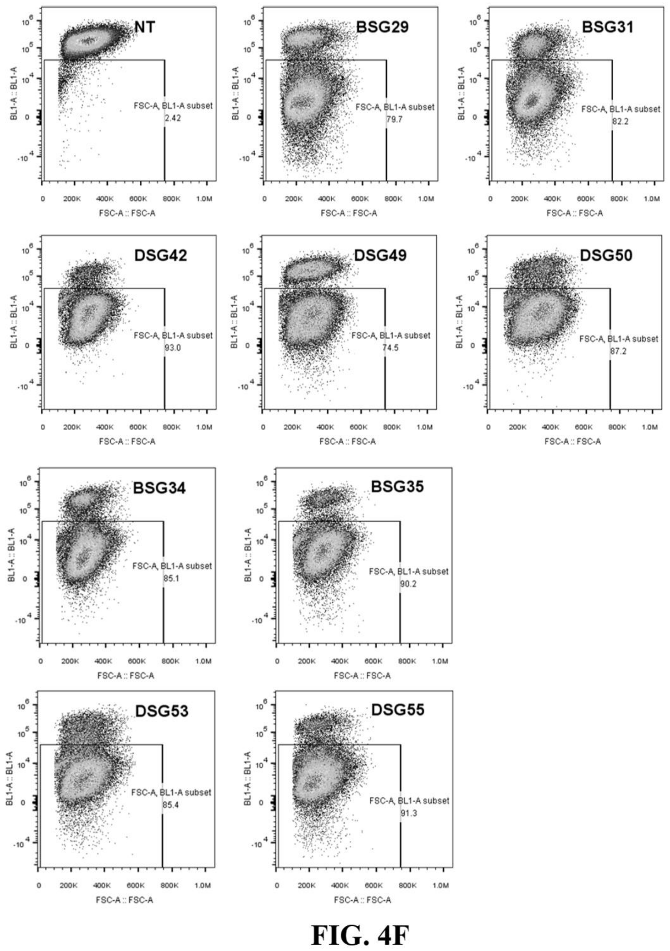

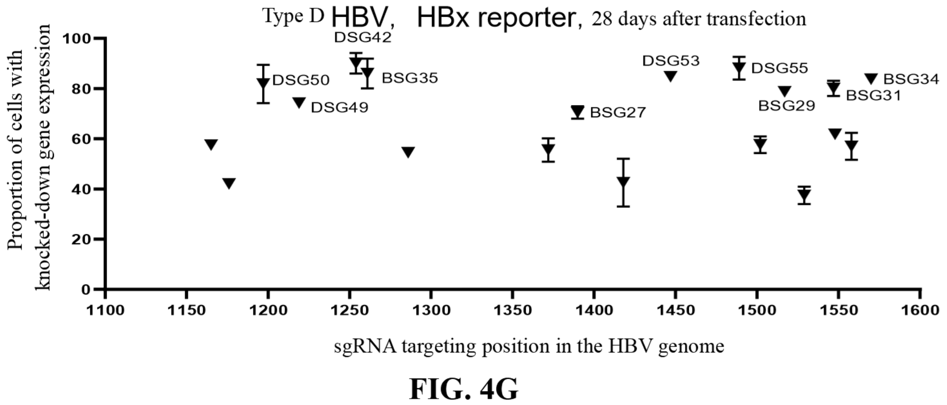

FIGs. 4A-4F show the changes in fluorescence intensity 16, 21, and 28 days after epigenetic editing of the type D HBV gene using the HBx reporter cell line shown in FIG. 1A. FIG. 4G shows the relationship between the target site of sgRNA in the type D HBV genome and the knockdown ability of the sgRNA (the vertical axis represents the proportion of cells with knocked-down gene expression by different sgRNAs 28 days after transfection, which can represent the editing ability of sgRNA; the horizontal axis represents the starting position of the target sequence of each sgRNA in the type D HBV genome).
FIG. 5A shows a schematic diagram of the method described in the present application for functional validation of epigenetic editing in a primary hepatocyte (PHH) HBV infection model.
FIG. 5B shows the antigen level results of the pharmaceutical composition described in the present application (comprising different sgRNAs, respectively) 2 days, 4 days, 6 days, 8 days, and 10 days after being delivered to HBV-infected primary hepatocytes, respectively.
FIG. 6 shows a schematic diagram of the process for screening sgRNA libraries using reporter cell lines.
FIG. 7 shows the results of screening sgRNA libraries using reporter cell lines for three type D HBV genes HBx, HBcAg, and HBsAg (the vertical axis represents the fold change in NGS sequencing reads of different sgRNAs in the GFP-negative cell population compared to the original library 14 days after transfection, which can represent the editing ability of the sgRNA; the horizontal axis represents the starting position of the target sequence of each sgRNA in the type D HBV genome).
FIG. 8 shows the validation of candidate sgRNAs screened from the library in primary hepatocytes (PHH). The results shown are the inhibition rates of various HBV markers and PHH cell viability 14 days after HBV infection.
FIGs. 9A-9C show the knockdown effect of EPIREG on HBV markers in transgenic HBV mice. The results shown in the figure are the overall change curves of HBV markers in each control/administration group, the change curves of HBV markers in each mouse in each administration group, and the HBsAb change curves of three mice that produced antibodies after administration. The HBV marker content shown in each curve is log10-transformed for data analysis.
FIG. 10 shows the knockdown effect of EPIREG on HBV markers in AAV-HBV mice. The HBV marker contents shown in the figure are all log10-transformed for data analysis.

### DETAILED DESCRIPTION

The embodiments of the invention of the present application will be illustrated below with specific examples. Those skilled in the art may easily understand other advantages and effects of the invention of the present application from the disclosure of the specification.

### DEFINITION OF TERMS

In the application, the term "fusion molecule" generally refers to a molecule composed of at least two moieties (bipartite molecule), for example, in the present application, it comprises at least one DNA binding protein described in the present application and at least one gene expression regulator described in the present application, thereby forming a single entity. For example, the at least one gene expression modulator may be fused to the at least one DNA binding protein at the N-terminus, C-terminus or any amino acid other than terminal amino acid, and other molecules or moieties may also be fused to moieties already included in the fusion molecule. The moieties making up the fusion molecule can be separated by a linker or can be directly coupled. In certain embodiments, the fusion molecule is a fusion protein, which may be a chimeric protein produced by covalently or non-covalently linking, directly or indirectly, two or more genes that initially encode separate proteins. In certain embodiments, translation of the fusion gene results in a single polypeptide having functional properties derived from each of the original proteins. The optimal order and/or combination of assays for determining moieties in the fusion molecules of the present application are well known to those skilled in the art.

In the present application, the terms "polynucleotide," "nucleotide," "nucleotide sequence," "nucleic acid," and "oligonucleotide" are used interchangeably. They generally refer to a polymeric form of nucleotides (either deoxyribonucleotides or ribonucleotides, or analogs thereof) of any length. A polynucleotide may have any three-dimensional structure and may perform any function, known or unknown. The following are non-limiting examples of polynucleotides: coding or non-coding regions of a gene or gene fragment, multiple loci (a locus) defined by linkage analysis, exons, introns, messenger RNA (mRNA), transfer RNA, ribosomal RNA, short hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. Polynucleotides may include one or more modified nucleotides, such as methylated nucleotides and nucleotide analogs. If present, modifications to the nucleotide structure may be conducted before or after polymer assembly. The sequence of nucleotides may be interrupted by non-nucleotide components. Polynucleotides can be further modified after polymerization, such as by conjugation with labeled components.

In the present application, the term "DNA binding protein" generally refers to a larger protein composed of one or more DNA-binding domains (domains of different functions), and the DNA-binding domains are independently folded protein domains containing at least one motif that recognizes double-stranded or single-stranded DNA. For example, the DNA binding domain may recognize a specific DNA sequence (recognition or regulatory sequence) or have a general affinity for DNA. In certain instances, other domains of the DNA binding protein often modulate the activity of the DNA binding domain; the DNA binding function may be structural or involve transcriptional regulation, and sometimes the two functions overlap. In certain embodiments of the methods and compositions provided according to the present application, the DNA binding protein may comprise a (DNA) nuclease, such as a nuclease capable of targeting DNA in a sequence-specific manner or capable of being directed or instructed to target DNA in a sequence-specific manner, such as a CRISPR-Cas system, a zinc finger nuclease (ZFN), a transcription activator-like effector nuclease (TALEN), or a meganuclease. In some embodiments, the DNA binding protein is a DNA nuclease derived from the CRISPR-Cas system. For example, the DNA nuclease derived from the CRISPR-Cas system is a Cas protein.

In the present application, the term "Cas protein" is used interchangeably with "CRISPR protein", "CRISPR enzyme", "CRISPR-Cas protein", "CRISPR-Cas enzyme", "Cas", "CRISPR effector" or "Cas effector protein" and is one of the components of the CRISPR-Cas system. The Cas protein (e.g., an engineered Cas protein) may have a nuclease activity that is substantially identical (e.g., between 80% and 100%, between 90% and 100%, between 95% and 100%, between 98% and 100%, between 99% and 100%, between 99.9% and 100%, or about 100%) to the wild-type counterpart Cas protein. In certain cases, the engineered Cas protein has a nuclease activity that is higher (e.g., at least 5%, at least 10%, at least 20%, at least 30%, at least 40%, at least 50%, at least 60%, at least 70%, at least 80%, or at least 90% higher) than the wild-type counterpart Cas protein. Alternatively or additionally, the Cas protein (e.g., an engineered Cas protein) may have a specificity that is at least 5%, at least 10%, at least 20%, at least 30%, at least 40%, at least 50%, at least 60%, at least 70%, at least 80%, or at least 90% higher than the wild-type counterpart Cas protein. In particular examples, the Cas protein (e.g., an engineered Cas protein) has a specificity that is at least 30% higher than the wild-type counterpart Cas protein. As used herein, the term "specificity" of Cas may correspond to the number or percentage of on-target polynucleotide cleavage events relative to the number or percentage of all polynucleotide cleavage events, including on-target and off-target events. The activity and specificity of the Cas protein are consistent with those described in the following documents: Hsu PD et al., DNA targeting specificity of RNA-guided Cas9 nucleases, Nat Biotechnol. September 2013; 31 (9): 827-832 and Slaymaker IM et al., Rationally engineered Cas9 nucleases with improved specificity, Science. January 1 2016; 351(6268): 84-88, which also describe examples of methods for detecting the activity and specificity of Cas proteins, and are incorporated herein by reference in their entirety.

The nucleic acid molecule encoding Cas can be codon optimized. Examples of codon-optimized sequences in this case are sequences that are optimized for expression in a eukaryote (e.g., a human) (i.e., optimized for expression in a human), or sequences that are optimized for another eukaryote, such as an animal or mammal as discussed herein; see, e.g., the SaCas9 human codon-optimized sequences in WO 2014/093622 (PCT/US2013/074667). While this is preferred, it should be understood that other examples are possible and that codon optimization for host species other than humans or codon optimization for specific organs is known. In some embodiments, the enzyme coding sequence encoding Cas is codon-optimized for expression in a particular cell, such as a eukaryotic cell. The eukaryotic cell may be a cell of or a cell derived from a particular organism, such as a mammal, including but not limited to a human or non-human eukaryote or an animal or mammal described herein, such as a mouse, rat, rabbit, dog, livestock or non-human mammal or primate. In some embodiments, methods for altering the germline genetic characteristics of humans and/or methods for altering the genetic characteristics of animals that may cause them to suffer without any substantial medical benefit to humans or animals, as well as animals produced by such methods, may be excluded. In general, codon optimization refers to the process of modifying a nucleic acid sequence to enhance expression in a target host cell by replacing at least one codon (e.g., about or more than about 1, 2, 3, 4, 5, 10, 15, 20, 25, 50, or more codons) of a native sequence with a codon that is more frequently or most frequently used in a gene of that host cell, while maintaining the native amino acid sequence. Different species exhibit specific bias towards specific codons for specific amino acids. Codon bias (differences in codon usage between organisms) is generally associated with the translation efficiency of messenger RNA (mRNA), which in turn is believed to depend on, among other things, the nature of the codon being translated and the availability of a particular transport RNA (tRNA) molecule. The predominance of the selected tRNA in a cell typically reflects the codons most commonly used in peptide synthesis. Accordingly, genes can be tailored for optimal gene expression in a given organism based on codon optimization. codon usage tables are readily available in the "Codon Usage Database" available, for example, at www.kazusa.orjp/codon/, and these tables can be modified in a variety of ways. See Nakamura, Y. et al. "Codon usage tabulated from the international DNA sequence databases: status for the year 2000" Nucl. Acids Res. 28: 292

(2000). Computer algorithms for codon optimization of specific sequences for expression in specific host cells, such as Gene Forge (Appagen; Jacobus, PA.), are also available. In some embodiments, one or more codons (e.g., 1, 2, 3, 4, 5, 10, 15, 20, 25, 50 or more, or all codons) in the sequence encoding Cas correspond to the most commonly used codon for a particular amino acid.

In some embodiments, the Cas protein may have nucleic acid cleavage activity. The Cas protein may have RNA binding and DNA cleavage functions. In some embodiments, Cas can direct cleavage of one or both nucleic acid strands at or near the location of the target sequence, such as within the target sequence and/or within a sequence complementary to the target sequence or a sequence related to the target sequence, for example within about 1, 2, 3, 4, 5, 6, 7, 8, 9, 10, 15, 20, 25, 50, 100, 200, 500 or more base pairs from the first or last nucleotide of the target sequence. In some embodiments, the Cas protein can direct more than one cleavage (e.g., one, two, three, four, five, or more cleavages) of one or both strands within the target sequence and/or within a sequence complementary to the target sequence or at a sequence related to the target sequence and/or within about 1, 2, 3, 4, 5, 6, 7, 8, 9, 10, 15, 20, 25, 50, 100, 200, 500, or more base pairs from the first or last nucleotide of the target sequence. In some embodiments, the cleavage can be blunt-ended, i.e., produce blunt ends. In some embodiments, the cleavage can be staggered, i.e., generating sticky ends.

In some embodiments, the vector encodes a nucleic acid-targeting Cas protein that can be mutated relative to a corresponding wild-type enzyme such that the mutated nucleic acid-targeting Cas protein lacks the ability to cleave one or both strands of a target polynucleotide containing a target sequence, e.g., an alteration or mutation in the HNH domain produces a mutated Cas that lacks substantially all DNA cleavage activity, e.g., the DNA cleavage activity of the mutated enzyme is about no more than 25%, 10%, 5%, 1%, 0.1%, 0.01% or less of the nucleic acid cleavage activity of the non-mutated form of the enzyme; an example is when the nucleic acid cleavage activity of the mutated form is zero or negligible compared to the non-mutated form.

In some embodiments, the Cas protein may form a component of an inducible system. The inducible nature of the system would allow spatiotemporal control of gene editing or gene expression using one form of energy. Forms of energy may include, but are not limited to, electromagnetic radiation, acoustic energy, chemical energy, and thermal energy. Examples of inducible systems include tetracycline-inducible promoters (Tet-On or Tet-Off), small molecule two-hybrid transcription activation systems (FKBP, ABA, etc.), or light-inducible systems (photochrome, LOV domain, or cryptochrome). In one embodiment, the CRISPR effector protein may be part of a light-inducible transcriptional effector (LITE) that directs changes in transcriptional activity in a sequence-specific manner. Components of light may include a CRISPR effector protein, a photo-responsive cytochrome heterodimer (e.g. from Arabidopsis thaliana), and a transcriptional activation/repression domain. Other examples of inducible DNA binding proteins and methods of use thereof are provided in US 61/736465 and US 61/721,283 and WO 2014018423 A2, which are hereby incorporated by reference in their entireties.

In some embodiments, the mutated Cas may have one or more mutations that result in a reduction in off-target effects, e.g., an improved CRISPR enzyme for effecting modification of a target locus but reducing or eliminating off-target activity (such as when complexed with a guide RNA), and an improved CRISPR enzyme for enhancing CRISPR enzyme activity (such as when complexed with a guide RNA). It should be understood that the mutated enzymes as described below can be used in any of the methods according to the present application as described elsewhere herein. Any of the methods, products, compositions, and uses as described elsewhere herein are equally applicable to mutated CRISPR enzymes as described in further detail below.

Methods and mutations that can be used in various combinations to enhance or reduce the activity and/or specificity of on-target activity versus off-target activity, or to enhance or reduce the binding and/or specificity of on-target binding versus off-target binding, can be used to compensate for or enhance mutations or modifications made to promote other effects. Such mutations or modifications made to promote other effects include mutations or modifications to Cas and/or mutations or modifications to guide RNA. The methods and mutations of the present application are used to modulate Cas nuclease activity and/or binding to chemically modified guide RNA.

In certain embodiments, the catalytic activity of the Cas protein of the present application is altered or modified. it should be understood that a mutated Cas has an altered or modified catalytic activity if the catalytic activity differs from the catalytic activity of the corresponding wild-type Cas protein (e.g., an unmutated Cas protein). The catalytic activity can be determined by methods known in the art. By way of example, and not limitation, catalytic activity can be determined in vitro or in vivo by determining the percentage of insertions/deletions (indels) (e.g., after a given time, or at a given dose). In certain embodiments, the catalytic activity is enhanced. In certain embodiments, the catalytic activity is enhanced by at least 5%, at least 10%, at least 20%, at least 30%, at least 40%, at least 50%, at least 60%, at least 70%, at least 80%, at least 90%, or at least 100%. In certain embodiments, the catalytic activity is reduced. In certain embodiments, the catalytic activity is reduced by at least 5%, preferably at least 10%, more preferably at least 20%, such as at least 30%, at least 40%, at least 50%, at least 60%, at least 70%, at least 80%, at least 90%, or (substantially) 100%. One or more mutations herein can inactivate catalytic activity, which can significantly reduce overall catalytic activity, reduce catalytic activity to below detectable levels, or reduce catalytic activity to non-measurable.

One or more characteristics of an engineered Cas protein may differ from those of a corresponding wild-type Cas protein. Examples of such characteristics include catalytic activity, gRNA binding, specificity of the Cas protein (e.g., specificity for editing a determined target), stability of the Cas protein, off-target binding, target binding, protease activity, nickase activity, PFS recognition. In some examples, the engineered Cas protein may comprise one or more mutations of the corresponding wild-type Cas protein. In some embodiments, the engineered Cas protein has an enhanced catalytic activity compared to the corresponding wild-type Cas protein. In some embodiments, the engineered Cas protein has an reduced catalytic activity compared to the corresponding wild-type Cas protein. In some embodiments, the engineered Cas protein has an increased gRNA binding compared to the corresponding wild-type Cas protein. In some embodiments, the engineered Cas protein has an decreased gRNA binding compared to the corresponding wild-type Cas protein. In some embodiments, the Cas protein has an enhanced specificity compared to the corresponding wild-type Cas protein. In some embodiments, the Cas protein has a reduceds pecificity compared to the corresponding wild-type Cas protein. In some embodiments, the Cas protein has an enhanced stability compared to the corresponding wild-type Cas protein. In some embodiments, the Cas protein has an reduced stability compared to the corresponding wild-type Cas protein. In some embodiments, the engineered Cas protein further comprises one or more mutations that inactivate catalytic activity. In some embodiments, the Cas protein has an increased off-target binding compared to the corresponding wild-type Cas protein. In some embodiments, the Cas protein has a decreased off-target binding compared to the corresponding wild-type Cas protein. In some embodiments, the Cas protein has an increased target binding compared to the corresponding wild-type Cas protein. In some embodiments, the Cas protein has a decreased target binding compared to the corresponding wild-type Cas protein. In some embodiments, the engineered Cas protein has a higher protease activity or polynucleotide binding ability as compared to the corresponding wild-type Cas protein. In some embodiments, PFS recognition is altered as compared to the corresponding wild-type Cas protein.

Examples of Cas proteins include class I (e.g., type I, type III, and type IV) and class 2 (e.g., type II, type V, and type VI) Cas proteins, e.g., Cas9, Cas12 (e.g., Cas12a, Cas12b, Cas12c, Cas12d), Cas13 (e.g., Cas13a, Cas13b, Cas13c, Cas13d), CasX, CasY, Cas14, variants thereof (e.g., mutant forms, truncated forms), homologs thereof, and orthologs thereof. The terms "orthologs" and "homologs" are well known in the art. By way of further guidance, as used herein, a "homolog" of a protein is a protein from the same species that performs the same or a similar function as the protein to which it is a homolog. Homologous proteins may be, but need not be, structurally related, or only partially structurally related. As used herein, an "ortholog" of a protein is a protein from a different species that performs the same or a similar function as the protein to which it is an ortholog. Orthologous proteins may be, but need not be, structurally related, or only partially structurally related.

In some embodiments, the Cas protein is a class 2 Cas protein, i.e., a Cas protein of a class 2 CRISPR-Cas system. The class 2 CRISPR-Cas system may have subtypes, such as type II-A, type II-B, type II-C, type V-A, type V-B, type V-C, or type V-U. In some embodiments, the Cas protein is Cas9, Cas12a, Cas12b, Cas12c, or Cas12d. In some embodiments, Cas9 may be SpCas9, SaCas9, StCas9, and other Cas9 orthologs. Cas12 may be Cas12a, Cas12b, and Cas12c, including FnCas12a or a homolog or ortholog thereof. Definitions and exemplary members of CRISPR-Cas systems include those described in Kira S. Makarova and Eugene V. Koonin, Annotation and Classification of CRISPR-Cas systems, Methods Mol Biol. 2015; 1311: 47-75 and Sergey Shmakov et al., Diversity and evolution of class 2 CRISPR-Cas systems, Nat Rev Microbial. March 2017; 15(3): 169-182.

In some examples, the Cas protein comprises at least one RuvC domain and at least one HNH domain. The Cas protein may further comprise a first and second linker domains linking the RuvC domain to the HNH domain. The first linker (L1) and the second linker (L2) linking the HNH and RuvC domains in Cas9 are described in Nishimasu, H. et al. "Crystal structure of Cas9 in complex with guide RNA and target RNA" Cell 156 (February 27, 2014): 935-949 and Ribeiro, L. et al. (2018) "Protein engineering strategies to expand CRISPR-Cas9 applications" International Journal of Genomics Volume 2018, Article ID 1652567 (doi.org/10.1155/2018/1652567). FIG. 1 of Ribeiro shows the overall organization, structure, and function of Cas9, which is specifically incorporated herein by reference. Specifically, FIG. 1A shows a schematic diagram of the domain organization of SpCas9, demonstrating the genetic structures of the HNH and RuvC domains including linkers L1 (spanning amino acids 765-780) and L2 (spanning amino acids 906-918) as described herein. Similarly, when referring to the first and second linker domains, the domain organization of Staphylococcus aureus Cas9 (SaCas9) can be utilized. In one aspect, the linker 1 domain region spans residues 481-519 and links the RuvC-II domain to the HNH domain in SaCas9. In some embodiments, the Linker 2 region spans residues 629-649 and links the RuvC-III domain to the HNH domain in SaCas9. Thus, the first and/or second linker domains can be mutated in Cas9 orthologs, and amino acid residues corresponding to amino acids of wild-type SaCas9 can be referenced. See, Nishimasu, Cell. August 27, 2015; 162(5): 1113- 1126; doi: 10.1016/j.cell.2015. 08.007, incorporated herein by reference. In particular, FIG. 1, S1-S3 of Nishimasu details the domain organization of the Cas9 protein, the teachings of which are specifically incorporated herein by reference. The first and second linkers may comprise about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 or more amino acids. The first and second linkers may correspond to wild-type linkers. In some aspects, the first and second linkers may comprise one or more mutations in the first and/or second linkers. In one aspect, the first and/or second linker comprises one or more mutations that increase the specificity of the Cas9 protein. In some embodiments, linkers L1 and L2 linking the HNH to the RuvC domain in Cas9 contain a wild-type amino acid sequence. In some embodiments, the linker linking the HNH to the RuvC domain contains a mutation in one or more amino acids. In embodiments, the first linker (L1) comprises a mutation corresponding to amino acid T769I of SpCas9, and/or the second linker (L2) comprises a mutation corresponding to amino acid G915M of SpCas9. In embodiments, one or more linker mutations, such as T769I and G915M, confer increased specificity to the Cas9 protein. In one embodiment, as described herein, one or more mutations in the first and second linkers can be combined with one or more mutations in other moieties of the Cas9 protein to further increase specificity and/or maintain activity substantially equivalent to the wild-type Cas9 protein. In one embodiment, mutations in the linker and/or additional mutations in the Cas protein can be identified using methods detailed herein that enhance/improve specificity for wild-type Cas9 and substantially retain the wild-type activity.

In some embodiments, the Cas protein may be a Cas protein of a class 2 type II CRISPR-Cas system (type II Cas protein). In some embodiments, the Cas protein may be a class 2 type II Cas protein, such as Cas9. In some embodiments, the CRISPR/Cas9-based system may include a Cas9 protein or a fragment thereof, a Cas9 fusion protein, a nucleic acid encoding a Cas9 protein or a fragment thereof, or a nucleic acid encoding a Cas9 fusion protein. "Cas9 (CRISPR-associated protein 9)" refers to a polypeptide or a fragment thereof having at least about 85% amino acid identity to NCBI Accession No. NP_269215 and having RNA binding activity, DNA binding activity, and/or DNA cleavage activity (e.g., endonuclease or nickase activity). The function of Cas9 can be defined by any of a variety of assays including, but not limited to, fluorescence polarization-based nucleic acid binding assays, fluorescence polarization-based strand invasion assays, transcription assays, EGFP disruption assays, DNA cleavage assays, and/or Surveyor assays. A "Cas9 nucleic acid molecule" refers to a polynucleotide encoding a Cas9 polypeptide or a fragment thereof. An exemplary Cas9 nucleic acid molecule sequence is provided under genomic SEQ ID NO: NC_002737. In some embodiments, disclosed herein are inhibitors of Cas9, such as Cas9 naturally occurring in Streptococcus pyogenes (SpCas9) or Staphylococcus aureus (SaCas9), or variants thereof. Cas9 recognizes foreign DNA by using the Protospacer Adjacent Motif (PAM) sequence and base pairing of guide RNA (gRNA) to target DNA. The relative ease with which Cas9 induces targeted strand breaks at any genomic locus enables highly efficient genome editing in a variety of cell types and organisms. Cas9 derivatives can also be used as transcriptional activators/repressors.

In some cases, the CRISPR-Cas protein is Cas9 or a variant thereof. In some examples, the Cas9 may be a wild-type Cas9 including any naturally occurring bacterial Cas9, may be in a codon-optimized or modified form, including any chimera, mutant, homolog, or ortholog. In another aspect of the present application, the Cas9 enzyme may comprise one or more mutations and may be used as a universal DNA binding protein fused to or not fused to a functional domain. The mutation may be an artificially introduced mutation or a gain-of-function or loss-of-function mutation. Other aspects of the present application relate to mutated Cas9 enzymes fused to domains including, but not limited to, nucleases, transcriptional activators, transcriptional repressors, recombinases, transposases, histone remodelers, demethylases, DNA methyltransferases, cryptochromes, photo-inducible/controllable domains, or chemically inducible/controllable domains. In some cases, the Cas9 enzyme may be from or derived from SpCas9 (Streptococcus pyogenes Cas9), saCas9 (Staphylococcus aureus Cas9), or StCas9 (wild-type Cas9 from Streptococcus thermophilus). As used herein, the term "derivative" with respect to an enzyme means that the derived enzyme is largely based on the sense of having a high degree of sequence homology to a wild-type enzyme, but has been mutated (modified) in some manner known in the art or described herein. In examples, the mutation may include one or more mutations in the first linker domain, the second linker domain, and/or other moieties of the protein. A high degree of sequence homology can include at least 80%, 85%, 86%, 87%, 88%, 89%, 90%, 91%, 92%, 93%, 94%, 95%, 96%, 97%, 98%, 99% or more relative to the wild-type enzyme.

In particular embodiments, the CRISPR enzyme may be a Cas9 protein from or derived from an organism of the genus comprising: Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacte, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium or Acidaminococcus, Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacte, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma or Campylobacter.

In some embodiments, the CRISPR enzyme may be a Cas9 protein from or derived from an organism including: S. mutans, S. agalactiae, S. equisimilis, S. sanguinis, S. pneumonia, Campylobacter jejuni, C. Coli; Nitratifractor salsuginis, N tergarcus; S. auricularis, S. carnosus; Neisseria meningitidis, N gonorrhoeae, L. monocytogenes, L.ivanovii; C. botulinum, C. difficile, C. tetani or C. sordellii, Francisella tularensis 1, Francisella tularensis subsp. novicida, Prevotella albensis, Lachnospiraceae bacterium MC2017 1, Butyrivibrio proteoclasticus, Peregrinibacteria bacterium GW2011 GWA2_33_10, Parcubacteria bacterium GW2011 GWC2_44_17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens and Porphyromonas macacae. In some embodiments, the Cas9 protein is from an organism that is from or derived from Streptococcus pyogenes, Staphylococcus aureus, or Streptococcus thermophilus Cas9.

In a more preferred embodiment, the Cas9 protein is derived from a bacterial species selected from Streptococcus pyogenes, Staphylococcus aureus or Streptococcus thermophilus Cas9. In certain embodiments, Cas9 is derived from a bacterial species selected from Francisella tularensis 1, Prevotella albensis, Lachnospiraceae bacterium MC20171, Butyrivibrio proteoclasticus, Peregrinibacteria bacterium GW2011 GWA2 33 JO, Parcubacteria bacterium GW2011 GWC2_44_17, Smithella sp. SCADC, Acidaminococcus sp. BV3L6BV3L6, Lachnospiraceae bacterium MA2020, Candidatus Methanoplasma termitum, Eubacterium eligens, Moraxella bovoculi 237237, Leptospira inadai, Lachnospiraceae bacterium ND2006, Porphyromonas crevioricanis 3, Prevotella disiens and Porphyromonas macacae. In certain embodiments, the Cas9 protein is derived from a bacterial species selected from Acidaminococcus sp. BV3L6, Lachnospiraceae bacterium MA2020. In certain embodiments, the effector protein is derived from a subspecies of Francisella tularensis 1, including, but not limited to, Francisella tularensis subsp. novicida.

Cas9 proteins include, but are not limited to, Streptococcus pyogenes M1 serotype (UniProt ID: Q99ZW2), Staphylococcus aureus Cas9 (UniProt ID: J7RUA5), *Eubacterium ventriosum* Cas9 (UniProt ID: A5Z395), Azospirillum (strain B510) Cas9 (UniProt ID: D3NT09), Gluconacetobacter diazotrophicus (strain ATCC 49037) Cas9 (UnitProt ID: A9HKP2), Neisseria cinerea Cas9 (UniProt ID: D0W2Z9), Roseburia intestinalis Cas9 (UniProt ID: C7G697), Parvibaculum lavamentivorans (strain DS-1)Cas9 (UniProt ID: A7HP89), Nitratifractor salsuginis (strain DSM 16511) Cas9 (UniProt ID: E6WZS9), Campylobacter lari Cas9 (UniProt ID: G1UFN3).

Enzymatic action of Cas9 derived from Streptococcus pyogenes or any closely related Cas9 generates a double-stranded break on a target site sequence that hybridizes to 20 nucleotides of the guide sequence and has a protospacer sequence adjacent motif (PAM) sequence (examples include NGG/NRG, which can be determined as described herein) that is 20 nucleotides after the target sequence. CRISPR activity, which involves site-specific DNA recognition and cleavage by Cas9, is defined by a guide sequence, a tracr sequence that partially hybridizes to the guide sequence, and a PAM sequence. Further aspects of the CRISPR system are described in Karginov and Hannon, The CRISPR system: small RNA-guided defense in bacteria and archaea, Mole Cell January 15 2010; 37(1):7. The type II CRISPR locus from Streptococcus pyogenes SF370 contains a cluster of four genes, Cas9, Cas1, Cas2, and Csnl, as well as two non-coding RNA elements, tracrRNA, and a characteristic array of repeat sequences (direct repeats) separated by short segments of non-repetitive sequence (spacers, approximately 30 bp each). In this system, targeted DNA double-strand breaks (DSBs) are generated in four consecutive steps. First, two non-coding RNAs (pre-crRNA array and tracrRNA) are transcribed from the CRISPR locus. Second, tracrRNA hybridizes to the direct repeat sequence of pre-crRNA and is then processed into mature crRNA containing a single spacer sequence. Third, the mature crRNA:tracRNA complex is formed by heteroduplex formation between the crRNA spacer and the protospacer DNA, directing Cas9 to a DNA target consisting of the protospacer sequence and the corresponding PAM. Finally, Cas9 mediates cleavage of the target DNA upstream of the PAM to generate a DSB within the protospacer. In certain embodiments, Cas9 may be constitutively present or inducibly present or conditionally present or administered or delivered. Cas9 optimization can be used to enhance function or develop new functions. A chimeric Cas9 protein can be produced, and Cas9 can be used as a universal DNA binding protein. The structural information provided for Cas9 can be used to further engineer and optimize the CRISPR-Cas system, and this can also be used to infer structure-function relationships in other CRISPR enzyme systems, particularly in other type II CRISPR enzymes or Cas9 orthologs. Furthermore, the Cas9 protein comprises a readily identifiable C-terminus region that is homologous to the transposon ORF-B and includes an active RuvC-like nuclease (an arginine-rich region).

In the present application, the term "gene expression modulator" may generally be selected from a repressor of gene expression (e.g., KRAB), an activator of gene expression, or a modulator of epigenetic modification (e.g., DNMT3A, DNMT3L, a DNMT3A-DNMT3L fusion peptide), or any combination thereof. Various gene expression modulators are known in the art, see, for example, Thakore et al., Nat Methods. 2016; 13: 127-37, which is incorporated herein by reference in its entirety.

In some embodiments, the gene expression modulator comprises a repressor of gene expression. The repressor may be any known repressor of gene expression, for example, may be selected from Krüppel-associated box (KRAB) domain, mSin3 interaction domain (SID), MAX interaction protein 1 (MXI1), chromo shadow domain, ear repressor domain (SRDX), eukaryotic release factor 1 (ERFl), eukaryotic release factor 3 (ERF3), tetracycline repressor, lad repressor, Catharanthus roseus G-box binding factors 1 and 2, Drosophila Groucho, triplet motif 28 (TRTM28), nuclear receptor corepressor 1, nuclear receptor corepressor 2, or fragments or fusions of the above. For example, the Krüppel-associated box (KRAB) domain is a type of transcriptional repression domain that is present in the N-terminus portion of many zinc finger protein-based transcription factors. The KRAB domain functions as a transcriptional repressor when tethered to a target DNA through a DNA binding domain. The KRAB domain is rich in charged amino acids and can be divided into subdomains A and B. The KRAB A and B subdomains can be separated by a variable spacer segment, and many KRAB proteins contain only the A subdomain. A 45-amino acid sequence in the KRAB A subdomain has been shown to be important for transcriptional inhibition. The B subdomain itself does not inhibit transcription, but reinforces the inhibition exerted by the KRAB A subdomain. The KRAB domain recruits the corepressor KAP1 (KRAB-associated protein-1, also known as transcription intermediary factor 1 beta, KRAB-A interaction protein, and triplet motif protein 28) and heterochromatin protein 1 (HP1), as well as other chromatin regulatory proteins, leading to transcriptional inhibition through heterochromatin formation. In some embodiments, the methods and compositions provided herein comprise a fusion molecule comprising dCas9 fused to a KRAB domain or a fragment thereof. In some embodiments, the KRAB domain or fragment thereof is fused to the N-terminus of dCas9. In some embodiments, the KRAB domain or fragment thereof is fused to the C-terminus of dCas9. In one embodiment, the KRAB domain or fragment thereof is fused to both the N-terminus and the C-terminus of the dCas9 molecule. In some embodiments, the fusion molecule comprises a KRAB domain comprising a sequence as set forth in SEQ ID NO: 1168, a sequence substantially identical (e.g., at least 80%, 85%, 90%, 92%, 95%, 97%, 98%, 99% or more identical) to SEQ ID NO: 1168, or a sequence having 1, 2, 3, 4, 5 or more alterations (e.g., amino acid substitutions, insertions, or deletions) relative to SEQ ID NO: 1160, or any fragment thereof. In some embodiments, the zinc finger protein-based transcription factor is a KRAB domain found in many Krüppel-type C2H2 zinc finger proteins, e.g., it is a KRAB domain derived from ZIM3 (ZIM3 KRAB). For example, the fusion molecule comprises a ZIM3 KRAB domain comprising a sequence as set forth in SEQ ID NO: 1196, a sequence substantially identical (e.g., at least 80%, 85%, 90%, 92%, 95%, 97%, 98%, 99% or more identical) to SEQ ID NO: 1196, or a sequence having 1, 2, 3, 4, 5 or more alterations (e.g., amino acid substitutions, insertions, or deletions) relative to SEQ ID NO: 1196, or any fragment thereof. Active fragments of other KRAB domains can be identified by any suitable alignment method in the art.

In some embodiments, the gene expression modulator comprises an activator of gene expression. The activator may be any known activator of gene expression, for example, a VP16 activation domain, a VP64 activation domain, a p65 activation domain, an Epstein-Barr virus R transactivator Rta molecule, or a fragment of the above. Activation that can be used for dCas9 is known in the art. See, for example, Chavez et al., Nat Methods. (2016) 13: 563-67, incorporated herein by reference in its entirety. In some embodiments, the methods and compositions provided herein comprise a fusion molecule comprising dCas9 fused to VP64, p65, Rta, or any combination thereof. The triplet activator VP64-p65-Rta (also known as VPR), in which three transcriptional activation domains are fused using a short amino acid linker, can efficiently upregulate target gene expression when fused to dCas9. In some embodiments, the methods and compositions provided herein comprise a fusion molecule comprising dCas9 fused to a VPR.

In some embodiments, the methods and compositions provided herein comprise a fusion molecule comprising dCas9 fused to a gene expression modulator, and the gene expression modulator comprises an epigenetic modification modulator. In some embodiments, the fusion molecule modulates the expression of the target gene at a regulatory element (e.g., a promoter, enhancer, or transcription initiation site) of the target gene by epigenetic modification, e.g., by histone acetylation or methylation, or DNA methylation. The modulator may be any known modulator of epigenetic modification, e.g., histone acetyltransferase (e.g., p300 catalytic domain), histone deacetylase, histone methyltransferase (e.g., SUV39H1 or G9a (EHMT2)), histone demethylase (e.g., LSD1), DNA methyltransferase (e.g., DNMT3A or DNMT3A-DNMT3L), DNA demethylase (e.g., TET1 catalytic domain or TDG), or a fragment of the above.

In certain embodiments, the epigenetic modification regulator may have histone modification activity; the histone modification activity may include but is not limited to histone deacetylase activity, histone acetyltransferase activity, histone demethylase activity or histone methyltransferase activity. For example, the epigenetic modification regulator may have histone acetyltransferase activity, and the histone acetyltransferase may be p300 or CREB binding protein (CBP) protein or a fragment thereof. In some embodiments, the methods and compositions provided herein comprise a fusion molecule comprising dCas9 fused to acetyltransferase p300 or a fragment thereof (e.g., the catalytic core of p300). In some embodiments, the methods and compositions provided herein comprise a fusion molecule comprising dCas9 fused to a CREB binding protein (CBP) or a fragment thereof. For another example, the epigenetic modification modulator may have histone demethylase activity. In some embodiments, the epigenetic modification modulator may comprise an enzyme that removes a methyl (CH3 -) group from a nucleic acid or protein (e.g., histone). In some embodiments, the methods and compositions provided herein comprise a fusion molecule comprising dCas9 fused to Lys-specific histone demethylase 1 (LSD1) or a fragment thereof. As another example, the epigenetic modification modulator may have histone methyltransferase activity. In some embodiments, the methods and compositions provided herein comprise a fusion molecule comprising dCas9 fused to SUV39H1 or a fragment thereof. In some embodiments, the methods and compositions provided herein comprise a fusion molecule comprising dCas9 fused to G9a (EHMT2) or a fragment thereof.

In certain embodiments, the epigenetic modification modulator may have DNA demethylase activity. In some embodiments, the epigenetic modification modulator can convert methyl to hydroxymethylcytosine as a mechanism to demethylate DNA. In some embodiments, the methods and compositions provided herein comprise a fusion molecule comprising dCas9 fused to a 10-11 translocation methylcytosine dioxygenase 1 (TET1) or a fragment thereof. In some embodiments, the methods and compositions provided herein comprise a fusion molecule comprising dCas9 fused to a thymine DNA glycosylase (TDG) or a fragment thereof. As another example, the epigenetic modification modulator may have DNA methylase activity. In some embodiments, the epigenetic modification modulator may have methylase activity involving the transfer of methyl to DNA, RNA, protein, small molecule, cytosine, or adenine. In some embodiments, the methods and compositions provided herein comprise a fusion molecule comprising dCas9 fused to DNMT3A or a fragment thereof. In some embodiments, the methods and compositions provided herein comprise a fusion molecule comprising dCas9 fused to DNMT3L or a fragment thereof. In some embodiments, the methods and compositions provided herein comprise a fusion molecule comprising dCas9 fused to DNMT3L and DNMT3L or fragments thereof. In some embodiments, the methods and compositions provided herein comprise a fusion molecule comprising dCas9 fused to a DNMT3A-DNMT3L fusion peptide. In some embodiments, the epigenetic modification modulator having DNA methylase activity is human-derived DNMT3L comprising an amino acid sequence as set forth in SEQ ID NO: 1195.

In the present application, the term "guide RNA (gRNA)" is used interchangeably with "guide molecule", "guide sequence" and "single guide RNA (sgRNA)", which in the context of a CRIPR-Cas system generally includes any polynucleotide sequence that has sufficient complementarity to a target DNA sequence to hybridize to the target DNA sequence and direct a nucleic acid targeting complex (e.g., a composition described herein) to specifically bind to the target DNA sequence. The guide RNA may form a duplex with the target DNA sequence. In some embodiments, the guide RNA is capable of forming a complex with the CRISPR-Cas protein and comprises a guide sequence having sufficient complementarity to the target DNA sequence to hybridize to the target DNA sequence and directing sequence-specific binding of the complex to the target DNA sequence. The guide molecule or guide RNA of the CRISPR-Cas protein may comprise a tracr-mate sequence ("comprising a direct repeat sequence" in the case of an endogenous CRISPR system) and a guide sequence (also referred to as a "spacer" in the case of an endogenous CRISPR system). In some embodiments, the CRISPR-Cas system or complex described herein does not comprise a tracr sequence and/or is independent of the presence of a tracr sequence. In certain embodiments, the guide molecule may comprise, consist essentially of, or consist of a direct repeat sequence fused or linked to a guide sequence or a spacer sequence. In general, a CRISPR-Cas system is characterized by an element that promotes formation of a CRISPR complex at the site of a target DNA sequence, wherein hybridization between the target DNA sequence and a guide sequence promotes formation of the CRISPR complex.

In certain embodiments, the guide sequence or spacer of the guide molecule is 15 to 50 nucleotides in length. In certain embodiments, the spacer of the guide RNA is at least 15 nucleotides in length. In certain embodiments, the spacer is 15 to 17 nucleotides in length, 17 to 20 nucleotides in length, 20 to 24 nucleotides in length, 23 to 25 nucleotides in length, 24 to 27 nucleotides in length, 27 to 30 nucleotides in length, 30 to 35 nucleotides in length, or more than 35 nucleotides in length. In some embodiments, the guide sequence is 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nucleotides in length.

In some embodiments, the sequence of the guide molecule (direct repeat sequence and/or spacer) is selected to reduce the degree of secondary structure within the guide molecule. In some embodiments, when optimally folded, about or less than about 75%, 50%, 40%, 30%, 25%, 20%, 15%, 10%, 5%, 1% or less of the nucleotides of a nucleic acid-targeting guide RNA participate in self-complementary base pairing. Optimal folding can be determined by any suitable polynucleotide folding algorithm. Some programs are based on calculating the minimum Gibbs free energy. An example of such an algorithm is mFold, as described by Zuker and Stiegler (Nucleic Acids Res9 (1981), 133-148). Another exemplary folding algorithm is the online web server RNAfold developed by the Institute of Theoretical Chemistry of the University of Vienna, which uses a centroid structure prediction algorithm (see, for example, A.R. Gruber et al., 2008, Cell 106 (1): 23-24 and PA Carr and GM Church, 2009, Nature Biotechnology 27 (12): 1151-62).

As described above, the CRISPR/Cas9 system utilizes gRNA to provide targeting of the CRISPR/Cas9-based system. gRNA is a fusion of two non-coding RNAs:crRNA and tracrRNA. The sgRNA can be targeted to any desired DNA sequence by exchanging a sequence encoding a 20-bp protospacer sequence that confers targeting specificity through complementary base pairing with the desired DNA target. The gRNA mimics the naturally occurring crRNA:tracrRNA duplex involved in the type II effector system. This duplex, which may include, for example, a 42-nucleotide crRNA and a 75-nucleotide tracrRNA, serves as a guide for Cas9 to cleave a target nucleic acid.

In another aspect, the sgRNA provided herein may further comprise a scaffold sequence having a nucleotide sequence as set forth in SEQ ID NO: 1190; or a nucleotide sequence having at least 90%, at least 91%, at least 92%, at least 93%, at least 94%, at least 95%, at least 96%, at least 97%, at least 98%, at least 99%, at least 99.9%, or at least 100% sequence identity to and retaining the biological activity of the nucleotide sequence as set forth in SEQ ID NO: 1190; or a nucleotide sequence engineered based on and retaining the biological activity of the nucleotide sequence as set forth in SEQ ID NO: 1190. For example, the engineering may be one or more of base phosphorylation, base sulfidation, base methylation, base hydroxylation, sequence shortening, and sequence lengthening. Further, the sequence shortening and the sequence lengthening may comprise deletions or additions of 1, 2, 3, 4, 5, 6, 7, 8, 9, or 10 bases relative to the reference sequence.

As an example only, the scaffold sequence may be:

NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGCUUUU (SEQ ID
NO: 1190).

In some embodiments, the sgRNA provided herein may further include the CRISPR spacer sequence at the 5' end of the scaffold sequence, wherein the CRISPR spacer sequence is a sequence having a length of 20, 21, 22, 23, 24, 25, 26, 27, 28, 29, or 30 nucleotides and capable of complementary pairing with the target sequence. In some preferred embodiments, the CRISPR spacer sequence is a sequence having a length of 20 or 21 nucleotides and capable of complementary pairing with the target sequence. In some embodiments, the sgRNA may further comprise a terminator at the 3' end of the spacer sequence. For example, the terminator may be a terminator composed of multiple Us, such as at least 6 (e.g., 7 or 8) Us.

In the present application, the terms "target DNA" and "target sequence" or "protospacer sequence" are used interchangeably and generally refer to a nucleotide sequence present in a target nucleic acid comprising a nucleobase sequence complementary to an oligonucleotide (e.g., a guide RNA) of the present application. In certain instances, the target sequence consists of a region on a target nucleic acid that is complementary to a contiguous nucleotide sequence of an oligonucleotide of the present application. In some instances, the target sequence is longer than the complementary sequence of a single oligonucleotide, and can, for example, represent available regions of a target nucleic acid that can be targeted by several oligonucleotides of the present application. In certain instances, a "target sequence" may mean a portion of a target gene, such as one or more exon sequences, intron sequences, or regulatory sequences of a target gene, or a combination of exon and intron sequences, a combination of intron and regulatory sequences, a combination of exon and regulatory sequences, or a combination of exon, intron, and regulatory sequences of a target gene. In the context of the formation of a CRISPR complex or system of the present application, "target DNA" refers to a sequence to which a guide RNA sequence is designed to be complementary, wherein hybridization between the target DNA and the guide RNA sequence promotes the formation of the CRISPR complex or system. In some embodiments, the target DNA is located in the nucleus or cytoplasm of a cell. The CRISPR/Cas9-based system can include at least one gRNA, wherein the gRNAs target different DNA sequences. The target DNA sequences may be overlapping. The target sequence or protospacer sequence is followed by a PAM sequence located at the 3' end of the protospacer sequence. Different Type II systems have different PAM requirements. For example, the Streptococcus pyogenes type II system uses an "NGG" sequence, where "N" can be any nucleotide.

In some embodiments, the number of gRNAs administered to the cells can be at least 1 gRNA, at least 2 different gRNAs, at least 3 different gRNAs, at least 4 different gRNAs, at least 5 different gRNAs, at least 6 different gRNAs, at least 7 different gRNAs, at least 8 different gRNAs, at least 9 different gRNAs, at least 10 different gRNAs, at least 11 different gRNAs, at least 12 different gRNAs, at least 13 different gRNAs, at least 14 different gRNAs, at least 15 different gRNAs, at least 16 different gRNAs, at least 17 different gRNAs, at least 18 different gRNAs, at least 19 different gRNAs, at least 20 different gRNAs, at least 25 different gRNAs, at least 30 different gRNAs, at least 35 different gRNAs, at least 40 different gRNAs, at least 45 different gRNAs, or at least 50 different gRNAs.

In some embodiments, the number of gRNAs administered to a cell can be at least 1 gRNA to at least 50 different gRNAs, at least 1 gRNA to at least 45 different gRNAs, at least 1 gRNA to at least 40 different gRNAs, at least 1 gRNA to at least 35 different gRNAs, at least 1 gRNA to at least 30 different gRNAs, at least 1 gRNA to at least 25 different gRNAs, at least 1 gRNA to at least 20 different gRNAs, at least 1 gRNA to at least 16 different gRNAs, at least 1 gRNA to at least 12 different gRNAs, at least 1 gRNA to at least 8 different gRNAs, at least 1 gRNA to at least 4 different gRNAs, at least 4 different gRNAs to at least 50 different gRNAs, at least 4 different gRNAs to at least 45 different gRNAs, at least 4 different gRNAs to at least 40 different gRNAs, at least 4 different gRNAs to at least 35 different gRNAs, at least 4 different gRNAs to at least 30 different gRNAs, at least 4 different gRNAs to at least 25 different gRNAs, at least 4 different gRNAs to at least 20 different gRNAs, at least 4 different gRNAs to at least 16 different gRNAs, at least 4 different gRNAs to at least 12 different gRNAs, at least 4 different gRNAs to at least 8 different gRNAs, at least 8 different gRNAs to at least 50 different gRNAs, at least 8 different gRNAs to at least 45 different gRNAs, at least 8 different gRNAs to at least 40 different gRNAs, at least 8 different gRNAs to at least 35 different gRNAs, 8 different gRNAs to at least 30 different gRNAs, at least 8 different gRNAs to at least 25 different gRNAs, 8 different gRNAs to at least 20 different gRNAs, at least 8 different gRNAs to at least 16 different gRNAs or 8 different gRNAs to at least 12 different gRNAs. In some embodiments, the transcription of the target gene is increased or decreased by selecting the gRNA.

As used herein, the term "regulatory element" refers to a genetic element capable of controlling the expression of a nucleic acid sequence. For example, a splicing signal, a promoter sequence, a polyadenylation signal, a transcription termination sequence, an upstream regulatory domain, an origin of replication, an internal ribosome entry site ("IRES"), an enhancer, and the like, which collectively provide for the replication, transcription, and translation of the coding sequence in a recipient cell. Not all of these control sequences need to be present. Transcriptional control signals in eukaryotes typically contain "promoter" and "enhancer" elements. Promoters and enhancers consist of short arrays of DNA sequences, with promoters being regulatory elements that promote the initiation of transcription of an operably linked coding region and enhancers being regulatory elements that increase the rate of genetic transcription by increasing the activity of the closest promoter located on the same DNA molecule, all these sequences specifically interact with cellular proteins involved in transcription (Maniatis et al., Science 236:1237 (1987), incorporated herein by reference in its entirety). Promoter and enhancer elements have been isolated from a variety of eukaryotic sources, including genes in yeast cells, insect cells, mammalian cells, and viruses (similar control sequences, known as promoters, are also found in prokaryotes). The choice of specific promoters and enhancers depends on the recipient cell type. Some eukaryotic promoters and enhancers have a broad host range, while others are functional in a limited subset of cell types (for reviews, see, e.g., Voss et al., Trends Biochem. Sci., 11:287 (1986); and Maniatis et al., supra, incorporated herein by reference in their entireties). For example, the SV40 early gene enhancer is very active in a variety of cell types from many mammalian species and has been used to express proteins in a variety of mammalian cells (Dijkema et al., EMBO J. 4:761 (1985), incorporated herein by reference in its entirety). Promoter and enhancer elements derived from the human elongation factor 1-alpha gene (Uetsuki et al., J. Biol. Chem., 264:5791 (1989); Kim et al., Gene 91:217 (1990); and Mizushima and Nagata, Nucl. Acids. Res., 18:5322 (1990)), the long terminal repeat of Rous sarcoma virus (Gorman et al., Proc. Natl. Acad. Sci. U.S.A. 79:6777 (1982)), and human cytomegalovirus (Boshart et al., Cell 41:521 (1985)), which references are incorporated herein by reference in their entireties, can also be used to express proteins in various mammalian cell types. Promoters and enhancers can occur naturally separately or together. For example, the retroviral long terminal repeat comprises both promoter and enhancer elements. In general, promoters and enhancers function independently of the gene being transcribed or translated. Thus, the enhancers and promoters used may be "endogenous," "exogenous," or "heterologous" relative to the gene to which they are operably linked. An "endogenous" enhancer/promoter is one that is naturally linked to a given gene in the genome. An "exogenous" or "heterologous" enhancer or promoter is one that is placed in juxtaposition with a gene via genetic manipulation (i.e., molecular biology techniques), with this juxtaposition enabling the transcription of the gene to be directed by the linked enhancer/promoter. The presence of a "splicing signal" on an expression vector typically results in high levels of expression of the recombinant transcript.

In certain embodiments, a "splicing signal" mediates the removal of introns from a primary RNA transcript and consists of a splice donor and acceptor site (Sambrook et al., Molecular Cloning: A Laboratory Manual, 2nd ed., Cold Spring Harbor Laboratory Press, New York (1989), pp. 16.7-16.8, incorporated herein by reference in its entirety). Commonly used splice donor and acceptor sites are the splice sites of the 16S RNA from SV40.

In certain embodiments, a "transcription termination signal" is typically present downstream of the polyadenylation signal and is several hundred nucleotides in length. For example, the term "poly A signal" or "poly A sequence" refers to a DNA sequence that directs the termination and polyadenylation of a nascent RNA transcript. Efficient polyadenylation of recombinant transcripts is often necessary because transcripts lacking a poly A signal are unstable and rapidly degraded. The poly A signal used in an expression vector can be either "heterologous" or "endogenous." An endogenous poly A signal is a signal that is naturally present at the 3' end of the coding region of a given gene in the genome. A heterologous poly A signal is a signal that is isolated from one gene and operably linked to the 3' end of another gene. A commonly used heterologous poly A signal is the SV40 poly A signal. The SV40 poly A signal is contained on the 237 bp BamHI/Bcll restriction fragment and directs termination and polyadenylation (Sambrook et al., supra, 16.6-16.7, incorporated by reference in its entirety).

In the present application, the term "inactivated Cas9 protein" may be referred to as a "dCas9" protein. Known methods for generating Cas9 proteins (or fragments thereof) with inactivated DNA cleavage domains are described, for example, in Jinek et al., Science. 337: 816-821 (2012); Qi et al., "Repurposing CRISPR as an RNA-GuidedPlatform for Sequence-Specific Control of Gene Expression", Cell. 28, 152(5): 1173-83 (2013), the entire contents of each of which are incorporated herein by reference). For example, it is known that the DNA cleavage domain of Cas9 includes two subdomains, the HNH nuclease subdomain and the RuvC1 subdomain. The HNH subdomain cleavages the strand complementary to the gRNA, while the RuvC1 subdomain cleaves the non-complementary strand. Mutations in these subdomains can silence the nuclease activity of Cas9. For example, mutations D10A and H840A completely inactivate the nuclease activity of Streptococcus pyogenes Cas9 (Jinek et al., Science. 337:816-821 (2012); Qi et al., Cell. 28;152(5):1173-83 (2013)). Suitable CRISPR inactivating or nicking DNA-binding domains include, but are not limited to, nuclease-inactive variant Cas9 domains including D10A, D10A/D839A/H840A and D10A/D839A/H840A/N863A mutant domains as described in WO2015089406A1, which is incorporated herein by reference. In some cases, the endonuclease-inactive dCas9 from Streptococcus pyogenes has been targeted to genes in bacteria, yeast, and human cells by gRNA to silence gene expression through steric hindrance. As used herein, "dCas" may refer to a dCas protein or a fragment thereof. As used herein, "dCas9" may refer to a dCas9 protein or a fragment thereof. As used herein, the terms "iCas" and "dCas" are used interchangeably to refer to a catalytically inactive CRISPR-associated protein. In one embodiment, the dCas protein comprises one or more mutations in DNA cleavage domain. In one embodiment, the dCas protein comprises one or more mutations in RuvC or domain. In one embodiment, the dCas molecule comprises one or more mutations in both RuvC and HNH domains. In one embodiment, the dCas protein is a fragment of a wild-type Cas protein. In one embodiment, the dCas protein comprises a function domain from a wild-type Cas protein, wherein the function domain is selected from a Reel domain, a bridge helix domain, or a PAM interaction domain. In one embodiment, the nuclease activity of the dCas is reduced by at least 40%, 45%, 50%, 55%, 60%, 65%, 70%, 75%, 80%, 85%, 90%, 95% or 99% as compared to the nuclease activity of the corresponding wild-type Cas protein.

Suitable dCas can be derived from wild-type Cas proteins. The Cas protein can be from a type I, type II, or type III CRISPR-Cas system. In one embodiment, a suitable dCas may be derived from Cas1, Cas2, Cas3, Cas4, Cas5, Cas6, Cas7, Cas8, Cas9, or Cas 10. In one embodiment, the dCas is derived from a Cas9 protein. For example, dCas9 can be obtained by introducing point mutations (e.g., substitutions, deletions or additions) in the DNA cleavage domain (e.g., the nuclease domain, such as RuvC and/or HNH domains) of the Cas9 protein. See, e.g., Jinek et al., Science (2012) 337:816-21, incorporated herein by reference in its entirety. For example, the introduction of two point mutations in the RuvC and HNH domains reduced Cas9 nuclease activity while retaining Cas9 sgRNA- and DNA-binding activities. In one embodiment, the two point mutations within the RuvC and HNH active sites are the D10A and H840A mutations of Streptococcus pyogenes Cas9. Alternatively, D10 and H840 of Streptococcus pyogenes Cas9 can be deleted to eliminate Cas9 nuclease activity while retaining its sgRNA-and DNA-binding activities. In one embodiment, the two point mutations within the RuvC and HNH active sites are the D10A and N580A mutations of Streptococcus pyogenes Cas9.

In various embodiments, the present application relates to a dCas protein or any variant or mutant thereof. All variants and mutants of dCas9 can be used in the methods, compositions, fusion molecules or kits disclosed herein, and include but not limited to those variants and mutants derived from SpCas9 (Cas9 isolated from Streptococcus pyogenes), SaCas9 (Cas9 isolated from Staphylococcus aureus), StCas9 (Cas9 isolated from Streptococcus thermophilus), NmCas9 (Cas9 isolated from Neisseria meningitidis), FnCas9 (Cas9 isolated from Francisella novicida), CjCas9 (Cas9 isolated from Campylobacter jejuni), ScCas9 (Cas9 isolated from Streptococcus canis), and any variants and mutant forms of Cas9 listed above, such as high-fidelity Cas9 (Kleinstiver et al., Nature. January 28, 2016) and enhanced SpCas9 (Slaymaker et al., Sciences. January 1, 2016). For example, the dCas9 sequences as set forth in SEQ ID NOs: 1170-1187 of the present application only provide a few exemplary options and are not exclusive. In one embodiment, the dCas protein is a Streptococcus pyogenes dCas9 protein comprising a mutation at D10 and/or H840 (as set forth in SEQ ID NO: 1170). In one embodiment, the dCas protein is a Streptococcus pyogenes dCas9 protein comprising the D10A and/or H840A mutations (as set forth in SEQ ID NO: 1170). In one embodiment, the dCas9 protein is a Staphylococcus aureus dCas9 protein comprising an amino acid sequence as set forth in any one of SEQ ID NOs: 1171-1173, a sequence substantially identical (e.g., at least 80%, 85%, 90%, 91%, 92%, 93%, 94%, 95%, 96%, 97%, 98%, 99% or more sequence identity) to any one of SEQ ID NOs: 1171-1173, or a sequence having 1, 2, 3, 4, 5 or more alterations (e.g., amino acid substitutions, insertions or deletions) relative to any one of SEQ ID NOs: 1171-1173, or any fragment thereof.

Similar mutations can also be applied to any other naturally occurring Cas9 (e.g., Cas9 from other species) or engineered Cas9. In certain embodiments, the dCas9 comprises Streptococcus pyogenes dCas9, Staphylococcus aureus dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (e.g., strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (e.g., strain DSM 16511) dCas9, Campylobacter lari (e.g., strain CF89-12) dCas9, Streptococcus thermophilus (e.g., strain LMD-9) dCas9, or fragments of the above. In certain embodiments, the present application further provides a vector comprising a nucleotide encoding the following protein molecules: Streptococcus pyogenes dCas9, Staphylococcus aureus dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (strain DSM 16511) dCas9, Campylobacter lari (strain CF89-12) dCas9, Streptococcus thermophilus (strain LMD-9) dCas9, or fragments of the above.

In the present application, the term "modification of nucleotides" may mean that the nucleic acids described in the present invention are synthesized or modified by methods well-established in the art, such as those described in "Current protocols innucleic acid chemistry" Beaucage, S.L. et al., (Edrs.), John Wiley & Sons, Inc., NewYork, NY, USA, which is hereby incorporated by reference. Such modifications may include, but are not limited to: terminal modifications such as 5'-terminal modifications (e.g., phosphorylation, conjugation, inverted linkage) or 3'-terminal modifications (e.g., conjugation, DNA nucleotide, inverted linkage, etc.); base modifications, such as substitution with stabilized bases, destabilized bases, or bases that are paired with bases of an expanded pairing repertoire, removal of bases (abasic nucleotides), or conjugated bases; sugar modifications (e.g., sugar modifications at the 2'-position or the 4'-position) or substitution of sugars; or backbone modifications, including modification or substitution of phosphodiester linkages.

In the present application, the terms "DNA methylation" and "nucleic acid methylation" are used interchangeably, and generally refer to the methylation state of gene fragments, nucleotides or their bases in the present application, which is a process that often occurs inside cells that have been transfected with nucleic acids, wherein the cells have been transfected with nucleic acids containing a structural gene encoding a polypeptide that is operably linked to a promoter, the cytosine of the promoter nucleic acid is converted to 5-methylcytosine during the process. The promoter nucleic acid in which at least one cytosine is converted to 5-methylcytosine is referred to as a "methylated" nucleic acid or DNA. In the present application, the DNA fragment where the gene is located may have methylation on one strand or multiple strands, and may also have methylation at one site or multiple sites.

In the present application, the term "portion thereof" generally refers to a portion or fragment of a designated whole. For example, when used in the present application with respect to a specified polypeptide sequence, the term "portion thereof" refers to a contiguous length of a sequence of a specified polypeptide that is shorter than the full-length sequence of the specified polypeptide. A portion of a specified polypeptide can be defined by its first position and its last position, wherein the first and last position each correspond to a position in the sequence of the specified polypeptide, wherein the sequence position corresponding to the first position is located N-terminally to the sequence position corresponding to the last position, and whereby the sequence of the portion is a contiguous sequence of amino acids in the specified polypeptide that begins at the sequence position corresponding to the first position and ends at the sequence position corresponding to the last position. A portion can also be defined by reference to a position in a specified polypeptide sequence and a length of residues relative to the reference position, whereby the sequence of the portion is a contiguous amino acid sequence in the specified polypeptide having a defined length and positioned in the specified polypeptide according to the defined position.

In the present application, the term "direct or indirect fuse" generally refers to the relative "direct fuse" or "indirect fuse". The term "directly fuse" generally refers to being directly linked or directly associated. For example, the direct linking may be a case where the linked substances (e.g., amino acid sequence segments) are directly linked without any spacer component (e.g., an amino acid residue or a derivative thereof); for example, an amino acid sequence segment X is directly linked to another amino acid sequence segment Y via an amide bond formed by the C-terminal amino acid of the amino acid sequence segment X and the N-terminal amino acid of the amino acid sequence segment Y. "Indirectly fuse" generally refers to a case where the linked substances (e.g., amino acid sequence segments) are indirectly linked, and there is a spacer component (e.g., an amino acid residue or a derivative thereof) between them.

In the present application, the vectors used to package the compositions, fusion molecules and/or guide molecules (sgRNAs) described in the present application may comprise lipid particles, for example, the lipid particles may be lipid nanoparticles (LNPs) and liposomes.

For example, as used herein, the term "lipid nanoparticle (LNP)" or "one LNP" or "plurality of LNPs" generally refers to a particle comprising plurality of (i.e., more than one) lipid molecules physically associated (e.g., covalently or non-covalently) to each other by intermolecular forces. LNPs may be, for example, microspheres (including unilamellar and multilamellar vesicles, such as liposomes), dispersed phases in emulsions, micelles, or internal phases in suspensions. LNPs can encapsulate nucleic acids within cationic lipid particles (eg, liposomes), and can be delivered to cells relatively easily. In some examples, the lipid nanoparticles do not contain any viral components, which helps to minimize safety and immunogenicity issues. The lipid particles are useful for in vitro, ex vivo and in vivo delivery. The lipid particles are also useful for cell populations of various sizes. The LNP of the present application can be readily prepared by various methods known in the art, such as by mixing an organic phase with an aqueous phase. The mixing of the two phases can be achieved by a microfluidic device and an impinging flow reactor. The more thoroughly the organic phase and the aqueous phase are mixed, the better the encapsulation efficiency and the particle size distribution of the obtained LNPs. Preferably, the particle size of LNP can be adjusted by changing the mixing speed of the organic phase and the aqueous phase. The faster the mixing speed, the smaller the particle size of the prepared LNPs. The encapsulation efficiency can be optimized by adjusting the N/P (ionizable lipid/nucleic acid) ratio of the LNP system. In some preferred embodiments, the N/P ratio is 1:1-9:1. In some examples, LNPs can be used to deliver DNA molecules (e.g., molecules comprising coding sequences for DNA binding proteins and/or sgRNAs) and/or RNA molecules (e.g., mRNAs for Cas or sgRNAs). In certain cases, LNPs can be used to deliver RNP complexes of Cas/gRNA. In some embodiments, LNPs are used to deliver mRNAs and gRNAs (e.g., an mRNA fusion molecule comprising DNMT3A-DNMT3L (3A-3L)-dCas9-KRAB or DNMT3A-DNMT3L-ZIM3 KRAB-dCas9, and at least one sgRNA targeting an HBV gene).

The components of LNPs can include cationic lipids 1,2-phthalic acid-3-dimethylammonium-propane (DLinDAP), 1,2-dilinoleyloxy-3-N,N-dimethylaminopropane (DLinDMA), 1,2-dilinoleyloxyketo-N,N-dimethyl-3-aminopropane (DLinK-DMA), 1,2-dilinoleyl-4-(2-dimethylaminoethyl)-. In some embodiments, LNPs may include ionizable lipids. In some embodiments, ionizable lipids include, but are not limited to, pH-responsive ionizable lipids, thermo-responsive ionizable lipids and photo-responsive ionizable lipids. In some embodiments, ionizable lipids include cationic lipids and anionic lipids that are ionized under certain conditions, such as, but not limited to, pH, temperature, or light. In some embodiments, the molar ratio of ionizable lipids of LNPs is 20% to about 70% (e.g., about 20% to about 70%, about 20% to about 65%, about 20% to about 60%, about 20% to about 55%, about 20% to about 50%, about 20% to about 45%, about 20% to about 40%, about 20% to about 35%, about 20% to about 30%, about 20% to about 25%, about 30% to about 70%, about 30% to about 65%, about 30% to about 60%, about 30% to about 55%, about 30% to about 50%, about 30% to about 45%, about 30% to about 40%, about 30% to about 35%, about 40% to about 70%, about 40% to about 65%, about 40% to about 60%, about 40% to about 55%, about 40% to about 50%, about 40% to about 45%, about 50% to about 70%, about 50% to about 65%, about 50% to about 60%, about 50% to about 55%, about 60% to about 70% or about 60% to about 65%). In some embodiments, LNPs may include pegylated lipids. In some embodiments, the molar ratio of the PEGylated lipids of the LNPs is 0% to about 30% (e.g., about 0% to about 30%, about 0% to about 25%, about 0% to about 20%, about 0% to about 15%, about 0% to about 10%, about 10% to about 30%, about 10% to about 25%, about 10% to about 20%, about 10% to about 15%, about 20% to about 30% or about 20% to about 25%). In some embodiments, LNPs may comprise supporting lipids. In some embodiments, the molar ratio of supporting lipids of the LNPs is 30% to about 50% (e.g., about 30% to about 50%, about 30% to about 45%, about 30% to about 40%, about 30% to about 35%, about 40% to about 50% or about 40% to about 45%). In some embodiments, LNPs may comprise cholesterol. In some embodiments, the molar ratio of cholesterol of the LNPs is 10% to about 50% (e.g., about 10% to about 50%, about 10% to about 45%, about 10% to about 40%, about 10% to about 35%, about 10% to about 30%, about 10% to about 25%, about 10% to about 20%, about 10% to about 15%, about 20% to about 50%, about 20% to about 45%, about 20% to about 40%, about 20% to about 35%, about 20% to about 30%, about 20% to about 25%, about 30% to about 50%, about 30% to about 45%, about 30% to about 40%, about 30% to about 35%, about 40% to about 50% or about 40% to about 45%). In some embodiments, LNPs may comprise a mixture of ionizable lipids (20%-70%, molar ratio), PEGylated lipids (0%-30%, molar ratio), supporting lipids (30%-50%, molar ratio), and cholesterol (10%-50%, molar ratio).

For example, as used herein, the term "liposome" generally refers to a vesicle having an internal space separated from the external medium by one or more bilayer membranes. In some embodiments, the bilayer membrane may be formed by amphoteric molecules, such as synthetic or naturally derived lipids containing spatially isolated hydrophilic and hydrophobic domains; In some other embodiments, the bilayer membrane may be formed by amphiphilic polymers and surfactants. In some embodiments, the liposome is a spherical vesicle structure composed of a monolayer or multilayer lipid bilayer surrounding an inner aqueous compartment, and a relatively impermeable outer lipophilic phospholipid bilayer. In some embodiments, liposomes are biocompatible, non-toxic, can deliver hydrophilic and lipophilic drug molecules, protect their cargo from degradation by plasma enzymes, and transport their cargo across biological membranes and blood-brain barrier (BBB). Liposomes can be made from several different types of lipids, such as phospholipids. The liposomes may comprise natural phospholipids and lipids (such as 1,2-distearoyl-sn-glycero-3-phosphatidylcholine (DSPC), sphingomyelin, phosphatidylcholine, monosialoganglioside, or any combination thereof. In order to modify the structure and properties of liposomes, several other additives can be added to the liposomes. For example, the liposomes may further comprise cholesterol, sphingomyelin and/or 1,2-dioleoyl-sn-glycero-3-phosphoethanolamine (DOPE), for example, to increase stability and/or prevent leakage of cargo inside the liposomes.

In the present application, the term "adenovirus-associated virus (AAV) vector" generally refers to a vector having a functional or partially functional ITR sequence and a transgene. As used herein, the term "ITR" refers to an inverted terminal repeat sequence. The ITR sequence may be derived from adeno-associated virus serotypes including, but not limited to, AAV-1, AAV-2, AAV-3, AAV-4, AAV-5, AAV-6 6, AAV7, AAV8, AAV9, AAV10, AAV11, AAV12, and AAV13, as well as any AAV variant or mixture. However, the ITR needs not be a wild-type nucleotide sequence and can be altered (e.g., by insertion, deletion, or substitution of nucleotides) as long as the sequence retains the function of providing functional rescue, replication, and packaging. AAV vectors may have one or more AAV wild-type genes, preferably the rep and/or cap genes, completely or partially deleted, but retain functional flanking ITR sequences. Functional ITR sequences function, for example, to rescue, replicate, and package AAV virions or particles. Accordingly, an "AAV vector" is defined in the present application as comprising at least those sequences required for insertion of a transgene into cells of a subject. Those cis sequences necessary for viral replication and packaging (e.g., functional ITRs) are optionally included.

In the present application, the term "pharmaceutically acceptable carrier" generally refers to a carrier for administering therapeutic agents, such as antibodies or polypeptides, genes and other therapeutic agents. The term refers to any pharmaceutical carrier that does not itself induce the production of antibodies harmful to the individual receiving the composition and that can be administered without undue toxicity. Suitable carriers may be large, slowly metabolized macromolecules such as proteins, polysaccharides, polylactic acids, polyglycolic acids, polyamino acids, amino acid copolymers, lipid aggregates, and inactivated viral particles. These vectors are well known to those skilled in the art. Pharmaceutically acceptable carriers in therapeutic compositions may include liquids such as water, saline, glycerol, and ethanol. Auxiliary substances such as wetting or emulsifying agents, pH buffering substances and the like may also be present in these carriers.

In the present application, the term "sequence encoding" or "nucleic acid encoding" generally refers to a nucleic acid (RNA or DNA molecule) comprising a nucleotide sequence encoding a protein. The coding sequence may further comprise initiation and termination signals operably linked to regulatory elements comprising a promoter and a polyadenylation signal capable of directing expression in cells of an individual or mammal to which the nucleic acid has been administered. The coding sequence may be codon optimized. In some embodiments, the coding nucleic acid may be mRNA; one or more modification techniques can be used to produce a more stable mRNA. Known mRNA modification techniques can be roughly divided into three categories: using artificially synthesized non-natural ribonucleic acid instead of natural ribonucleic acid to synthesize mRNA; adding 5' caps, 3' poly (A) "tail" and UTR (untranslated region) sequences; and using special new formulation techniques to effectively protect mRNA. Among them, the preferred mRNA modification techniques may involve synthesizing mRNA by replacing natural ribonucleic acids with artificially synthesized non-natural ribonucleic acids. Chemical modifications on eukaryotic mRNA can be roughly divided into three categories: methylation, pseudouridine (Ψ) and hypoxanthine. For example, the chemical modification may be selected from: pseudouridine, N1-methylpseudouridine, N1-ethylpseudouridine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methyluridine, 5-methoxyuridine and 2'-O-methyluridine.

In the present application, the term "complementary" generally refers to Watson-Crick (e.g., A-T/U and C-G) or Hoogsteen base pairing between nucleotides or nucleotide analogs of nucleic acid molecules. "Complementarity" refers to a property shared between two nucleic acid sequences such that when they are arranged antiparallel to each other, the nucleotide bases at each position will be complementary.

In the present application, the terms "subject" and "patient" are used interchangeably and generally refer to humans and non-human animals. As used herein, the term "non-human animal" includes all vertebrates, for example, mammals and non-mammals, such as non-human primates, sheep, dogs, cats, horses, cows, chickens, amphibians, reptiles, and the like.

In the present application, the terms "effective amount" and "therapeutically effective amount" or "therapeutically effective dose" are used interchangeably and generally refer to the amount or dose of a fusion molecule (protein), polypeptide, nucleic acid, lipid nanoparticle, liposome, one or more AAV particles, or one or more virions that is capable of producing a sufficient amount of the desired protein to modulate protein activity in a desired manner, thereby providing a relieving tool for clinical intervention. In certain embodiments, a therapeutically effective amount or dose of a transfected fusion protein, polypeptide, nucleic acid, one or more AAV particles, or one or more virions as described herein is sufficient to confer inhibition of a gene targeted by the fusion protein/gene therapy construct.

As used herein, the term "treat" e.g., a disease, means that a subject (e.g., a human) having the disease, at risk of having the disease, and/or experiencing symptoms of the disease would, in one embodiment, experience less severe symptoms and/or would recover more quickly when administered, e.g., a fusion molecule or a nucleic acid encoding the fusion molecule and/or a gRNA or a nucleic acid encoding the gRNA as described herein, compared to when the fusion molecule or the nucleic acid encoding the fusion molecule and/or the gRNA or the nucleic acid encoding the gRNA has never been administered.

Without wishing to be bound by any theory, the following examples are only for illustrating the compositions, methods of use, and uses of the present application, and are not used to limit the scope of the invention of the present application.

### EXAMPLES

### Example 1

### Construction and functional screening of sgRNA libraries targeting the hepatitis B virus (HBV) gene

### (1) Construction of sgRNA libraries for type B, type C and type D HBV genes

This example uses the genome sequence of hepatitis B virus (HBV), which includes the complete genome of polynucleotides encoding HBV polymerase Pol, surface antigen HBsAg, HBV X protein, and core region antigen precursor HBcAg. These genomes are divided into type B HBV (serotype adw subtype), type C HBV (serotype adr subtype), and type D HBV (serotype ayw subtype), as shown in the following table.

| **Guide RNA (nucleotide sequence complementary to the target sequence + PAM sequence at 3' end)** | | **Genotype** |
|---|---|---|
| | AGACCACCGTGAACGCCCACAGG | Type B |
| | GTTCCTGTGGGCGTTCACGGTGG | Type B |
| | CAGGTTCCTGTGGGCGTTCACGG | Type B |
| | GGTCTCCATGCGACGTGCAGAGG | Type B |
| | CGCCCACAGGAACCTGCCCAAGG | Type B |
| | GACCTTGGGCAGGTTCCTGTGGG | Type B |
| | CTTCACCTCTGCACGTCGCATGG | Type B |
| | AGACCTTGGGCAGGTTCCTGTGG | Type B |
| | TCTTATGCAAGACCTTGGGCAGG | Type B |
| | TGCCCAAGGTCTTGCATAAGAGG | Type B |
| | GTCCTCTTATGCAAGACCTTGGG | Type B |
| | AGTCCTCTTATGCAAGACCTTGG | Type B |
| | GAGGTGAAGCGAAGTGCACACGG | Type B |
| | GTCTTGCATAAGAGGACTCTTGG | Type B |
| | GAAGCGAAGTGCACACGGTCCGG | Type B |
| | ACACGGTCCGGCAGATGAGAAGG | Type B |
| | GTCTGTGCCTTCTCATCTGCCGG | Type B |
| | GGCAGATGAGAAGGCACAGACGG | Type B |
| | GCAGATGAGAAGGCACAGACGGG | Type B |
| | CAGATGAGAAGGCACAGACGGGG | Type B |
| | AATGTCAACGACCGACCTTGAGG | Type B |
| | CGGGGAGTCCGCGTAAAGAGAGG | Type B |
| | TTTGAAGTATGCCTCAAGGTCGG | Type B |
| | AGTCTTTGAAGTATGCCTCAAGG | Type B |
| | GGGGCGCACCTCTCTTTACGCGG | Type B |
| | GTAAAGAGAGGTGCGCCCCGTGG | Type B |
| | AGAGAGGTGCGCCCCGTGGTCGG | Type B |
| | AGGTGCGCCCCGTGGTCGGCCGG | Type B |
| | AAGACTGTGTGTTTACTGAGTGG | Type B |
| | AGACTGTGTGTTTACTGAGTGGG | Type B |
| | TATTGTACCGGCCGACCACGGGG | Type B |
| | CTGTGTGTTTACTGAGTGGGAGG | Type B |
| | CTATTGTACCGGCCGACCACGGG | Type B |
| | CCTATTGTACCGGCCGACCACGG | Type B |
| | CCGTGGTCGGCCGGTACAATAGG | Type B |
| | TGGTCGGCCGGTACAATAGGCGG | Type B |
| | GTTTACTGAGTGGGAGGAGTTGG | Type B |
| | TTTACTGAGTGGGAGGAGTTGGG | Type B |
| | TTACTGAGTGGGAGGAGTTGGGG | Type B |
| | TACTGAGTGGGAGGAGTTGGGGG | Type B |
| | CGCTTCTCCGCCTATTGTACCGG | Type B |
| | TGAGTGGGAGGAGTTGGGGGAGG | Type B |
| | CGGTACAATAGGCGGAGAAGCGG | Type B |
| | GGTACAATAGGCGGAGAAGCGGG | Type B |
| | GTGGGAGGAGTTGGGGGAGGAGG | Type B |
| | ACAATAGGCGGAGAAGCGGGCGG | Type B |
| | AGGAGTTGGGGGAGGAGGTTAGG | Type B |
| | GGGGGAGGAGGTTAGGTTAAAGG | Type B |
| | GCGGGCGGTAGAGTCCCAAGCGG | Type B |
| | AGGTTAAAGGTCTTTGTACTAGG | Type B |
| | GAGTCCCAAGCGGCCCCGAGAGG | Type B |
| | TTAAAGGTCTTTGTACTAGGAGG | Type B |
| | AGTCCCAAGCGGCCCCGAGAGGG | Type B |
| | GTCCCAAGCGGCCCCGAGAGGGG | Type B |
| | GACCCCTCTCGGGGCCGCTTGGG | Type B |
| | CGACCCCTCTCGGGGCCGCTTGG | Type B |
| | TCTTTGTACTAGGAGGCTGTAGG | Type B |
| | GCCCCGAGAGGGGTCGTCCGCGG | Type B |
| | CCCGCGGACGACCCCTCTCGGGG | Type B |
| | CCCCGAGAGGGGTCGTCCGCGGG | Type B |
| | TCCCGCGGACGACCCCTCTCGGG | Type B |
| | ATCCCGCGGACGACCCCTCTCGG | Type B |
| | AGGAGGCTGTAGGCATAAATTGG | Type B |
| | TCCGCGGGATTCAGCGCCGACGG | Type B |
| | CCGCGGGATTCAGCGCCGACGGG | Type B |
| | CCCGTCGGCGCTGAATCCCGCGG | Type B |
| | CGCCGACGGGACGTAAACAAAGG | Type B |
| | GTCCTTTGTTTACGTCCCGTCGG | Type B |
| | GTGAAAAAGTTGCATGGTGCTGG | Type B |
| | GCAGAGGTGAAAAAGTTGCATGG | Type B |
| | AAACAAAGGACGTCCCGCGCAGG | Type B |
| | CGTCCCGCGCAGGATCCAGTTGG | Type B |
| | CTGCCAACTGGATCCTGCGCGGG | Type B |
| | GCTGCCAACTGGATCCTGCGCGG | Type B |
| | AACATGAGATGATTAGGCAGAGG | Type B |
| | GACATGAACATGAGATGATTAGG | Type B |
| | TGCTAGGCTGTGCTGCCAACTGG | Type B |
| | GCAGCACAGCCTAGCAGCCATGG | Type B |
| | ACATCATTTCCATGGCTGCTAGG | Type B |
| | AGCTTGGAGGCTTGAACAGTAGG | Type B |
| | GCAAGTATACATCATTTCCATGG | Type B |
| | ATGGAAATGATGTATACTTGCGG | Type B |
| | CAAGCCTCCAAGCTGTGCCTTGG | Type B |
| | TGGAAATGATGTATACTTGCGGG | Type B |
| | AAGCCTCCAAGCTGTGCCTTGGG | Type B |
| | CCACCCAAGGCACAGCTTGGAGG | Type B |
| | CCTCCAAGCTGTGCCTTGGGTGG | Type B |
| | AAGCCACCCAAGGCACAGCTTGG | Type B |
| | AGCTGTGCCTTGGGTGGCTTTGG | Type B |
| | GCTGTGCCTTGGGTGGCTTTGGG | Type B |
| | CTGTGCCTTGGGTGGCTTTGGGG | Type B |
| | CCTTGGGTGGCTTTGGGGCATGG | Type B |
| | CCATGCCCCAAAGCCACCCAAGG | Type B |
| | CAGAATTGTCAGTCCCGATGAGG | Type B |
| | ATTGACCCGTATAAAGAATTTGG | Type B |
| | GGTCTGGGGCAAACCTCATCGGG | Type B |
| | AGGTCTGGGGCAAACCTCATCGG | Type B |
| | AAGCTCCAAATTCTTTATACGGG | Type B |
| | GAAGCTCCAAATTCTTTATACGG | Type B |
| | TAAAGAATTTGGAGCTTCTGTGG | Type B |
| | TTTTGCTCGCAGCAGGTCTGGGG | Type B |
| | GTTTTGCTCGCAGCAGGTCTGGG | Type B |
| | TGTTTTGCTCGCAGCAGGTCTGG | Type B |
| | CCTGCTGCGAGCAAAACAAGCGG | Type B |
| | CCGCTTGTTTTGCTCGCAGCAGG | Type B |
| | TGCGAGCAAAACAAGCGGCTAGG | Type B |
| | CGGCTAGGAGTTCCGCAGTATGG | Type B |
| | AGGAGTTCCGCAGTATGGATCGG | Type B |
| | ATAGAAGGAAAGAAGTCAGAAGG | Type B |
| | TCCGCAGTATGGATCGGTAGAGG | Type B |
| | TCCTCTACCGATCCATACTGCGG | Type B |
| | GATCGGTAGAGGAGACACAAAGG | Type B |
| | TCGAGGAGATCTCGAATAGAAGG | Type B |
| | ACAGAGCAGAGGCGGTGTCGAGG | Type B |
| | AGGTTCCACGCATGCGCTGATGG | Type B |
| | ACACCGCCTCTGCTCTGTATCGG | Type B |
| | CACCGCCTCTGCTCTGTATCGGG | Type B |
| | CTCCCGATACAGAGCAGAGGCGG | Type B |
| | ATAGGCCATCAGCGCATGCGTGG | Type B |
| | CGCCTCTGCTCTGTATCGGGAGG | Type B |
| | ACGCATGCGCTGATGGCCTATGG | Type B |
| | GGCCTCCCGATACAGAGCAGAGG | Type B |
| | TCGGGAGGCCTTAGAGTCTCCGG | Type B |
| | ACTGGTTGGGGCTTGGCCATAGG | Type B |
| | TATGGCCAAGCCCCAACCAGTGG | Type B |
| | ATGGCCAAGCCCCAACCAGTGGG | Type B |
| | TGGCCAAGCCCCAACCAGTGGGG | Type B |
| | GGCCAAGCCCCAACCAGTGGGGG | Type B |
| | ACAATGTTCCGGAGACTCTAAGG | Type B |
| | AACCCCCACTGGTTGGGGCTTGG | Type B |
| | GACGCAACCCCCACTGGTTGGGG | Type B |
| | TGACGCAACCCCCACTGGTTGGG | Type B |
| | CTGACGCAACCCCCACTGGTTGG | Type B |
| | TGGTGAGGTGAACAATGTTCCGG | Type B |
| | TTTGCTGACGCAACCCCCACTGG | Type B |
| | ACATTGTTCACCTCACCATACGG | Type B |
| | GGGTTGCGTCAGCAAACACTTGG | Type B |
| | CACCTCACCATACGGCACTCAGG | Type B |
| | TGCCTGAGTGCCGTATGGTGAGG | Type B |
| | GCAAACACTTGGCACAGACCAGG | Type B |
| | TAGCTTGCCTGAGTGCCGTATGG | Type B |
| | TCAGGCAAGCTATCCTGTGTTGG | Type B |
| | CAGGCAAGCTATCCTGTGTTGGG | Type B |
| | AGGCAAGCTATCCTGTGTTGGGG | Type B |
| | ACCAGGCCGTTGCCGAGCAACGG | Type B |
| | CCCGTTGCTCGGCAACGGCCTGG | Type B |
| | CCAGGCCGTTGCCGAGCAACGGG | Type B |
| | CAGGCCGTTGCCGAGCAACGGGG | Type B |
| | TTTACCCCGTTGCTCGGCAACGG | Type B |
| | TCATCAACTCACCCCAACACAGG | Type B |
| | GTTGCCGAGCAACGGGGTAAAGG | Type B |
| | TGAACCTTTACCCCGTTGCTCGG | Type B |
| | AGTTGATGAATCTAGCTACCTGG | Type B |
| | GTTGATGAATCTAGCTACCTGGG | Type B |
| | GATGAATCTAGCTACCTGGGTGG | Type B |
| | ATGAATCTAGCTACCTGGGTGGG | Type B |
| | CAGATACTGTTTACTTAGAAAGG | Type B |
| | TACCTGGGTGGGAAGTAATTTGG | Type B |
| | TTCCAAATTACTTCCCACCCAGG | Type B |
| | CTTAGAAAGGCCTTGTAAGTTGG | Type B |
| | ATTTGGAAGATCCAGCATCCAGG | Type B |
| | TTTGGAAGATCCAGCATCCAGGG | Type B |
| | TACTTTCTCGCCAACTTACAAGG | Type B |
| | ACTACTAATTCCCTGGATGCTGG | Type B |
| | ATAGCTGACTACTAATTCCCTGG | Type B |
| | TGCATGTATACAAGCGAAACAGG | Type B |
| | CAGCTATGTCAACGTTAACATGG | Type B |
| | AGCTATGTCAACGTTAACATGGG | Type B |
| | GCTTGTATACATGCATATAAAGG | Type B |
| | GCATATAAAGGCATTAAAGCAGG | Type B |
| | ACAAGAGTTGTCTGATTTTTAGG | Type B |
| | TAAAAATCAGACAACTCTTGTGG | Type B |
| | GGATATCCACATTGCGTGAAAGG | Type B |
| | GATATCCACATTGCGTGAAAGGG | Type B |
| | ATATCCACATTGCGTGAAAGGGG | Type B |
| | TCCACATTGCGTGAAAGGGGCGG | Type B |
| | GCCGCCCCTTTCACGCAATGTGG | Type B |
| | CACATTTCCTGTCTTACTTTTGG | Type B |
| | ACATTTCCTGTCTTACTTTTGGG | Type B |
| | TTCTCTCCCAAAAGTAAGACAGG | Type B |
| | AACGAATTGTGGGTCTTTTGGGG | Type B |
| | CAACGAATTGTGGGTCTTTTGGG | Type B |
| | TCAACGAATTGTGGGTCTTTTGG | Type B |
| | AAAGTATGTCAACGAATTGTGGG | Type B |
| | AGAAACTGTCCTTGAATATTTGG | Type B |
| | GAAAGTATGTCAACGAATTGTGG | Type B |
| | CAAAAGACACCAAATATTCAAGG | Type B |
| | CTTGAATATTTGGTGTCTTTTGG | Type B |
| | TGACATACTTTCCAATCAATAGG | Type B |
| | ATTTGGTGTCTTTTGGAGTGTGG | Type B |
| | CTGTAAACAGGCCTATTGATTGG | Type B |
| | CAATCAATAGGCCTGTTTACAGG | Type B |
| | TTAGGAAACTTCCTGTAAACAGG | Type B |
| | TTTGGTGGTCTATATGCAGGAGG | Type B |
| | AAAAAATCAAAATGTGTTTTAGG | Type B |
| | GCATTTGGTGGTCTATATGCAGG | Type B |
| | GATAAGATAGGGGCATTTGGTGG | Type B |
| | GTTGATAAGATAGGGGCATTTGG | Type B |
| | TTTTGTACAATATGTTCCTGTGG | Type B |
| | CGGAAGAGTTGATAAGATAGGGG | Type B |
| | CCCTATCTTATCAACTCTTCCGG | Type B |
| | CCGGAAGAGTTGATAAGATAGGG | Type B |
| | TCCGGAAGAGTTGATAAGATAGG | Type B |
| | GAGTTGGGGCACATTGCCACAGG | Type B |
| | CGTCTAACAACAGTAGTTTCCGG | Type B |
| | ACTACTGTTGTTAGACGACGAGG | Type B |
| | GGATATGTAATTGGGAGTTGGGG | Type B |
| | GGGATATGTAATTGGGAGTTGGG | Type B |
| | TGGGATATGTAATTGGGAGTTGG | Type B |
| | CTGTTGTTAGACGACGAGGCAGG | Type B |
| | ACTTCATGGGATATGTAATTGGG | Type B |
| | AACTTCATGGGATATGTAATTGG | Type B |
| | TTACATATCCCATGAAGTTAAGG | Type B |
| | TACATATCCCATGAAGTTAAGGG | Type B |
| | GGATATTCCCTTAACTTCATGGG | Type B |
| | GGGATATTCCCTTAACTTCATGG | Type B |
| | CGAGGGAGTTCTTCTTCTAGGGG | Type B |
| | GCGAGGGAGTTCTTCTTCTAGGG | Type B |
| | GGCGAGGGAGTTCTTCTTCTAGG | Type B |
| | CCCCATCTTTTTGTTTTGTGAGG | Type B |
| | CTCCCTCGCCTCGCAGACGAAGG | Type B |
| | CCTCACAAAACAAAAAGATGGGG | Type B |
| | CCCTCACAAAACAAAAAGATGGG | Type B |
| | CCCATCTTTTTGTTTTGTGAGGG | Type B |
| | GACCTTCGTCTGCGAGGCGAGGG | Type B |
| | ACCCTCACAAAACAAAAAGATGG | Type B |
| | AGACCTTCGTCTGCGAGGCGAGG | Type B |
| | GATTGAGACCTTCGTCTGCGAGG | Type B |
| | GATTGATATCTTCTGCGACGCGG | Type B |
| | ACCCAAAGACAAAAGAAAATTGG | Type B |
| | GTCGCAGAAGATATCAATCTCGG | Type B |
| | ACCAATTTTCTTTTGTCTTTGGG | Type B |
| | TCGCAGAAGATATCAATCTCGGG | Type B |
| | TACCAATTTTCTTTTGTCTTTGG | Type B |
| | CAAAAGAAAATTGGTAACAGCGG | Type B |
| | AATTGGTAACAGCGGCATAAAGG | Type B |
| | ATTGGTAACAGCGGCATAAAGGG | Type B |
| | ATCTCAATGTTAGTATTCCTTGG | Type B |
| | TAGTATTCCTTGGACACATAAGG | Type B |
| | TATTCCTTGGACACATAAGGTGG | Type B |
| | ATTCCTTGGACACATAAGGTGGG | Type B |
| | CTCAAGATGTTGTACAGACTTGG | Type B |
| | TTTCCCACCTTATGTGTCCAAGG | Type B |
| | ACATAAGGTGGGAAACTTTACGG | Type B |
| | CATAAGGTGGGAAACTTTACGGG | Type B |
| | ATAAGGTGGGAAACTTTACGGGG | Type B |
| | TATGGATGATGTGGTTTTGGGGG | Type B |
| | ATATGGATGATGTGGTTTTGGGG | Type B |
| | TATATGGATGATGTGGTTTTGGG | Type B |
| | TTATATGGATGATGTGGTTTTGG | Type B |
| | TACGGGGCTTTATTCTTCTACGG | Type B |
| | TTTCAGTTATATGGATGATGTGG | Type B |
| | CTGTCTGGCTTTCAGTTATATGG | Type B |
| | TATAACTGAAAGCCAGACAGTGG | Type B |
| | ATAACTGAAAGCCAGACAGTGGG | Type B |
| | TAACTGAAAGCCAGACAGTGGGG | Type B |
| | AACTGAAAGCCAGACAGTGGGGG | Type B |
| | TACCTTGCTTTAATCCTAAATGG | Type B |
| | TGCCATTTAGGATTAAAGCAAGG | Type B |
| | TAGGGCTTTCCCCCACTGTCTGG | Type B |
| | AAAGAAGGAGTTTGCCATTTAGG | Type B |
| | CATTTGTTCAGTGGTTCGTAGGG | Type B |
| | CCTACGAACCACTGAACAAATGG | Type B |
| | CCATTTGTTCAGTGGTTCGTAGG | Type B |
| | AAATGAATGTCAGGAAAAGAAGG | Type B |
| | TACTAGTGCCATTTGTTCAGTGG | Type B |
| | TTTTCCTGACATTCATTTGCAGG | Type B |
| | TCCTGACATTCATTTGCAGGAGG | Type B |
| | TCCTCCTGCAAATGAATGTCAGG | Type B |
| | GTAAACTGAGCCAAGAGAAACGG | Type B |
| | GAGCCAAGAGAAACGGACTGAGG | Type B |
| | GGGCCTCAGTCCGTTTCTCTTGG | Type B |
| | TGATAGATGTAAGCAATTTGTGG | Type B |
| | GATAGATGTAAGCAATTTGTGGG | Type B |
| | ATAGATGTAAGCAATTTGTGGGG | Type B |
| | GGACTGAGGCCCACTCCCATAGG | Type B |
| | CGCAAAATACCTATGGGAGTGGG | Type B |
| | TCGCAAAATACCTATGGGAGTGG | Type B |
| | GGCTTTCGCAAAATACCTATGGG | Type B |
| | GGGCTTTCGCAAAATACCTATGG | Type B |
| | CTGTTTTCATTTACTGTAAGGGG | Type B |
| | CCCTTACAGTAAATGAAAACAGG | Type B |
| | CCTGTTTTCATTTACTGTAAGGG | Type B |
| | TCCTGTTTTCATTTACTGTAAGG | Type B |
| | TTTGCGAAAGCCCAAGATGATGG | Type B |
| | TTGCGAAAGCCCAAGATGATGGG | Type B |
| | GAAAGCCCAAGATGATGGGATGG | Type B |
| | AAAGCCCAAGATGATGGGATGGG | Type B |
| | TATTCCCATCCCATCATCTTGGG | Type B |
| | GTATTCCCATCCCATCATCTTGG | Type B |
| | AGATGATGGGATGGGAATACAGG | Type B |
| | TGAAATTAATTATGCCTGCTAGG | Type B |
| | CAGGTGCAGTTTCCGTCCGTAGG | Type B |
| | ACATTGGGATAAAACCTAGCAGG | Type B |
| | TGCTGTACAAAACCTACGGACGG | Type B |
| | ATGTTGCTGTACAAAACCTACGG | Type B |
| | GGCAAATATTTAGTAACATTGGG | Type B |
| | GGGCAAATATTTAGTAACATTGG | Type B |
| | AGGTTTTGTACAGCAACATGAGG | Type B |
| | GGTTTTGTACAGCAACATGAGGG | Type B |
| | AAATATTTGCCCTTAGATAAAGG | Type B |
| | AATATTTGCCCTTAGATAAAGGG | Type B |
| | GCAACATGAGGGAAACATAGAGG | Type B |
| | GGTTTGATCCCTTTATCTAAGGG | Type B |
| | CGGTTTGATCCCTTTATCTAAGG | Type B |
| | AACATAGAGGTTCCTTGAGCAGG | Type B |
| | TCCTTGAGCAGGAGTTGTGCAGG | Type B |
| | ACCTGCACAACTCCTGCTCAAGG | Type B |
| | ACTACATACTCTGGATAATACGG | Type B |
| | GGAGTTGTGCAGGTTTTGCATGG | Type B |
| | TAATGATTAACTACATACTCTGG | Type B |
| | TGTGCAGGTTTTGCATGGTCCGG | Type B |
| | GGTTTTGCATGGTCCGGTGCTGG | Type B |
| | GGATCATCAACAACCAGCACCGG | Type B |
| | GTGCTGGTTGTTGATGATCCTGG | Type B |
| | TGTGTAAATAATGTCGCGTCTGG | Type B |
| | GACATTATTTACACACTCTTTGG | Type B |
| | GTTGATGATCCTGGAATTAGAGG | Type B |
| | TTATTTACACACTCTTTGGAAGG | Type B |
| | TTTACACACTCTTTGGAAGGCGG | Type B |
| | TTACACACTCTTTGGAAGGCGGG | Type B |
| | TACACACTCTTTGGAAGGCGGGG | Type B |
| | TCCTGGAATTAGAGGACAAACGG | Type B |
| | CCCGTTTGTCCTCTAATTCCAGG | Type B |
| | CCTGGAATTAGAGGACAAACGGG | Type B |
| | TTGGTTCTTCTGGACTATCAAGG | Type B |
| | CATCTTCTTGTTGGTTCTTCTGG | Type B |
| | GCAAAATGAGGCGCTACGTGTGG | Type B |
| | ACACGTAGCGCCTCATTTTGCGG | Type B |
| | GAAGAACCAACAAGAAGATGAGG | Type B |
| | CACGTAGCGCCTCATTTTGCGGG | Type B |
| | GCTATGCCTCATCTTCTTGTTGG | Type B |
| | ATATGGTGACCCGCAAAATGAGG | Type B |
| | AGAAGATGAGGCATAGCAGCAGG | Type B |
| | TTTGCGGGTCACCATATTCTTGG | Type B |
| | TTGCGGGTCACCATATTCTTGGG | Type B |
| | GGCATAGCAGCAGGATGCAGAGG | Type B |
| | AGATCTTGTTCCCAAGAATATGG | Type B |
| | TGGGAACAAGATCTACAGCATGG | Type B |
| | GGGAACAAGATCTACAGCATGGG | Type B |
| | AACAAGATCTACAGCATGGGAGG | Type B |
| | AGATCTACAGCATGGGAGGTTGG | Type B |
| | GTTATCGCTGGATGTGTCTGCGG | Type B |
| | AGACACATCCAGCGATAACCAGG | Type B |
| | TGGTCTTCCAAACCTCGAAAAGG | Type B |
| | TTCCAAACCTCGAAAAGGCATGG | Type B |
| | CAATTTGTCCTGGTTATCGCTGG | Type B |
| | TCCAAACCTCGAAAAGGCATGGG | Type B |
| | CAGCGATAACCAGGACAAATTGG | Type B |
| | CCCCATGCCTTTTCGAGGTTTGG | Type B |
| | CCAAACCTCGAAAAGGCATGGGG | Type B |
| | CGATAACCAGGACAAATTGGAGG | Type B |
| | TTTGTCCCCATGCCTTTTCGAGG | Type B |
| | TGTTGTCCTCCAATTTGTCCTGG | Type B |
| | AGGACAAATTGGAGGACAACAGG | Type B |
| | CAAATTGGAGGACAACAGGTTGG | Type B |
| | ACAACAGGTTGGTGAGTGACTGG | Type B |
| | TCTTTCTGTCCCCAATCCCCTGG | Type B |
| | CTTTCTGTCCCCAATCCCCTGGG | Type B |
| | TTGGTGAGTGACTGGAGATTTGG | Type B |
| | TGGTGAGTGACTGGAGATTTGGG | Type B |
| | GGAAGAATCCCAGGGGATTGGGG | Type B |
| | GGGAAGAATCCCAGGGGATTGGG | Type B |
| | GGGGAAGAATCCCAGGGGATTGG | Type B |
| | TGATCGGGGAAGAATCCCAGGGG | Type B |
| | ATGATCGGGGAAGAATCCCAGGG | Type B |
| | GATGATCGGGGAAGAATCCCAGG | Type B |
| | AGATTTGGGACTGCGAATTTTGG | Type B |
| | ATTCTTCCCCGATCATCAGTTGG | Type B |
| | CAGGGTCCAACTGATGATCGGGG | Type B |
| | GCAGGGTCCAACTGATGATCGGG | Type B |
| | TGCAGGGTCCAACTGATGATCGG | Type B |
| | CGAATTTTGGCCAAGACACACGG | Type B |
| | GAATTTTGGCCAAGACACACGGG | Type B |
| | GGGGGAACACCCGTGTGTCTTGG | Type B |
| | CTGAGTTGGCTTTGAATGCAGGG | Type B |
| | TCTGAGTTGGCTTTGAATGCAGG | Type B |
| | CCAATCTGGACTTTCTGAGTTGG | Type B |
| | ACTTCTCTCAATTTTCTAGGGGG | Type B |
| | CCAACTCAGAAAGTCCAGATTGG | Type B |
| | CAACTCAGAAAGTCCAGATTGGG | Type B |
| | GACTTCTCTCAATTTTCTAGGGG | Type B |
| | GGACTTCTCTCAATTTTCTAGGG | Type B |
| | TGGACTTCTCTCAATTTTCTAGG | Type B |
| | TGCGGGTTGAGGTCCCAATCTGG | Type B |
| | TTGGGACCTCAACCCGCACAAGG | Type B |
| | CACAGAGTCTAGACTCGTGGTGG | Type B |
| | CACCACGAGTCTAGACTCTGTGG | Type B |
| | AGTTGTCCTTGTGCGGGTTGAGG | Type B |
| | TACCACAGAGTCTAGACTCGTGG | Type B |
| | TCAACCCGCACAAGGACAACTGG | Type B |
| | CCGGCCAGTTGTCCTTGTGCGGG | Type B |
| | CCCGCACAAGGACAACTGGCCGG | Type B |
| | TCCGGCCAGTTGTCCTTGTGCGG | Type B |
| | CTAGACTCTGTGGTATTGTGAGG | Type B |
| | CAACTGGCCGGACGCCAACAAGG | Type B |
| | CTGGCCGGACGCCAACAAGGTGG | Type B |
| | TGGCCGGACGCCAACAAGGTGGG | Type B |
| | ACTCCCACCTTGTTGGCGTCCGG | Type B |
| | GGACGCCAACAAGGTGGGAGTGG | Type B |
| | GACGCCAACAAGGTGGGAGTGGG | Type B |
| | TGCTCCCACTCCCACCTTGTTGG | Type B |
| | AAGGTGGGAGTGGGAGCATTCGG | Type B |
| | AGGTGGGAGTGGGAGCATTCGGG | Type B |
| | GGAGTGGGAGCATTCGGGCCAGG | Type B |
| | GAGTGGGAGCATTCGGGCCAGGG | Type B |
| | ACCCCGCCTGTAACACGAGCAGG | Type B |
| | CCCCGCCTGTAACACGAGCAGGG | Type B |
| | CCCTGCTCGTGTTACAGGCGGGG | Type B |
| | CCCGCCTGTAACACGAGCAGGGG | Type B |
| | CCCCTGCTCGTGTTACAGGCGGG | Type B |
| | ACCCCTGCTCGTGTTACAGGCGG | Type B |
| | AGGACCCCTGCTCGTGTTACAGG | Type B |
| | GTAACACGAGCAGGGGTCCTAGG | Type B |
| | CCATGGGGAGGGGTGAACCCTGG | Type B |
| | CCAGGGTTCACCCCTCCCCATGG | Type B |
| | CAGGGTTCACCCCTCCCCATGGG | Type B |
| | AGGGTTCACCCCTCCCCATGGGG | Type B |
| | GGGTTCACCCCTCCCCATGGGGG | Type B |
| | CAACAGTCCCCCATGGGGAGGGG | Type B |
| | CCCTCCCCATGGGGGACTGTTGG | Type B |
| | CCAACAGTCCCCCATGGGGAGGG | Type B |
| | CCCAACAGTCCCCCATGGGGAGG | Type B |
| | CCTCCCCATGGGGGACTGTTGGG | Type B |
| | CTCCCCATGGGGGACTGTTGGGG | Type B |
| | CACCCCAACAGTCCCCCATGGGG | Type B |
| | CCCATGGGGGACTGTTGGGGTGG | Type B |
| | AACATCGCATCAGGACTCCTAGG | Type B |
| | CCACCCCAACAGTCCCCCATGGG | Type B |
| | TCCACCCCAACAGTCCCCCATGG | Type B |
| | AACATGGAGAACATCGCATCAGG | Type B |
| | ACTGTTGGGGTGGAGCCCTCAGG | Type B |
| | GATGCGATGTTCTCCATGTTCGG | Type B |
| | GGGGTGGAGCCCTCAGGCTCAGG | Type B |
| | GGGTGGAGCCCTCAGGCTCAGGG | Type B |
| | ATGTTCTCCATGTTCGGTACAGG | Type B |
| | TGTTCTCCATGTTCGGTACAGGG | Type B |
| | TGAGTAGGCCCTGAGCCTGAGGG | Type B |
| | TGGGGACCCTGTACCGAACATGG | Type B |
| | GTGAGTAGGCCCTGAGCCTGAGG | Type B |
| | CTGCTGGCACAGTTGTGAGTAGG | Type B |
| | GTCAATCTTATCGAAGACTGGGG | Type B |
| | CGTCAATCTTATCGAAGACTGGG | Type B |
| | TCGTCAATCTTATCGAAGACTGG | Type B |
| | CTTCGATAAGATTGACGATATGG | Type B |
| | GAGGCAGGAGGAGGAGCTGCTGG | Type B |
| | CGATTGGTGGAGGCAGGAGGAGG | Type B |
| | CTCCTCCTGCCTCCACCAATCGG | Type B |
| | TGCCGATTGGTGGAGGCAGGAGG | Type B |
| | GACTGCCGATTGGTGGAGGCAGG | Type B |
| | GCAGAGACAGTATTCTGAGCAGG | Type B |
| | CAGAGACAGTATTCTGAGCAGGG | Type B |
| | GCCTCCACCAATCGGCAGTCAGG | Type B |
| | TCCTGACTGCCGATTGGTGGAGG | Type B |
| | CCTTCCTGACTGCCGATTGGTGG | Type B |
| | CCACCAATCGGCAGTCAGGAAGG | Type B |
| | CTGCCTTCCTGACTGCCGATTGG | Type B |
| | AGGGCTCACTGTTCCTGAACTGG | Type B |
| | AGAGGTGGAGATAAGGGAGTAGG | Type B |
| | CCTGAACTGGAGCCACCAGCAGG | Type B |
| | CCTGCTGGTGGCTCCAGTTCAGG | Type B |
| | CTCCCTTATCTCCACCTCTAAGG | Type B |
| | TCCCTTATCTCCACCTCTAAGGG | Type B |
| | TCCCTTAGAGGTGGAGATAAGGG | Type B |
| | GTCCCTTAGAGGTGGAGATAAGG | Type B |
| | AGCCACCAGCAGGAAAGTACAGG | Type B |
| | GCCACCAGCAGGAAAGTACAGGG | Type B |
| | GCCCTGTACTTTCCTGCTGGTGG | Type B |
| | GGATGAGTGTCCCTTAGAGGTGG | Type B |
| | AGGGCCCTGTACTTTCCTGCTGG | Type B |
| | TGAGGATGAGTGTCCCTTAGAGG | Type B |
| | TCTAAGGGACACTCATCCTCAGG | Type B |
| | AAAGTACAGGGCCCTGACTCTGG | Type B |
| | AAGTACAGGGCCCTGACTCTGGG | Type B |
| | CTCATCCTCAGGCCATGCAGTGG | Type B |
| | TCTTCAAGATCCCAGAGTCAGGG | Type B |
| | CTCTTCAAGATCCCAGAGTCAGG | Type B |
| | CTCTGGGATCTTGAAGAGTTTGG | Type B |
| | TGGGATCTTGAAGAGTTTGGTGG | Type B |
| | TTGAAGAGTTTGGTGGAAAGTGG | Type B |
| | AAGAGTTTGGTGGAAAGTGGTGG | Type B |
| | GAGTTCCACTGCATGGCCTGAGG | Type B |
| | AGTGGTGGAGTTCCACTGCATGG | Type B |
| | CAGAGCTTGGTGGAATGTTGTGG | Type C |
| | TGGGATCTAGCAGAGCTTGGTGG | Type C |
| | CTCTGGGATCTAGCAGAGCTTGG | Type C |
| | CTCTGCTAGATCCCAGAGTGAGG | Type C |
| | TCTGCTAGATCCCAGAGTGAGGG | Type C |
| | CTGCTAGATCCCAGAGTGAGGGG | Type C |
| | AAATATAGGCCCCTCACTCTGGG | Type C |
| | AAAATATAGGCCCCTCACTCTGG | Type C |
| | AGGGGCCTATATTTTCCTGCTGG | Type C |
| | GGCCTATATTTTCCTGCTGGTGG | Type C |
| | AGCCACCAGCAGGAAAATATAGG | Type C |
| | CCTGCTGGTGGCTCCAGTTCCGG | Type C |
| | CCGGAACTGGAGCCACCAGCAGG | Type C |
| | AGGGTTTACTGTTCCGGAACTGG | Type C |
| | CGGAACAGGGTTTACTGTTCCGG | Type C |
| | GTGAGGCAGTAGTCGGAACAGGG | Type C |
| | GGTGAGGCAGTAGTCGGAACAGG | Type C |
| | GATATGGGTGAGGCAGTAGTCGG | Type C |
| | GAAGATTGACGATATGGGTGAGG | Type C |
| | CTCGAGAAGATTGACGATATGGG | Type C |
| | CCTCGAGAAGATTGACGATATGG | Type C |
| | CCATATCGTCAATCTTCTCGAGG | Type C |
| | TCGTCAATCTTCTCGAGGACTGG | Type C |
| | CGTCAATCTTCTCGAGGACTGGG | Type C |
| | GTCAATCTTCTCGAGGACTGGGG | Type C |
| | TGGGGACCCTGCACCGAACATGG | Type C |
| | TGTTCTCCATGTTCGGTGCAGGG | Type C |
| | GTGTTCTCCATGTTCGGTGCAGG | Type C |
| | GATGTTGTGTTCTCCATGTTCGG | Type C |
| | AACATGGAGAACACAACATCAGG | Type C |
| | AACACAACATCAGGATTCCTAGG | Type C |
| | GTAACACGAGCAGGGGTCCTAGG | Type C |
| | AGGACCCCTGCTCGTGTTACAGG | Type C |
| | ACCCCTGCTCGTGTTACAGGCGG | Type C |
| | CCCCTGCTCGTGTTACAGGCGGG | Type C |
| | CCCGCCTGTAACACGAGCAGGGG | Type C |
| | CCCTGCTCGTGTTACAGGCGGGG | Type C |
| | CCCCGCCTGTAACACGAGCAGGG | Type C |
| | ACCCCGCCTGTAACACGAGCAGG | Type C |
| | CTAGACTCTGTGGTATTGTGAGG | Type C |
| | TACCACAGAGTCTAGACTCGTGG | Type C |
| | CACCACGAGTCTAGACTCTGTGG | Type C |
| | CACAGAGTCTAGACTCGTGGTGG | Type C |
| | TGGACTTCTCTCAATTTTCTAGG | Type C |
| | GGACTTCTCTCAATTTTCTAGGG | Type C |
| | GACTTCTCTCAATTTTCTAGGGG | Type C |
| | ACTTCTCTCAATTTTCTAGGGGG | Type C |
| | GGGGGAGCACCCACGTGTCCTGG | Type C |
| | GAATTTTGGCCAGGACACGTGGG | Type C |
| | CGAATTTTGGCCAGGACACGTGG | Type C |
| | GGGGACTGCGAATTTTGGCCAGG | Type C |
| | AGGTTGGGGACTGCGAATTTTGG | Type C |
| | TGGTGAGTGATTGGAGGTTGGGG | Type C |
| | TTGGTGAGTGATTGGAGGTTGGG | Type C |
| | GTTGGTGAGTGATTGGAGGTTGG | Type C |
| | AGAGGTTGGTGAGTGATTGGAGG | Type C |
| | ACAAGAGGTTGGTGAGTGATTGG | Type C |
| | CAAATTGGAGGACAAGAGGTTGG | Type C |
| | AGGACAAATTGGAGGACAAGAGG | Type C |
| | TCTTGTCCTCCAATTTGTCCTGG | Type C |
| | CGATAGCCAGGACAAATTGGAGG | Type C |
| | CAGCGATAGCCAGGACAAATTGG | Type C |
| | CAATTTGTCCTGGCTATCGCTGG | Type C |
| | AGACACATCCAGCGATAGCCAGG | Type C |
| | GCTATCGCTGGATGTGTCTGCGG | Type C |
| | GGCATAGCAGCAGGATGAAGAGG | Type C |
| | AGAAGATGAGGCATAGCAGCAGG | Type C |
| | GCTATGCCTCATCTTCTTGTTGG | Type C |
| | GAAGAACCAACAAGAAGATGAGG | Type C |
| | CATCTTCTTGTTGGTTCTTCTGG | Type C |
| | TTGGTTCTTCTGGACTACCAAGG | Type C |
| | GACAAACGGGCAACATACCTTGG | Type C |
| | CCTGGAAGTAGAGGACAAACGGG | Type C |
| | CCCGTTTGTCCTCTACTTCCAGG | Type C |
| | TCCTGGAAGTAGAGGACAAACGG | Type C |
| | GTTGATGTTCCTGGAAGTAGAGG | Type C |
| | GTGCTGGTGGTTGATGTTCCTGG | Type C |
| | AGGAACATCAACCACCAGCACGG | Type C |
| | GGAACATCAACCACCAGCACGGG | Type C |
| | GAACATCAACCACCAGCACGGGG | Type C |
| | CTTGCATGGCCCCGTGCTGGTGG | Type C |
| | GGTCTTGCATGGCCCCGTGCTGG | Type C |
| | GGAATCGTGCAGGTCTTGCATGG | Type C |
| | ACCTGCACGATTCCTGCTCAAGG | Type C |
| | TCCTTGAGCAGGAATCGTGCAGG | Type C |
| | AACATAGAGGTTCCTTGAGCAGG | Type C |
| | GCAACAAGAGGGAAACATAGAGG | Type C |
| | GGTTTTGTACAGCAACAAGAGGG | Type C |
| | AGGTTTTGTACAGCAACAAGAGG | Type C |
| | TTGTTGCTGTACAAAACCTTCGG | Type C |
| | TGCTGTACAAAACCTTCGGACGG | Type C |
| | CAAGTGCAGTTTCCGTCCGAAGG | Type C |
| | GTATTCCCATCCCATCATCCTGG | Type C |
| | TATTCCCATCCCATCATCCTGGG | Type C |
| | AAAGCCCAGGATGATGGGATGGG | Type C |
| | GAAAGCCCAGGATGATGGGATGG | Type C |
| | TTGCGAAAGCCCAGGATGATGGG | Type C |
| | CTTGCGAAAGCCCAGGATGATGG | Type C |
| | TAGGAATCTTGCGAAAGCCCAGG | Type C |
| | GGGCTTTCGCAAGATTCCTATGG | Type C |
| | GGCTTTCGCAAGATTCCTATGGG | Type C |
| | TCGCAAGATTCCTATGGGAGTGG | Type C |
| | CGCAAGATTCCTATGGGAGTGGG | Type C |
| | GGACTGAGGCCCACTCCCATAGG | Type C |
| | GGGCCTCAGTCCGTTTCTCCTGG | Type C |
| | GAGCCAGGAGAAACGGACTGAGG | Type C |
| | GTAAACTGAGCCAGGAGAAACGG | Type C |
| | TGGCACTAGTAAACTGAGCCAGG | Type C |
| | TACTAGTGCCATTTGTTCAGTGG | Type C |
| | CCTACGAACCACTGAACAAATGG | Type C |
| | CCATTTGTTCAGTGGTTCGTAGG | Type C |
| | CATTTGTTCAGTGGTTCGTAGGG | Type C |
| | TAGGGCTTTCCCCCACTGTTTGG | Type C |
| | AACTGAAAGCCAAACAGTGGGGG | Type C |
| | TAACTGAAAGCCAAACAGTGGGG | Type C |
| | ATAACTGAAAGCCAAACAGTGGG | Type C |
| | TATAACTGAAAGCCAAACAGTGG | Type C |
| | CTGTTTGGCTTTCAGTTATATGG | Type C |
| | TTTCAGTTATATGGATGATGTGG | Type C |
| | TTATATGGATGATGTGGTATTGG | Type C |
| | TATATGGATGATGTGGTATTGGG | Type C |
| | ATATGGATGATGTGGTATTGGGG | Type C |
| | TATGGATGATGTGGTATTGGGGG | Type C |
| | CTCAAGATGTTGTACAGACTTGG | Type C |
| | ATTGGTAATAGAGGTAAAAAGGG | Type C |
| | AATTGGTAATAGAGGTAAAAAGG | Type C |
| | CAAAAGAAAATTGGTAATAGAGG | Type C |
| | TACCAATTTTCTTTTGTCTTTGG | Type C |
| | ACCAATTTTCTTTTGTCTTTGGG | Type C |
| | ACCCAAAGACAAAAGAAAATTGG | Type C |
| | ACCCTAATAAAACCAAACGTTGG | Type C |
| | CCCAACGTTTGGTTTTATTAGGG | Type C |
| | CCCTAATAAAACCAAACGTTGGG | Type C |
| | CCTAATAAAACCAAACGTTGGGG | Type C |
| | CCCCAACGTTTGGTTTTATTAGG | Type C |
| | AAGGGAGTAGCCCCAACGTTTGG | Type C |
| | GGGCTACTCCCTTAACTTCATGG | Type C |
| | GGCTACTCCCTTAACTTCATGGG | Type C |
| | TACATATCCCATGAAGTTAAGGG | Type C |
| | TTACATATCCCATGAAGTTAAGG | Type C |
| | AACTTCATGGGATATGTAATTGG | Type C |
| | TGGGATATGTAATTGGAAGTTGG | Type C |
| | GGGATATGTAATTGGAAGTTGGG | Type C |
| | GGATATGTAATTGGAAGTTGGGG | Type C |
| | AAGTTGGGGTACTTTACCGCAGG | Type C |
| | TTTAGTACAATATGTTCCTGCGG | Type C |
| | CAATCAATAGGTCTATTTACAGG | Type C |
| | CTGTAAATAGACCTATTGATTGG | Type C |
| | TGACAGACTTTCCAATCAATAGG | Type C |
| | GAAAGTCTGTCAAAGAATTGTGG | Type C |
| | AAAGTCTGTCAAAGAATTGTGGG | Type C |
| | TCAAAGAATTGTGGGTCTTTTGG | Type C |
| | CAAAGAATTGTGGGTCTTTTGGG | Type C |
| | GCTGCCCCTTTTACACAATGTGG | Type C |
| | ATAGCCACATTGTGTAAAAGGGG | Type C |
| | GATAGCCACATTGTGTAAAAGGG | Type C |
| | GGATAGCCACATTGTGTAAAAGG | Type C |
| | GCATATAAAGGCATCAAGGCAGG | Type C |
| | ACATGCATATAAAGGCATCAAGG | Type C |
| | GATTGTATACATGCATATAAAGG | Type C |
| | TGCATGTATACAATCTAAGCAGG | Type C |
| | CACTTTCTCGCCAACTTACAAGG | Type C |
| | CACAGAAAGGCCTTGTAAGTTGG | Type C |
| | CAGATATTGTTTACACAGAAAGG | Type C |
| | TGAACCTTTACCCCGTTGCCCGG | Type C |
| | GTTGCCGGGCAACGGGGTAAAGG | Type C |
| | TTTACCCCGTTGCCCGGCAACGG | Type C |
| | CTGACCGTTGCCGGGCAACGGGG | Type C |
| | CCCGTTGCCCGGCAACGGTCAGG | Type C |
| | CCTGACCGTTGCCGGGCAACGGG | Type C |
| | ACCTGACCGTTGCCGGGCAACGG | Type C |
| | GCAGAGACCTGACCGTTGCCGGG | Type C |
| | GGCAGAGACCTGACCGTTGCCGG | Type C |
| | GGGTTGCGTCAGCAAACACTTGG | Type C |
| | TTTGCTGACGCAACCCCCACTGG | Type C |
| | CTGACGCAACCCCCACTGGATGG | Type C |
| | TGACGCAACCCCCACTGGATGGG | Type C |
| | GACGCAACCCCCACTGGATGGGG | Type C |
| | AACCCCCACTGGATGGGGCTTGG | Type C |
| | GGCCAAGCCCCATCCAGTGGGGG | Type C |
| | TGGCCAAGCCCCATCCAGTGGGG | Type C |
| | ATGGCCAAGCCCCATCCAGTGGG | Type C |
| | TATGGCCAAGCCCCATCCAGTGG | Type C |
| | ACTGGATGGGGCTTGGCCATAGG | Type C |
| | GGGGCTTGGCCATAGGCCATCGG | Type C |
| | ACGCATGCGCCGATGGCCTATGG | Type C |
| | ATAGGCCATCGGCGCATGCGTGG | Type C |
| | AGGTTCCACGCATGCGCCGATGG | Type C |
| | GCGCATGCGTGGAACCTTTGTGG | Type C |
| | GATCGGCAGAGGAGCCACAAAGG | Type C |
| | TCCTCTGCCGATCCATACTGCGG | Type C |
| | TCCGCAGTATGGATCGGCAGAGG | Type C |
| | AGGAGTTCCGCAGTATGGATCGG | Type C |
| | CTGCTAGGAGTTCCGCAGTATGG | Type C |
| | TGCGAGCGAAACAAGCTGCTAGG | Type C |
| | CAGCTTGTTTCGCTCGCAGCCGG | Type C |
| | TGTTTCGCTCGCAGCCGGTCTGG | Type C |
| | CGATAAGTTTCGCTCCAGACCGG | Type C |
| | CGGTCTGGAGCGAAACTTATCGG | Type C |
| | AGAGAGGACAACAGAGTTGTCGG | Type C |
| | ACAACTCTGTTGTCCTCTCTCGG | Type C |
| | AGGAGGTGTATTTCCGAGAGAGG | Type C |
| | GGAAATACACCTCCTTTCCATGG | Type C |
| | ACCTCCTTTCCATGGCTGCTAGG | Type C |
| | CCTCCTTTCCATGGCTGCTAGGG | Type C |
| | CCCTAGCAGCCATGGAAAGGAGG | Type C |
| | ACACCCTAGCAGCCATGGAAAGG | Type C |
| | GCAGCACACCCTAGCAGCCATGG | Type C |
| | TGCTAGGGTGTGCTGCCAACTGG | Type C |
| | GCTGCCAACTGGATCCTGCGCGG | Type C |
| | CTGCCAACTGGATCCTGCGCGGG | Type C |
| | CGTCCCGCGCAGGATCCAGTTGG | Type C |
| | AGACAAAGGACGTCCCGCGCAGG | Type C |
| | GTCCTTTGTCTACGTCCCGTCGG | Type C |
| | CGCCGACGGGACGTAGACAAAGG | Type C |
| | CCGCGGGATTCAGCGCCGACGGG | Type C |
| | CCCGTCGGCGCTGAATCCCGCGG | Type C |
| | TCCGCGGGATTCAGCGCCGACGG | Type C |
| | ATCCCGCGGACGACCCGTCTCGG | Type C |
| | TCCCGCGGACGACCCGTCTCGGG | Type C |
| | CCCCGAGACGGGTCGTCCGCGGG | Type C |
| | CCCGCGGACGACCCGTCTCGGGG | Type C |
| | GCCCCGAGACGGGTCGTCCGCGG | Type C |
| | CGACCCGTCTCGGGGCCGTTTGG | Type C |
| | GACCCGTCTCGGGGCCGTTTGGG | Type C |
| | ACCCGTCTCGGGGCCGTTTGGGG | Type C |
| | GCCCCAAACGGCCCCGAGACGGG | Type C |
| | AGCCCCAAACGGCCCCGAGACGG | Type C |
| | GGGGACGGTAGAGCCCCAAACGG | Type C |
| | ACGGCAGATGAAGAAGGGGACGG | Type C |
| | CGGAACGGCAGATGAAGAAGGGG | Type C |
| | CCGGAACGGCAGATGAAGAAGGG | Type C |
| | CCCTTCTTCATCTGCCGTTCCGG | Type C |
| | GCCGGAACGGCAGATGAAGAAGG | Type C |
| | TCTGCCGTTCCGGCCGACCACGG | Type C |
| | CTGCCGTTCCGGCCGACCACGGG | Type C |
| | TGCCGTTCCGGCCGACCACGGGG | Type C |
| | CGCCCCGTGGTCGGCCGGAACGG | Type C |
| | AGGTGCGCCCCGTGGTCGGCCGG | Type C |
| | AGAGAGGTGCGCCCCGTGGTCGG | Type C |
| | GTAAAGAGAGGTGCGCCCCGTGG | Type C |
| | GGGGCGCACCTCTCTTTACGCGG | Type C |
| | CGGGGAGACCGCGTAAAGAGAGG | Type C |
| | CAGATGAGAAGGCACAGACGGGG | Type C |
| | GCAGATGAGAAGGCACAGACGGG | Type C |
| | GGCAGATGAGAAGGCACAGACGG | Type C |
| | GTCTGTGCCTTCTCATCTGCCGG | Type C |
| | ACACGGTCCGGCAGATGAGAAGG | Type C |
| | GAAGCGAAGTGCACACGGTCCGG | Type C |
| | GAGGTGAAGCGAAGTGCACACGG | Type C |
| | CTTCACCTCTGCACGTCGCATGG | Type C |
| | GGTCTCCATGCGACGTGCAGAGG | Type C |
| | GACCACCGTGAACGCCCACCAGG | Type C |
| | GACCTGGTGGGCGTTCACGGTGG | Type C |
| | CAAGACCTGGTGGGCGTTCACGG | Type C |
| | CGCCCACCAGGTCTTGCCCAAGG | Type C |
| | GACCTTGGGCAAGACCTGGTGGG | Type C |
| | AGACCTTGGGCAAGACCTGGTGG | Type C |
| | GTAAGACCTTGGGCAAGACCTGG | Type C |
| | TGCCCAAGGTCTTACATAAGAGG | Type C |
| | GTCCTCTTATGTAAGACCTTGGG | Type C |
| | AGTCCTCTTATGTAAGACCTTGG | Type C |
| | GTCTTACATAAGAGGACTCTTGG | Type C |
| | AATGTCAACGACCGACCTTGAGG | Type C |
| | TTTGAAGTATGCCTCAAGGTCGG | Type C |
| | AGTCTTTGAAGTATGCCTCAAGG | Type C |
| | AAGACTGTGTGTTTAAAGACTGG | Type C |
| | AGACTGTGTGTTTAAAGACTGGG | Type C |
| | CTGTGTGTTTAAAGACTGGGAGG | Type C |
| | GTTTAAAGACTGGGAGGAGTTGG | Type C |
| | TTTAAAGACTGGGAGGAGTTGGG | Type C |
| | TTAAAGACTGGGAGGAGTTGGGG | Type C |
| | TAAAGACTGGGAGGAGTTGGGGG | Type C |
| | AGACTGGGAGGAGTTGGGGGAGG | Type C |
| | AGGAGTTGGGGGAGGAGATTAGG | Type C |
| | GGGGGAGGAGATTAGGTTAAAGG | Type C |
| | AGGTTAAAGGTCTTTGTACTAGG | Type C |
| | TTAAAGGTCTTTGTACTAGGAGG | Type C |
| | TCTTTGTACTAGGAGGCTGTAGG | Type C |
| | AGGAGGCTGTAGGCATAAATTGG | Type C |
| | GTGAAAAAGTTGCATGGTGCTGG | Type C |
| | GCAGAGGTGAAAAAGTTGCATGG | Type C |
| | AACATGAGATGATTAGGCAGAGG | Type C |
| | GACATGAACATGAGATGATTAGG | Type C |
| | AGCTTGGAGGCTTGAACAGTAGG | Type C |
| | CAAGCCTCCAAGCTGTGCCTTGG | Type C |
| | AAGCCTCCAAGCTGTGCCTTGGG | Type C |
| | CCTCCAAGCTGTGCCTTGGGTGG | Type C |
| | CCACCCAAGGCACAGCTTGGAGG | Type C |
| | AAGCCACCCAAGGCACAGCTTGG | Type C |
| | AGCTGTGCCTTGGGTGGCTTTGG | Type C |
| | GCTGTGCCTTGGGTGGCTTTGGG | Type C |
| | CTGTGCCTTGGGTGGCTTTGGGG | Type C |
| | CCATGCCCCAAAGCCACCCAAGG | Type C |
| | CCTTGGGTGGCTTTGGGGCATGG | Type C |
| | ATTGACCCGTATAAAGAATTTGG | Type C |
| | ATGCTCCAAATTCTTTATACGGG | Type C |
| | GATGCTCCAAATTCTTTATACGG | Type C |
| | TAAAGAATTTGGAGCATCTGTGG | Type C |
| | ATAGAAGGAAAGAAGTCAGAAGG | Type C |
| | TCGAGGAGATCTCGAATAGAAGG | Type C |
| | ACAGAGCAGAAGCGGTGTCGAGG | Type C |
| | ACACCGCTTCTGCTCTGTATCGG | Type C |
| | CACCGCTTCTGCTCTGTATCGGG | Type C |
| | CTCCCGATACAGAGCAGAAGCGG | Type C |
| | CGCTTCTGCTCTGTATCGGGAGG | Type C |
| | TCGGGAGGCCTTAGAGTCTCCGG | Type C |
| | ACAATGTTCCGGAGACTCTAAGG | Type C |
| | TGGTGAGGTGAACAATGTTCCGG | Type C |
| | CACCTCACCATACAGCACTCAGG | Type C |
| | TGCCTGAGTGCTGTATGGTGAGG | Type C |
| | TAGCTTGCCTGAGTGCTGTATGG | Type C |
| | TCAGGCAAGCTATTCTGTGTTGG | Type C |
| | CAGGCAAGCTATTCTGTGTTGGG | Type C |
| | AGGCAAGCTATTCTGTGTTGGGG | Type C |
| | TTGGGGTGAGTTGATGAATCTGG | Type C |
| | AGTTGATGAATCTGGCCACCTGG | Type C |
| | GTTGATGAATCTGGCCACCTGGG | Type C |
| | GATGAATCTGGCCACCTGGGTGG | Type C |
| | ATGAATCTGGCCACCTGGGTGGG | Type C |
| | CAAATTACTTCCCACCCAGGTGG | Type C |
| | CACCTGGGTGGGAAGTAATTTGG | Type C |
| | TTCCAAATTACTTCCCACCCAGG | Type C |
| | ATTTGGAAGACCCAGCATCCAGG | Type C |
| | TTTGGAAGACCCAGCATCCAGGG | Type C |
| | CTACTAATTCCCTGGATGCTGGG | Type C |
| | ACTACTAATTCCCTGGATGCTGG | Type C |
| | ATAGCTGACTACTAATTCCCTGG | Type C |
| | CAGCTATGTCAATGTTAATATGG | Type C |
| | AGCTATGTCAATGTTAATATGGG | Type C |
| | ACAATAGTTGTCTGATTTTTAGG | Type C |
| | TAAAAATCAGACAACTATTGTGG | Type C |
| | CACATTTCCTGTCTTACTTTTGG | Type C |
| | TTCTCTTCCAAAAGTAAGACAGG | Type C |
| | AGAAACTGTTCTTGAGTATTTGG | Type C |
| | CTTGAGTATTTGGTATCTTTTGG | Type C |
| | ATTTGGTATCTTTTGGAGTGTGG | Type C |
| | TTTGGTGGTCTGTAAGCGGGAGG | Type C |
| | GCATTTGGTGGTCTGTAAGCGGG | Type C |
| | GGCATTTGGTGGTCTGTAAGCGG | Type C |
| | GATAAGATAGGGGCATTTGGTGG | Type C |
| | GTTGATAAGATAGGGGCATTTGG | Type C |
| | CGGAAGTGTTGATAAGATAGGGG | Type C |
| | CCGGAAGTGTTGATAAGATAGGG | Type C |
| | CCCTATCTTATCAACACTTCCGG | Type C |
| | TCCGGAAGTGTTGATAAGATAGG | Type C |
| | CGTCTAACAACAGTAGTTTCCGG | Type C |
| | ACTACTGTTGTTAGACGACGAGG | Type C |
| | CTGTTGTTAGACGACGAGGCAGG | Type C |
| | CGAGGGAGTTCTTCTTCTAGGGG | Type C |
| | GCGAGGGAGTTCTTCTTCTAGGG | Type C |
| | GGCGAGGGAGTTCTTCTTCTAGG | Type C |
| | GATCTTCGTCTGCGAGGCGAGGG | Type C |
| | AGATCTTCGTCTGCGAGGCGAGG | Type C |
| | GATTGAGATCTTCGTCTGCGAGG | Type C |
| | GATTGAGATCTTCTGCGACGCGG | Type C |
| | GTCGCAGAAGATCTCAATCTCGG | Type C |
| | TCGCAGAAGATCTCAATCTCGGG | Type C |
| | ATCTCAATGTTAGTATCCCTTGG | Type C |
| | TAGTATCCCTTGGACTCATAAGG | Type C |
| | TATCCCTTGGACTCATAAGGTGG | Type C |
| | ATCCCTTGGACTCATAAGGTGGG | Type C |
| | TTCCCACCTTATGAGTCCAAGGG | Type C |
| | TTTCCCACCTTATGAGTCCAAGG | Type C |
| | CATAAGGTGGGAAACTTTACTGG | Type C |
| | ATAAGGTGGGAAACTTTACTGGG | Type C |
| | TACCTGTCTTTAATCCCGAGTGG | Type C |
| | TGCCACTCGGGATTAAAGACAGG | Type C |
| | AAGGAGGGAGTTTGCCACTCGGG | Type C |
| | AAAGGAGGGAGTTTGCCACTCGG | Type C |
| | AAATGAATGTGAGGAAAGGAGGG | Type C |
| | TAAATGAATGTGAGGAAAGGAGG | Type C |
| | CTGTAAATGAATGTGAGGAAAGG | Type C |
| | CTTTCCTCACATTCATTTACAGG | Type C |
| | TCCTCACATTCATTTACAGGAGG | Type C |
| | TCCTCCTGTAAATGAATGTGAGG | Type C |
| | TAATAGATGTCAACAATATGTGG | Type C |
| | AATAGATGTCAACAATATGTGGG | Type C |
| | TTTTTTCATTAACTGTAAGAGGG | Type C |
| | CTTTTTTCATTAACTGTAAGAGG | Type C |
| | CTCTTACAGTTAATGAAAAAAGG | Type C |
| | TAAAATTAATTATGCCTGCTAGG | Type C |
| | AGGTTAGGATAGAACCTAGCAGG | Type C |
| | GGCAAATATTTGGTAAGGTTAGG | Type C |
| | CTAAGGGCAAATATTTGGTAAGG | Type C |
| | TTTGTCTAAGGGCAAATATTTGG | Type C |
| | AAATATTTGCCCTTAGACAAAGG | Type C |
| | GGTTTAATGCCTTTGTCTAAGGG | Type C |
| | CGGTTTAATGCCTTTGTCTAAGG | Type C |
| | ACTGCATGTTCAGGATAATACGG | Type C |
| | TAATGATTAACTGCATGTTCAGG | Type C |
| | TTAATCATTACTTCAAAACTAGG | Type C |
| | GGCATTATTTACATACTCTGTGG | Type C |
| | TTATTTACATACTCTGTGGAAGG | Type C |
| | TTACATACTCTGTGGAAGGCTGG | Type C |
| | ACACGCAGCGCCTCATTTTGTGG | Type C |
| | CACGCAGCGCCTCATTTTGTGGG | Type C |
| | ATATGGTGACCCACAAAATGAGG | Type C |
| | TTTGTGGGTCACCATATTCTTGG | Type C |
| | TTGTGGGTCACCATATTCTTGGG | Type C |
| | AGCTCTTGTTCCCAAGAATATGG | Type C |
| | TGGGAACAAGAGCTACAGCATGG | Type C |
| | GGGAACAAGAGCTACAGCATGGG | Type C |
| | AACAAGAGCTACAGCATGGGAGG | Type C |
| | AGAGCTACAGCATGGGAGGTTGG | Type C |
| | TGGTCTTCCAAACCTCGACAAGG | Type C |
| | TTCCAAACCTCGACAAGGCATGG | Type C |
| | TCCAAACCTCGACAAGGCATGGG | Type C |
| | CCCCATGCCTTGTCGAGGTTTGG | Type C |
| | CCAAACCTCGACAAGGCATGGGG | Type C |
| | TTCGTCCCCATGCCTTGTCGAGG | Type C |
| | TCTTTCTGTTCCCAATCCTCTGG | Type C |
| | CTTTCTGTTCCCAATCCTCTGGG | Type C |
| | GGAAAGAATCCCAGAGGATTGGG | Type C |
| | GGGAAAGAATCCCAGAGGATTGG | Type C |
| | TGATCGGGAAAGAATCCCAGAGG | Type C |
| | ATTCTTTCCCGATCACCAGTTGG | Type C |
| | GCAGGGTCCAACTGGTGATCGGG | Type C |
| | CGCAGGGTCCAACTGGTGATCGG | Type C |
| | CACCAGTTGGACCCTGCGTTCGG | Type C |
| | CTCCGAACGCAGGGTCCAACTGG | Type C |
| | TTGAGTTGGCTCCGAACGCAGGG | Type C |
| | TTTGAGTTGGCTCCGAACGCAGG | Type C |
| | CCAATCTGGATTGTTTGAGTTGG | Type C |
| | CCAACTCAAACAATCCAGATTGG | Type C |
| | CAACTCAAACAATCCAGATTGGG | Type C |
| | TTGGGGTTGAAGTCCCAATCTGG | Type C |
| | TTGGGACTTCAACCCCAACAAGG | Type C |
| | TCAACCCCAACAAGGATCACTGG | Type C |
| | CTGGCCAGTGATCCTTGTTGGGG | Type C |
| | TCTGGCCAGTGATCCTTGTTGGG | Type C |
| | CTCTGGCCAGTGATCCTTGTTGG | Type C |
| | CAACAAGGATCACTGGCCAGAGG | Type C |
| | TCACTGGCCAGAGGCAAATCAGG | Type C |
| | TGGCCAGAGGCAAATCAGGTAGG | Type C |
| | GCTCCTACCTGATTTGCCTCTGG | Type C |
| | AGAGGCAAATCAGGTAGGAGCGG | Type C |
| | GAGGCAAATCAGGTAGGAGCGGG | Type C |
| | CAGGTAGGAGCGGGAGCATTCGG | Type C |
| | AGGTAGGAGCGGGAGCATTCGGG | Type C |
| | GGAGCGGGAGCATTCGGGCCAGG | Type C |
| | GAGCGGGAGCATTCGGGCCAGGG | Type C |
| | CCGTGTGGTGGGGTGAACCCTGG | Type C |
| | CCAGGGTTCACCCCACCACACGG | Type C |
| | GGGTTCACCCCACCACACGGCGG | Type C |
| | CAAAAGACCGCCGTGTGGTGGGG | Type C |
| | CCCACCACACGGCGGTCTTTTGG | Type C |
| | CCAAAAGACCGCCGTGTGGTGGG | Type C |
| | CCCAAAAGACCGCCGTGTGGTGG | Type C |
| | CCACCACACGGCGGTCTTTTGGG | Type C |
| | CACCACACGGCGGTCTTTTGGGG | Type C |
| | CACCCCAAAAGACCGCCGTGTGG | Type C |
| | CACACGGCGGTCTTTTGGGGTGG | Type C |
| | TCTTTTGGGGTGGAGCCCTCAGG | Type C |
| | GGGGTGGAGCCCTCAGGCTCAGG | Type C |
| | GGGTGGAGCCCTCAGGCTCAGGG | Type C |
| | TCAATATGCCCTGAGCCTGAGGG | Type C |
| | GTCAATATGCCCTGAGCCTGAGG | Type C |
| | GAGGCAGGAGGAGGTGCTGCTGG | Type C |
| | CGATTGGTGGAGGCAGGAGGAGG | Type C |
| | CTCCTCCTGCCTCCACCAATCGG | Type C |
| | TGCCGATTGGTGGAGGCAGGAGG | Type C |
| | GACTGCCGATTGGTGGAGGCAGG | Type C |
| | GCCTCCACCAATCGGCAGTCAGG | Type C |
| | TCCTGACTGCCGATTGGTGGAGG | Type C |
| | TCTTCCTGACTGCCGATTGGTGG | Type C |
| | CTGTCTTCCTGACTGCCGATTGG | Type C |
| | AGAGGTGGAGAGATGGGAGTAGG | Type C |
| | TCTCTTAGAGGTGGAGAGATGGG | Type C |
| | GTCTCTTAGAGGTGGAGAGATGG | Type C |
| | GGATGACTGTCTCTTAGAGGTGG | Type C |
| | TGAGGATGACTGTCTCTTAGAGG | Type C |
| | TCTAAGAGACAGTCATCCTCAGG | Type C |
| | GTCATCCTCAGGCCATGCAGTGG | Type C |
| | GAGTTCCACTGCATGGCCTGAGG | Type C |
| | TGTTGTGGAGTTCCACTGCATGG | Type C |
| | GGTCTCCATGCGACGTGCAGAGG | Type D |
| | CTTCACCTCTGCACGTCGCATGG | Type D |
| | GAGGTGAAGCGAAGTGCACACGG | Type D |
| | GAAGCGAAGTGCACACGGTCCGG | Type D |
| | CATTCGGTGGGCGTTCACGGTGG | Type D |
| | CAACATTCGGTGGGCGTTCACGG | Type D |
| | CGCCCACCGAATGTTGCCCAAGG | Type D |
| | ACACGGTCCGGCAGATGAGAAGG | Type D |
| | GACCTTGGGCAACATTCGGTGGG | Type D |
| | AGACCTTGGGCAACATTCGGTGG | Type D |
| | GTAAGACCTTGGGCAACATTCGG | Type D |
| | GTCTGTGCCTTCTCATCTGCCGG | Type D |
| | GGCAGATGAGAAGGCACAGACGG | Type D |
| | GCAGATGAGAAGGCACAGACGGG | Type D |
| | CAGATGAGAAGGCACAGACGGGG | Type D |
| | TGCCCAAGGTCTTACATAAGAGG | Type D |
| | GTCCTCTTATGTAAGACCTTGGG | Type D |
| | AGTCCTCTTATGTAAGACCTTGG | Type D |
| | GTCTTACATAAGAGGACTCTTGG | Type D |
| | CGGGGAGTCCGCGTAAAGAGAGG | Type D |
| | GGGGCGCACCTCTCTTTACGCGG | Type D |
| | GTAAAGAGAGGTGCGCCCCGTGG | Type D |
| | AGAGAGGTGCGCCCCGTGGTCGG | Type D |
| | AGGTGCGCCCCGTGGTCGGTCGG | Type D |
| | AATGTCAACGACCGACCTTGAGG | Type D |
| | CGCCCCGTGGTCGGTCGGAACGG | Type D |
| | TGCCGTTCCGACCGACCACGGGG | Type D |
| | CTGCCGTTCCGACCGACCACGGG | Type D |
| | TCTGCCGTTCCGACCGACCACGG | Type D |
| | TGGTCGGTCGGAACGGCAGACGG | Type D |
| | TTTGAAGTATGCCTCAAGGTCGG | Type D |
| | GTCGGAACGGCAGACGGAGAAGG | Type D |
| | AGTCTTTGAAGTATGCCTCAAGG | Type D |
| | TCGGAACGGCAGACGGAGAAGGG | Type D |
| | CGGAACGGCAGACGGAGAAGGGG | Type D |
| | AAGACTGTTTGTTTAAAGACTGG | Type D |
| | AGACTGTTTGTTTAAAGACTGGG | Type D |
| | CTGTTTGTTTAAAGACTGGGAGG | Type D |
| | GTTTAAAGACTGGGAGGAGTTGG | Type D |
| | TTTAAAGACTGGGAGGAGTTGGG | Type D |
| | TTAAAGACTGGGAGGAGTTGGGG | Type D |
| | TAAAGACTGGGAGGAGTTGGGGG | Type D |
| | AGTCCCAAGCGACCCCGAGAAGG | Type D |
| | GTCCCAAGCGACCCCGAGAAGGG | Type D |
| | AGACTGGGAGGAGTTGGGGGAGG | Type D |
| | GACCCTTCTCGGGGTCGCTTGGG | Type D |
| | CGACCCTTCTCGGGGTCGCTTGG | Type D |
| | CCCCGAGAAGGGTCGTCCGCAGG | Type D |
| | CCTGCGGACGACCCTTCTCGGGG | Type D |
| | TCCTGCGGACGACCCTTCTCGGG | Type D |
| | ATCCTGCGGACGACCCTTCTCGG | Type D |
| | GGGGGAGGAGATTAGATTAAAGG | Type D |
| | TCCGCAGGATTCAGCGCCGACGG | Type D |
| | CCCGTCGGCGCTGAATCCTGCGG | Type D |
| | CCGCAGGATTCAGCGCCGACGGG | Type D |
| | AGATTAAAGGTCTTTGTACTAGG | Type D |
| | TTAAAGGTCTTTGTACTAGGAGG | Type D |
| | TCTTTGTACTAGGAGGCTGTAGG | Type D |
| | CGCCGACGGGACGTAAACAAAGG | Type D |
| | GTCCTTTGTTTACGTCCCGTCGG | Type D |
| | AGGAGGCTGTAGGCATAAATTGG | Type D |
| | AAACAAAGGACGTCCCGCGCAGG | Type D |
| | CGTCCCGCGCAGGATCCAGTTGG | Type D |
| | CTGCCAACTGGATCCTGCGCGGG | Type D |
| | GCTGCCAACTGGATCCTGCGCGG | Type D |
| | TGCTAGGCTGTGCTGCCAACTGG | Type D |
| | GTGAAAAAGTTGCATGGTGCTGG | Type D |
| | GCAGCACAGCCTAGCAGCCATGG | Type D |
| | GCAGAGGTGAAAAAGTTGCATGG | Type D |
| | ACATCGTATCCATGGCTGCTAGG | Type D |
| | AACAAGAGATGATTAGGCAGAGG | Type D |
| | GCAAATATACATCGTATCCATGG | Type D |
| | ATGGATACGATGTATATTTGCGG | Type D |
| | TGGATACGATGTATATTTGCGGG | Type D |
| | GACATGAACAAGAGATGATTAGG | Type D |
| | ACGATGTATATTTGCGGGAGAGG | Type D |
| | AGCTTGGAGGCTTGAACAGTAGG | Type D |
| | CAAGCCTCCAAGCTGTGCCTTGG | Type D |
| | AAGCCTCCAAGCTGTGCCTTGGG | Type D |
| | CCTCCAAGCTGTGCCTTGGGTGG | Type D |
| | CCACCCAAGGCACAGCTTGGAGG | Type D |
| | AAGCCACCCAAGGCACAGCTTGG | Type D |
| | AGCTGTGCCTTGGGTGGCTTTGG | Type D |
| | GCTGTGCCTTGGGTGGCTTTGGG | Type D |
| | CTGTGCCTTGGGTGGCTTTGGGG | Type D |
| | GGTCTGGAGCAAACATTATCGGG | Type D |
| | AGGTCTGGAGCAAACATTATCGG | Type D |
| | CCTTGGGTGGCTTTGGGGCATGG | Type D |
| | CCATGCCCCAAAGCCACCCAAGG | Type D |
| | TGTTTTGCTCGCAGCAGGTCTGG | Type D |
| | CCTGCTGCGAGCAAAACAAGCGG | Type D |
| | CCGCTTGTTTTGCTCGCAGCAGG | Type D |
| | TGCGAGCAAAACAAGCGGCTAGG | Type D |
| | ATCGACCCTTATAAAGAATTTGG | Type D |
| | TAGCTCCAAATTCTTTATAAGGG | Type D |
| | GTAGCTCCAAATTCTTTATAAGG | Type D |
| | CGGCTAGGAGTTCCGCAGTATGG | Type D |
| | TAAAGAATTTGGAGCTACTGTGG | Type D |
| | AGGAGTTCCGCAGTATGGATCGG | Type D |
| | TCCGCAGTATGGATCGGCAGAGG | Type D |
| | TCCTCTGCCGATCCATACTGCGG | Type D |
| | GATCGGCAGAGGAGCCGAAAAGG | Type D |
| | GCGCGTGCGTGGAACCTTTTCGG | Type D |
| | ACTGAAGGAAAGAAGTCAGAAGG | Type D |
| | AGGTTCCACGCACGCGCTGATGG | Type D |
| | ATGGGCCATCAGCGCGTGCGTGG | Type D |
| | TCTAGAAGATCTCGTACTGAAGG | Type D |
| | ACTGGCTGGGGCTTGGTCATGGG | Type D |
| | CACTGGCTGGGGCTTGGTCATGG | Type D |
| | CATGACCAAGCCCCAGCCAGTGG | Type D |
| | ATGACCAAGCCCCAGCCAGTGGG | Type D |
| | TGACCAAGCCCCAGCCAGTGGGG | Type D |
| | GACCAAGCCCCAGCCAGTGGGGG | Type D |
| | AACCCCCACTGGCTGGGGCTTGG | Type D |
| | GACGCAACCCCCACTGGCTGGGG | Type D |
| | ATACCGCCTCAGCTCTGTATCGG | Type D |
| | TACCGCCTCAGCTCTGTATCGGG | Type D |
| | TGACGCAACCCCCACTGGCTGGG | Type D |
| | CTGACGCAACCCCCACTGGCTGG | Type D |
| | TTCCCGATACAGAGCTGAGGCGG | Type D |
| | GGCTTCCCGATACAGAGCTGAGG | Type D |
| | TTTGCTGACGCAACCCCCACTGG | Type D |
| | GGGTTGCGTCAGCAAACACTTGG | Type D |
| | GCAAACACTTGGCACAGACCTGG | Type D |
| | ACAATGCTCAGGAGACTCTAAGG | Type D |
| | GGCACAGACCTGGCCGTTGCCGG | Type D |
| | GCACAGACCTGGCCGTTGCCGGG | Type D |
| | TGGTGAGGTGAACAATGCTCAGG | Type D |
| | ACCTGGCCGTTGCCGGGCAACGG | Type D |
| | CCTGGCCGTTGCCGGGCAACGGG | Type D |
| | CCCGTTGCCCGGCAACGGCCAGG | Type D |
| | CTGGCCGTTGCCGGGCAACGGGG | Type D |
| | TTTACCCCGTTGCCCGGCAACGG | Type D |
| | GTTGCCGGGCAACGGGGTAAAGG | Type D |
| | CACCTCACCATACTGCACTCAGG | Type D |
| | TGAACCTTTACCCCGTTGCCCGG | Type D |
| | TGCCTGAGTGCAGTATGGTGAGG | Type D |
| | GGGCAACGGGGTAAAGGTTCAGG | Type D |
| | TTGCTTGCCTGAGTGCAGTATGG | Type D |
| | TCAGGCAAGCAATTCTTTGCTGG | Type D |
| | CAGGCAAGCAATTCTTTGCTGGG | Type D |
| | AGGCAAGCAATTCTTTGCTGGGG | Type D |
| | GGCAAGCAATTCTTTGCTGGGGG | Type D |
| | GCAAGCAATTCTTTGCTGGGGGG | Type D |
| | CAGGTATTGTTTACACAGAAAGG | Type D |
| | CACAGAAAGGCCTTGTAAGTTGG | Type D |
| | AACTAATGACTCTAGCTACCTGG | Type D |
| | ACTAATGACTCTAGCTACCTGGG | Type D |
| | CACTTTCTCGCCAACTTACAAGG | Type D |
| | AATGACTCTAGCTACCTGGGTGG | Type D |
| | ATGACTCTAGCTACCTGGGTGGG | Type D |
| | TACCTGGGTGGGTGTTAATTTGG | Type D |
| | TTCCAAATTAACACCCACCCAGG | Type D |
| | TGCATGTATTCAATCTAAGCAGG | Type D |
| | AGATTGAATACATGCATACAAGG | Type D |
| | GATTGAATACATGCATACAAGGG | Type D |
| | ACTACTAGGTCTCTAGATGCTGG | Type D |
| | GCATACAAGGGCATTAACGCAGG | Type D |
| | TGTTGACATAACTGACTACTAGG | Type D |
| | CAGTTATGTCAACACTAATATGG | Type D |
| | AGTTATGTCAACACTAATATGGG | Type D |
| | GGATAACCACATTGTGTAAATGG | Type D |
| | GATAACCACATTGTGTAAATGGG | Type D |
| | ATAACCACATTGTGTAAATGGGG | Type D |
| | GCTGCCCCATTTACACAATGTGG | Type D |
| | CTAATATGGGCCTAAAGTTCAGG | Type D |
| | ACAAGAGTTGCCTGAACTTTAGG | Type D |
| | TAAAGTTCAGGCAACTCTTGTGG | Type D |
| | CAACGAATTGTGGGTCTTTTGGG | Type D |
| | TCAACGAATTGTGGGTCTTTTGG | Type D |
| | AAAGTATGTCAACGAATTGTGGG | Type D |
| | GAAAGTATGTCAACGAATTGTGG | Type D |
| | CACATTTCTTGTCTCACTTTTGG | Type D |
| | TGACATACTTTCCAATCAATAGG | Type D |
| | CTATTAACAGGCCTATTGATTGG | Type D |
| | CAATCAATAGGCCTGTTAATAGG | Type D |
| | AGAAACCGTTATAGAGTATTTGG | Type D |
| | TTAGAAAACTTCCTATTAACAGG | Type D |
| | AGACACCAAATACTCTATAACGG | Type D |
| | ATAGAGTATTTGGTGTCTTTCGG | Type D |
| | ATTTGGTGTCTTTCGGAGTGTGG | Type D |
| | TTTTGTATGATGTGTTCTTGTGG | Type D |
| | TATGATGTGTTCTTGTGGCAAGG | Type D |
| | TTTGGTGGTCTATAAGCTGGAGG | Type D |
| | GCATTTGGTGGTCTATAAGCTGG | Type D |
| | GATAGGATAGGGGCATTTGGTGG | Type D |
| | GTTATGTCATTGGAAGTTATGGG | Type D |
| | GTTGATAGGATAGGGGCATTTGG | Type D |
| | GGTTATGTCATTGGAAGTTATGG | Type D |
| | CGGAAGTGTTGATAGGATAGGGG | Type D |
| | CCGGAAGTGTTGATAGGATAGGG | Type D |
| | CCCTATCCTATCAACACTTCCGG | Type D |
| | TCCGGAAGTGTTGATAGGATAGG | Type D |
| | AATTTTATGGGTTATGTCATTGG | Type D |
| | TAGTTTCCGGAAGTGTTGATAGG | Type D |
| | GGTTACTCTCTGAATTTTATGGG | Type D |
| | GGGTTACTCTCTGAATTTTATGG | Type D |
| | CGTCTAACAACAGTAGTTTCCGG | Type D |
| | ACTACTGTTGTTAGACGACGAGG | Type D |
| | CTGTTGTTAGACGACGAGGCAGG | Type D |
| | CCCCATCTCTTTGTTTTGTTAGG | Type D |
| | CCTAACAAAACAAAGAGATGGGG | Type D |
| | CCCTAACAAAACAAAGAGATGGG | Type D |
| | CCCATCTCTTTGTTTTGTTAGGG | Type D |
| | ACCCTAACAAAACAAAGAGATGG | Type D |
| | CGAGGGAGTTCTTCTTCTAGGGG | Type D |
| | GCGAGGGAGTTCTTCTTCTAGGG | Type D |
| | GGCGAGGGAGTTCTTCTTCTAGG | Type D |
| | CTCCCTCGCCTCGCAGACGAAGG | Type D |
| | GACCTTCGTCTGCGAGGCGAGGG | Type D |
| | ACCCAAAGACAAAAGAAAATTGG | Type D |
| | ACCAATTTTCTTTTGTCTTTGGG | Type D |
| | AGACCTTCGTCTGCGAGGCGAGG | Type D |
| | TACCAATTTTCTTTTGTCTTTGG | Type D |
| | GATTGAGACCTTCGTCTGCGAGG | Type D |
| | CAAAAGAAAATTGGTAACAGCGG | Type D |
| | AATTGGTAACAGCGGTAAAAAGG | Type D |
| | ATTGGTAACAGCGGTAAAAAGGG | Type D |
| | GATTGAGATCTTCTGCGACGCGG | Type D |
| | GTCGCAGAAGATCTCAATCTCGG | Type D |
| | TCGCAGAAGATCTCAATCTCGGG | Type D |
| | CTCAAGATGCTGTACAGACTTGG | Type D |
| | ACCTCAATGTTAGTATTCCTTGG | Type D |
| | TCCAAGGAATACTAACATTGAGG | Type D |
| | TATGGATGATGTGGTATTGGGGG | Type D |
| | ATATGGATGATGTGGTATTGGGG | Type D |
| | TATATGGATGATGTGGTATTGGG | Type D |
| | TTATATGGATGATGTGGTATTGG | Type D |
| | TAGTATTCCTTGGACTCATAAGG | Type D |
| | TATTCCTTGGACTCATAAGGTGG | Type D |
| | ATTCCTTGGACTCATAAGGTGGG | Type D |
| | TTTCAGTTATATGGATGATGTGG | Type D |
| | TTCCTTGGACTCATAAGGTGGGG | Type D |
| | TTCCCCACCTTATGAGTCCAAGG | Type D |
| | CTGTTTGGCTTTCAGTTATATGG | Type D |
| | CATAAGGTGGGGAACTTTACTGG | Type D |
| | TATAACTGAAAGCCAAACAGTGG | Type D |
| | ATAACTGAAAGCCAAACAGTGGG | Type D |
| | TAACTGAAAGCCAAACAGTGGGG | Type D |
| | AACTGAAAGCCAAACAGTGGGGG | Type D |
| | TAGGGCTTTCCCCCACTGTTTGG | Type D |
| | CATTTGTTCAGTGGTTCGTAGGG | Type D |
| | CCATTTGTTCAGTGGTTCGTAGG | Type D |
| | CCTACGAACCACTGAACAAATGG | Type D |
| | TACTAGTGCCATTTGTTCAGTGG | Type D |
| | TACCTGTCTTTAATCCTCATTGG | Type D |
| | TTCCAATGAGGATTAAAGACAGG | Type D |
| | TGGCACTAGTAAACTGAGCCAGG | Type D |
| | AAAGATGGTGTTTTCCAATGAGG | Type D |
| | GTAAACTGAGCCAGGAGAAACGG | Type D |
| | TAAACTGAGCCAGGAGAAACGGG | Type D |
| | GAGCCAGGAGAAACGGGCTGAGG | Type D |
| | AAATGTATATTAGGAAAAGATGG | Type D |
| | GGGCCTCAGCCCGTTTCTCCTGG | Type D |
| | TCTTGGTGTAAATGTATATTAGG | Type D |
| | GGGCTGAGGCCCACTCCCATAGG | Type D |
| | CGGAAAATTCCTATGGGAGTGGG | Type D |
| | TCGGAAAATTCCTATGGGAGTGG | Type D |
| | CACATTTTTTGATAATGTCTTGG | Type D |
| | GGCTTTCGGAAAATTCCTATGGG | Type D |
| | GGGCTTTCGGAAAATTCCTATGG | Type D |
| | TAGGAATTTTCCGAAAGCCCAGG | Type D |
| | TTTCCGAAAGCCCAGGATGATGG | Type D |
| | AAAAAATGTGAACAGTTTGTAGG | Type D |
| | TTCCGAAAGCCCAGGATGATGGG | Type D |
| | ATCCCATCATCCTGGGCTTTCGG | Type D |
| | GAAAGCCCAGGATGATGGGATGG | Type D |
| | AAAGCCCAGGATGATGGGATGGG | Type D |
| | TATTCCCATCCCATCATCCTGGG | Type D |
| | GTATTCCCATCCCATCATCCTGG | Type D |
| | GGATGATGGGATGGGAATACAGG | Type D |
| | TTTTCTCATTAACTGTAAGTGGG | Type D |
| | CTTTTCTCATTAACTGTAAGTGG | Type D |
| | CAGGTGCAATTTCCGTCCGAAGG | Type D |
| | GCAATTTCCGTCCGAAGGTTTGG | Type D |
| | TGCTGTACCAAACCTTCGGACGG | Type D |
| | CTGTTGCTGTACCAAACCTTCGG | Type D |
| | CGAAGGTTTGGTACAGCAACAGG | Type D |
| | AGGTTTGGTACAGCAACAGGAGG | Type D |
| | TGCAATTGATTATGCCTGCTAGG | Type D |
| | GGTTTGGTACAGCAACAGGAGGG | Type D |
| | GCAACAGGAGGGATACATAGAGG | Type D |
| | GCCTGCTAGGTTTTATCCAAAGG | Type D |
| | ACCTTTGGATAAAACCTAGCAGG | Type D |
| | GGTAAATATTTGGTAACCTTTGG | Type D |
| | GGTTACCAAATATTTACCATTGG | Type D |
| | TCCTTGAGCAGTAGTCATGCAGG | Type D |
| | ACCTGCATGACTACTGCTCAAGG | Type D |
| | CTTATCCAATGGTAAATATTTGG | Type D |
| | CAAATATTTACCATTGGATAAGG | Type D |
| | GAGCAGTAGTCATGCAGGTTCGG | Type D |
| | AAATATTTACCATTGGATAAGGG | Type D |
| | GTAGTCATGCAGGTTCGGCATGG | Type D |
| | GGTTTAATACCCTTATCCAATGG | Type D |
| | GGTTCGGCATGGTCCCGTGCTGG | Type D |
| | TCGGCATGGTCCCGTGCTGGTGG | Type D |
| | TGGTCCCGTGCTGGTGGTTGAGG | Type D |
| | GGATCCTCAACCACCAGCACGGG | Type D |
| | ACTAGATGTTCTGGATAATAAGG | Type D |
| | AGGATCCTCAACCACCAGCACGG | Type D |
| | GTGCTGGTGGTTGAGGATCCTGG | Type D |
| | TAATGATTAACTAGATGTTCTGG | Type D |
| | GTTGAGGATCCTGGAATTAGAGG | Type D |
| | TCCTGGAATTAGAGGACAAACGG | Type D |
| | CCTGGAATTAGAGGACAAACGGG | Type D |
| | CCCGTTTGTCCTCTAATTCCAGG | Type D |
| | TGTGTAAATAGTGTCTAGTTTGG | Type D |
| | GACACTATTTACACACTCTATGG | Type D |
| | CTATTTACACACTCTATGGAAGG | Type D |
| | TTGGTTCTTCTGGACTATCAAGG | Type D |
| | TTTACACACTCTATGGAAGGCGG | Type D |
| | TTACACACTCTATGGAAGGCGGG | Type D |
| | CATCTTCTTGTTGGTTCTTCTGG | Type D |
| | GAAGAACCAACAAGAAGATGAGG | Type D |
| | GCTATGCCTCATCTTCTTGTTGG | Type D |
| | AGAAGATGAGGCATAGCAGCAGG | Type D |
| | GGCATAGCAGCAGGATGAAGAGG | Type D |
| | ACACATAGCGCCTCATTTTGTGG | Type D |
| | CACATAGCGCCTCATTTTGTGGG | Type D |
| | ATATGGTGACCCACAAAATGAGG | Type D |
| | TTTGTGGGTCACCATATTCTTGG | Type D |
| | TTGTGGGTCACCATATTCTTGGG | Type D |
| | GTTATCGCTGGATGTGTCTGCGG | Type D |
| | AGATCTTGTTCCCAAGAATATGG | Type D |
| | AGACACATCCAGCGATAACCAGG | Type D |
| | TGGGAACAAGATCTACAGCATGG | Type D |
| | CAACTTGTCCTGGTTATCGCTGG | Type D |
| | GGGAACAAGATCTACAGCATGGG | Type D |
| | CAGCGATAACCAGGACAAGTTGG | Type D |
| | GGAACAAGATCTACAGCATGGGG | Type D |
| | CGATAACCAGGACAAGTTGGAGG | Type D |
| | ACCAGGACAAGTTGGAGGACAGG | Type D |
| | TCCTGTCCTCCAACTTGTCCTGG | Type D |
| | AGGACAAGTTGGAGGACAGGAGG | Type D |
| | CAAGTTGGAGGACAGGAGGTTGG | Type D |
| | ACAGGAGGTTGGTGAGTGATTGG | Type D |
| | TCTTTCCACCAGCAATCCTCTGG | Type D |
| | CTTTCCACCAGCAATCCTCTGGG | Type D |
| | GGAGGTTGGTGAGTGATTGGAGG | Type D |
| | GAATCCCAGAGGATTGCTGGTGG | Type D |
| | GTTGGTGAGTGATTGGAGGTTGG | Type D |
| | TTGGTGAGTGATTGGAGGTTGGG | Type D |
| | TGGTGAGTGATTGGAGGTTGGGG | Type D |
| | AAAGAATCCCAGAGGATTGCTGG | Type D |
| | TGGTCGGGAAAGAATCCCAGAGG | Type D |
| | AGGTTGGGGACTGCGAATTTTGG | Type D |
| | ATTCTTTCCCGACCACCAGTTGG | Type D |
| | GCTGGATCCAACTGGTGGTCGGG | Type D |
| | GGCTGGATCCAACTGGTGGTCGG | Type D |
| | CGAATTTTGGCCAAGACACACGG | Type D |
| | TGAAGGCTGGATCCAACTGGTGG | Type D |
| | CTCTGAAGGCTGGATCCAACTGG | Type D |
| | GGGGGAACTACCGTGTGTCTTGG | Type D |
| | GCTGTGTTTGCTCTGAAGGCTGG | Type D |
| | ATTTGCTGTGTTTGCTCTGAAGG | Type D |
| | ACTTCTCTCAATTTTCTAGGGGG | Type D |
| | CAAACACAGCAAATCCAGATTGG | Type D |
| | AAACACAGCAAATCCAGATTGGG | Type D |
| | GACTTCTCTCAATTTTCTAGGGG | Type D |
| | GGACTTCTCTCAATTTTCTAGGG | Type D |
| | TGGACTTCTCTCAATTTTCTAGG | Type D |
| | TTGGGATTGAAGTCCCAATCTGG | Type D |
| | TTGGGACTTCAATCCCAACAAGG | Type D |
| | CGCAGAGTCTAGACTCGTGGTGG | Type D |
| | CACCACGAGTCTAGACTCTGCGG | Type D |
| | TACCGCAGAGTCTAGACTCGTGG | Type D |
| | TCAATCCCAACAAGGACACCTGG | Type D |
| | TCTGGCCAGGTGTCCTTGTTGGG | Type D |
| | GTCTGGCCAGGTGTCCTTGTTGG | Type D |
| | CTAGACTCTGCGGTATTGTGAGG | Type D |
| | CACCTGGCCAGACGCCAACAAGG | Type D |
| | TACCTTGTTGGCGTCTGGCCAGG | Type D |
| | TGGCCAGACGCCAACAAGGTAGG | Type D |
| | GCTCCTACCTTGTTGGCGTCTGG | Type D |
| | GACGCCAACAAGGTAGGAGCTGG | Type D |
| | TGCTCCAGCTCCTACCTTGTTGG | Type D |
| | AAGGTAGGAGCTGGAGCATTCGG | Type D |
| | AGGTAGGAGCTGGAGCATTCGGG | Type D |
| | AGGAGCTGGAGCATTCGGGCTGG | Type D |
| | GGAGCTGGAGCATTCGGGCTGGG | Type D |
| | ACCCCGCCTGTAACACGAGAAGG | Type D |
| | CCCTTCTCGTGTTACAGGCGGGG | Type D |
| | CCCCGCCTGTAACACGAGAAGGG | Type D |
| | CCCGCCTGTAACACGAGAAGGGG | Type D |
| | CCCCTTCTCGTGTTACAGGCGGG | Type D |
| | ACCCCTTCTCGTGTTACAGGCGG | Type D |
| | AGGACCCCTTCTCGTGTTACAGG | Type D |
| | GTAACACGAGAAGGGGTCCTAGG | Type D |
| | CTGGGTTTCACCCCACCGCACGG | Type D |
| | GGTTTCACCCCACCGCACGGAGG | Type D |
| | CAAAAGGCCTCCGTGCGGTGGGG | Type D |
| | CCAAAAGGCCTCCGTGCGGTGGG | Type D |
| | CCCACCGCACGGAGGCCTTTTGG | Type D |
| | CCCAAAAGGCCTCCGTGCGGTGG | Type D |
| | CCACCGCACGGAGGCCTTTTGGG | Type D |
| | CACCGCACGGAGGCCTTTTGGGG | Type D |
| | CACCCCAAAAGGCCTCCGTGCGG | Type D |
| | CGCACGGAGGCCTTTTGGGGTGG | Type D |
| | AACATCACATCAGGATTCCTAGG | Type D |
| | AACATGGAGAACATCACATCAGG | Type D |
| | CCTTTTGGGGTGGAGCCCTCAGG | Type D |
| | CCTGAGGGCTCCACCCCAAAAGG | Type D |
| | GGGGTGGAGCCCTCAGGCTCAGG | Type D |
| | GGGTGGAGCCCTCAGGCTCAGGG | Type D |
| | ATGTTCTCCATGTTCAGCGCAGG | Type D |
| | TGTTCTCCATGTTCAGCGCAGGG | Type D |
| | GTAGTATGCCCTGAGCCTGAGGG | Type D |
| | TGGGGACCCTGCGCTGAACATGG | Type D |
| | TGTAGTATGCCCTGAGCCTGAGG | Type D |
| | GTCAATCTTCTCGAGGATTGGGG | Type D |
| | CGTCAATCTTCTCGAGGATTGGG | Type D |
| | TCGTCAATCTTCTCGAGGATTGG | Type D |
| | CCTTATCGTCAATCTTCTCGAGG | Type D |
| | CCTCGAGAAGATTGACGATAAGG | Type D |
| | CTCGAGAAGATTGACGATAAGGG | Type D |
| | GAAGATTGACGATAAGGGAGAGG | Type D |
| | GAGGCAGGAGGCGGATTTGCTGG | Type D |
| | CGATTGGTGGAGGCAGGAGGCGG | Type D |
| | GATAAGGGAGAGGCAGTAGTCGG | Type D |
| | TGGCGATTGGTGGAGGCAGGAGG | Type D |
| | GTCTGGCGATTGGTGGAGGCAGG | Type D |
| | GGAGAGGCAGTAGTCGGAACAGG | Type D |
| | GAGAGGCAGTAGTCGGAACAGGG | Type D |
| | GCCTCCACCAATCGCCAGACAGG | Type D |
| | TCCTGTCTGGCGATTGGTGGAGG | Type D |
| | CCTTCCTGTCTGGCGATTGGTGG | Type D |
| | CCACCAATCGCCAGACAGGAAGG | Type D |
| | CTGCCTTCCTGTCTGGCGATTGG | Type D |
| | GGGTAGGCTGCCTTCCTGTCTGG | Type D |
| | AGGGTTTACTGCTCCTGAACTGG | Type D |
| | AAAGGTGGAGACAGCGGGGTAGG | Type D |
| | CCTGAACTGGAGCCACCAGCAGG | Type D |
| | CCTGCTGGTGGCTCCAGTTCAGG | Type D |
| | CTGAACTGGAGCCACCAGCAGGG | Type D |
| | TCTCAAAGGTGGAGACAGCGGGG | Type D |
| | TTCTCAAAGGTGGAGACAGCGGG | Type D |
| | TTTCTCAAAGGTGGAGACAGCGG | Type D |
| | AGCCACCAGCAGGGAAATACAGG | Type D |
| | GGCCTGTATTTCCCTGCTGGTGG | Type D |
| | GGATGAGTGTTTCTCAAAGGTGG | Type D |
| | AGAGGCCTGTATTTCCCTGCTGG | Type D |
| | TGAGGATGAGTGTTTCTCAAAGG | Type D |
| | TTTGAGAAACACTCATCCTCAGG | Type D |
| | GAAATACAGGCCTCTCACTCTGG | Type D |
| | AAATACAGGCCTCTCACTCTGGG | Type D |
| | CTCATCCTCAGGCCATGCAGTGG | Type D |
| | CTGCAAGATCCCAGAGTGAGAGG | Type D |
| | CTCTGGGATCTTGCAGAGTTTGG | Type D |
| | ATCTTGCAGAGTTTGGTGAAAGG | Type D |
| | CAGAGTTTGGTGAAAGGTTGTGG | Type D |
| | GAATTCCACTGCATGGCCTGAGG | Type D |
| | GGTTGTGGAATTCCACTGCATGG | Type D |

### (2) sgRNA functional screening

293FT was used to construct a reporter cell line for type B, type C and type D HBV surface antigen (HBsAg), core antigen (HBcAg), X protein (HBx) and polymerase (Pol), and the schematic diagram is shown in FIG. 1A. The genomic sequence containing the CpG island and regulatory elements before the protein transcription start site was retained, and the gene sequences of the self-cleaving peptide P2A and EGFP were fused after the 38th amino acid of the HBV surface antigen (HBsAg), the 129th amino acid of the core antigen (HBcAg), the 77th amino acid of the X protein (HBx), and the 177th amino acid of the polymerase (Pol), respectively. The reporter gene was integrated into the 293FT genome using the PiggyBac transposon system, and a stably transfected cell line was constructed. The intensity of fluorescence was used to reflect the expression level of the protein, thereby quickly performing functional validation and preliminary screening of sgRNAs near the CpG island.

The coding sequence of the self-cleaving peptide P2A used in this example is:

GCTACTAACTTCAGCCTGCTGAAGCAGGCTGGCGACGTGGAGGAGAACCCTGGACCT
GCCACC (SEQ ID NO: 1188).

The coding sequence of EGFP used is:

ATGGTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAGGGCGA
TGCCACCTACGGCAAGCTGACCCTGAAGTTCATCTGCACCACCGGCAAGCTGCCCGT
GCCCTGGCCCACCCTCGTGACCACCCTGACCTACGGCGTGCAGTGCTTCAGCCGCTA
CCCCGACCACATGAAGCAGCACGACTTCTTCAAGTCCGCCATGCCCGAAGGCTACGT
CCAGGAGCGCACCATCTTCTTCAAGGACGACGGCAACTACAAGACCCGCGCCGAGGT
GAAGTTCGAGGGCGACACCCTGGTGAACCGCATCGAGCTGAAGGGCATCGACTTCAA
GGAGGACGGCAACATCCTGGGGCACAAGCTGGAGTACAACTACAACAGCCACAACG
TCTATATCATGGCCGACAAGCAGAAGAACGGCATCAAGGTGAACTTCAAGATCCGCC
ACAACATCGAGGACGGCAGCGTGCAGCTCGCCGACCACTACCAGCAGAACACCCCC
ATCGGCGACGGCCCCGTGCTGCTGCCCGACAACCACTACCTGAGCACCCAGTCCGCC
CTGAGCAAAGACCCCAACGAGAAGCGCGATCACATGGTCCTGCTGGAGTTCGTGACC
GCCGCCGGGATCACTCTCGGCATGGACGAGCT (SEQ ID NO: 1189).

The screening scheme for sgRNA functional validation using the above-mentioned reporter cell line was designed as follows (FIG. 1B): after the reporter cells were plated, the composition EPIREG described in the present application (the domain of the composition was DNMT3A-hDNMT3L-xten80-ZIM3-xten80-dCas9, with an amino acid sequence as set forth in SEQ ID NO: 1194, and a nucleic acid sequence as set forth in SEQ ID NO: 1192 (CDS sequence) and 1193 (plasmid sequence)) containing different sgRNAs was delivered into the cells by transfection, and the two carried red fluorescence and blue fluorescence, respectively. Flow cytometry was used to sort red fluorescent and blue fluorescent double-positive cell populations, which were cultured for a long time. Flow cytometric analysis was performed every week to detect changes in the fluorescence intensity of the reporter cells, which could reflect changes in protein expression levels after epigenetic editing.

### (3) Analysis of experimental results

The results shown in FIGs. 2A-2F demonstrate that 14 days after transfection of the composition described in the present application containing different sgRNAs, the fluorescence intensity of the HBx reporter cell line of type B HBV significantly decreased, indicating that the expression of HBx was significantly inhibited. On day 21 after transfection, the results of each cell line were basically maintained, indicating that the inhibitory effect of epigenetic editing was maintained. This example summarizes the editing abilities of the above sgRNAs in this cell line at different time points. Among them, BSG30, BSG35, BSG43, and BSG50 all had extremely high editing effects and targeted relatively concentrated areas in the genome. Fig. 2G shows the relationship between the target site of the tested sgRNA in the type B HBV genome and the proportion of cells with knocked-down gene expression on the day 28 after transfection. The results show that the sgRNAs with better knockdown effects were mainly concentrated in the areas near both sides of the target sequences of BSG35 and BSG29, indicating that epigenetic editing of this part of the region could achieve better inhibition of HBx expression.

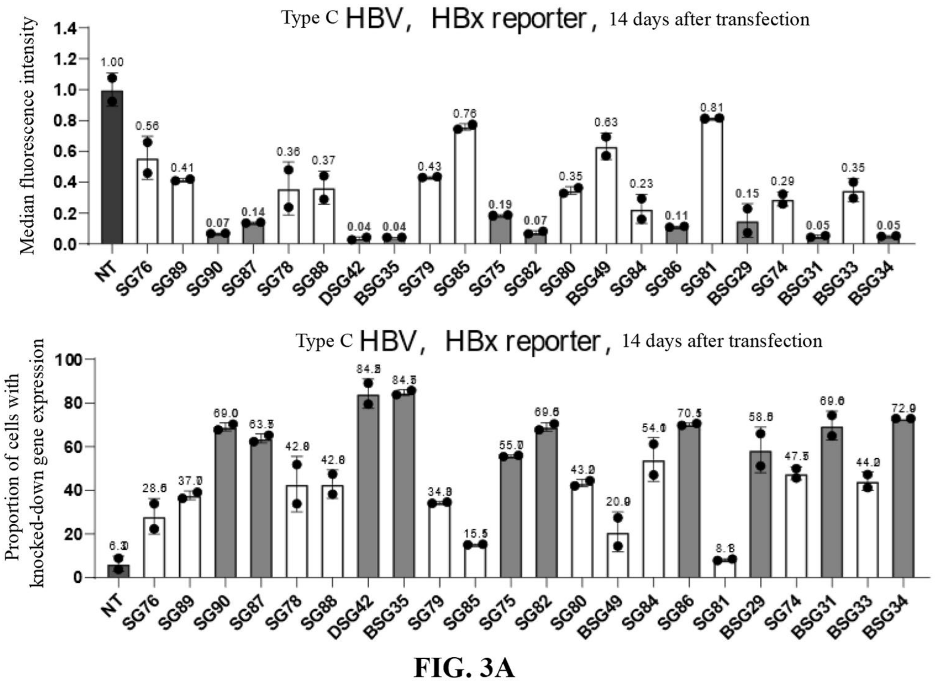

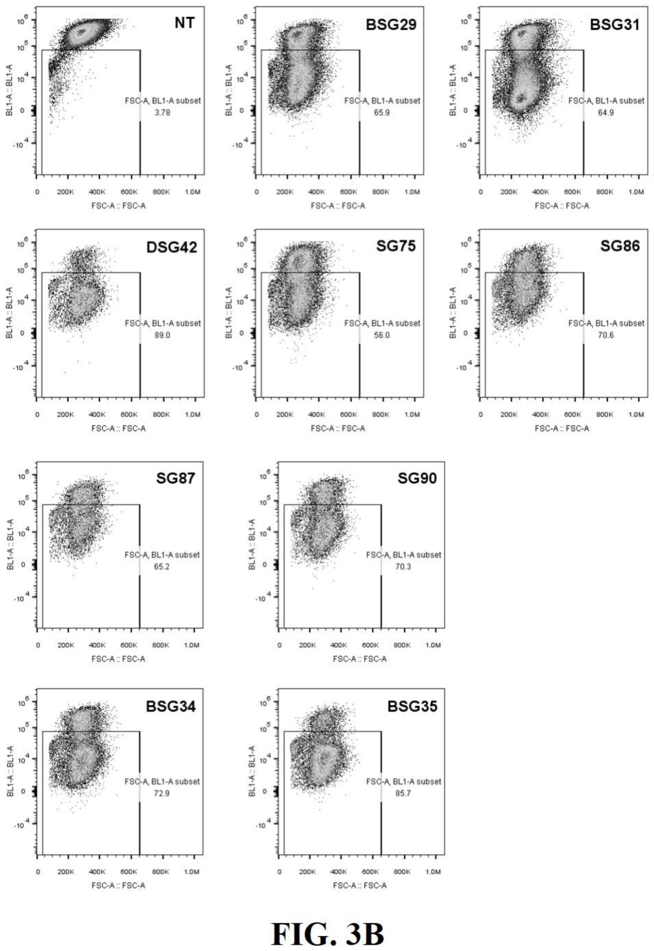

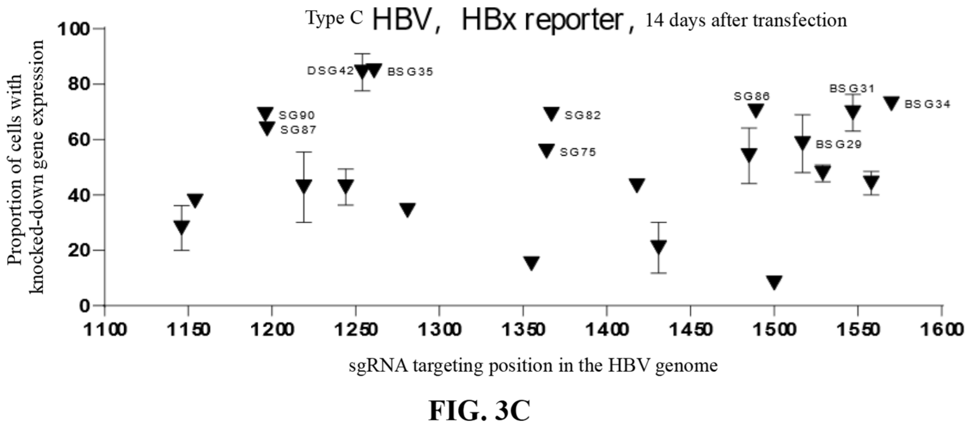

The transfection results of the HBx reporter cell line of type C HBV shown in FIGs. 3A-3B demonstrate that the fluorescence intensity decreased significantly on the day 14 after transfection, indicating that the expression of HBx was significantly inhibited. Fig. 3C shows the relationship between the target site of the tested sgRNA in the type C HBV genome and the proportion of cells with knocked-down gene expression on the day 14 after transfection. The results show that the sgRNAs with better knockdown effects were mainly concentrated in the areas near both sides of the target sequences of BSG35, SG75 and BSG29, indicating that epigenetic editing of this part of the region could achieve better inhibition of HBx expression.

The transfection results of the HBx reporter cell line of type D HBV shown in FIGs. 4A-4F demonstrate that the fluorescence intensity decreased significantly on the day 16 after transfection, indicating that the expression of HBx was significantly inhibited. On day 21 and day 28 after transfection, the results of each cell line were basically maintained, indicating that the inhibitory effect of epigenetic editing was maintained. Fig. 4G shows the relationship between the target site of the tested sgRNA in the type D HBV genome and the proportion of cells with knocked-down gene expression on the day 28 after transfection. The results show that the sgRNAs with better knockdown effects were mainly concentrated in the areas near both sides of the target sequences of BSG35 and BSG29, indicating that epigenetic editing of this part of the region could achieve better inhibition of HBx expression.

Combining the transfection results of the three genotypes of HBx reporter cell lines, the use of the epigenetic editing tool provided in the present application to target the type B, type C and type D HBV genomes could significantly inhibit the expression of HBx, and this inhibitory effect was long-term and could be maintained for at least 28 days. BSG29, BSG31, BSG34, and BSG35, which could simultaneously target three genomes, had nearly the best inhibitory effects on HBx expression of the three HBV genotypes. In the cell lines of the three genotypes, the sgRNAs with the best editing effects were mainly concentrated in the regions on both sides of the target sequences of BSG35 and BSG29, indicating that this part of the region may be the key area for epigenetic regulation of the HBV gene.

The functionally validated sgRNAs in this example are summarized in the following table (arranged by the starting position of the target sequence of each sgRNA):

| **Experiment number** | **SEQ ID NO:** | **Complementary nucleotide sequence of the target sequence** | **Genome** | **Target sequence location in the genome** |
|---|---|---|---|---|
| BSG42 | 345 | CAGGCCGTTGCCGA GCAACG | Type B | 1149-1168 |
| BSG39 | 657 | GCAAACACTTGGCA CAGACC | Type B | 1168-1187 |
| BSG44 | 231 | ATAGGCCATCAGCG CATGCG | Type B | 1219-1238 |
| BSG50 | 642 | GATCGGTAGAGGA GACACAA | Type B | 1247-1266 |
| BSG43 | 954 | TCCTCTACCGATCC ATACTG | Type B | 1254-1273 |
| BSG35 | 182 | AGGAGTTCCGCAGT ATGGAT | Type B | 1264-1283 |
| BSG30 | 451 | CCTGCTGCGAGCAA AACAAG | Type B | 1289-1308 |
| BSG54 | 253 | ATGGAAATGATGTA TACTTG | Type B | 1356-1375 |
| BSG36 | 671 | GCAGCACAGCCTAG CAGCCA | Type B | 1375-1394 |
| BSG28 | 484 | CGCCGACGGACGT AAACAA | Type B | 1421-1440 |
| BSG49 | 408 | CCCGTCGGCGCTGA ATCCCG | Type B | 1431-1450 |
| BSG52 | 693 | GCGGGCGGTAGAG TCCCAAG | Type B | 1473-1492 |
| BSG45 | 72 | ACAATAGGCGGAG AAGCGGG | Type B | 1488-1507 |
| BSG55 | 790 | GGTACAATAGGCG GAGAAGC | Type B | 1491-1510 |
| BSG48 | 1063 | TGGTCGGCCGGTAC AATAGG | Type B | 1500-1519 |
| BSG51 | 441 | CCTATTGTACCGGC CGACCA | Type B | 1500-1519 |
| BSG53 | 922 | TATTGTACCGGCCG ACCACG | Type B | 1502-1521 |
| BSG29 | 810 | GTAAAGAGAGGTG CGCCCCG | Type B | 1520-1539 |
| BSG26 | 498 | CGGGGAGTCCGCGT AAAGAG | Type B | 1532-1551 |
| BSG31 | 335 | CAGATGAGAAGGC ACAGACG | Type B | 1550-1569 |
| BSG34 | 591 | GAAGCGAAGTGCA CACGGTC | Type B | 1573-1592 |
| SG76 | 537 | CTGACCGTTGCCGG GCAACG | Type C | 1150-1169 |
| SG89 | 667 | GCAGAGACCTGACC GTTGCC | Type C | 1158-1177 |
| SG90 | 743 | GGCCAAGCCCCATC CAGTGG | Type C | 1200-1219 |
| SG87 | 1048 | TGGCCAAGCCCCAT CCAGTG | Type C | 1201-1220 |
| SG78 | 232 | ATAGGCCATCGGCG CATGCG | Type C | 1220-1239 |
| SG88 | 640 | GATCGGCAGAGGA GCCACAA | Type C | 1248-1267 |
| DSG42 | 955 | TCCTCTGCCGATCC ATACTG | Type C | 1255-1274 |
| BSG35 | 182 | AGGAGTTCCGCAGT ATGGAT | Type C | 1265-1284 |
| BSG79 | 1034 | TGCGAGCGAAACA AGCTGCT | Type C | 1285-1304 |
| BSG85 | 706 | GGAAATACACCTCC TTTCCA | Type C | 1356-1375 |
| SG75 | 417 | CCCTAGCAGCCATG GAAAGG | Type C | 1368-1387 |
| SG82 | 77 | ACACCCTAGCAGCC ATGGAA | Type C | 1371-1390 |
| SG80 | 485 | CGCCGACGGACGT AGACAA | Type C | 1422-1441 |
| BSG49 | 408 | CCCGTCGGCGCTGA ATCCCG ACGGCAGATGAAG AAGGGA | Type C | 1432-1451 |
| SG84 | 116 | | Type C | 1489-1508 |
| SG86 | 492 | CGGAACGGCAGAT GAAGAAG | Type C | 1493-1512 |
| SG81 | 986 | TCTGCCGTTCCGGC CGACCA | Type C | 1501-1520 |
| BSG29 | 810 | GTAAAGAGAGGTG CGCCCCG | Type C | 1521-1540 |
| SG74 | 497 | CGGGGAGACCGCG TAAAGAG | Type C | 1533-1552 |
| BSG31 | 335 | CAGATGAGAAGGC ACAGACG | Type C | 1551-1570 |
| BSG33 | 81 | ACACGGTCCGGCAG ATGAGA | Type C | 1562-1581 |
| BSG34 | 591 | GAAGCGAAGTGCA CACGGTC | Type C | 1574-1593 |
| DSG43 | 650 | GATTGAATACATGC ATACAA | Type D | 1060-1079 |
| BSG39 | 657 | GCAAACACTTGGCA CAGACC | Type D | 1168-1187 |
| BSG41 | 788 | GGGTTGCGTCAGCA AACACT | Type D | 1179-1198 |
| DSG45 | 601 | GACCAAGCCCCAGC CAGTGG | Type D | 1199-1218 |
| DSG50 | 1006 | TGACCAAGCCCCAG CCAGTG | Type D | 1200-1219 |
| DSG49 | 257 | ATGGGCCATCAGCG CGTGCG | Type D | 1219-1238 |
| DSG42 | 955 | TCCTCTGCCGATCC ATACTG | Type D | 1254-1273 |
| BSG35 | 182 | AGGAGTTCCGCAGT ATGGAT | Type D | 1264-1283 |
| BSG30 | 451 | CCTGCTGCGAGCAA AACAAG | Type D | 1289-1308 |
| BSG36 | 671 | GCAGCACAGCCTAG CAGCCA | Type D | 1375-1394 |
| BSG27 | 698 | GCTGCCAACTGGAT CCTGCG | Type D | 1390-1409 |
| BSG28 | 484 | CGCCGACGGACGT AAACAA | Type D | 1421-1440 |
| DSG53 | 450 | CCTGCGGACGACCC TTCTCG | Type D | 1447-1466 |
| DSG55 | 491 | CGGAACGGCAGAC GGAGAAG | Type D | 1492-1511 |
| DSG52 | 1028 | TGCCGTTCCGACCG ACCACG | Type D | 1502-1521 |
| BSG29 | 810 | GTAAAGAGAGGTG CGCCCCG | Type D | 1520-1539 |
| BSG26 | 498 | CGGGGAGTCCGCGT AAAGAG | Type D | 1532-1551 |
| BSG31 | 335 | CAGATGAGAAGGC ACAGACG | Type D | 1550-1569 |
| BSG38 | 669 | GCAGATGAGAAGG CACAGAC | Type D | 1551-1570 |
| BSG33 | 81 | ACACGGTCCGGCAG ATGAGA | Type D | 1561-1580 |
| BSG34 | 591 | GAAGCGAAGTGCA CACGGTC | Type D | 1573-1592 |

### Example 2

### Knockdown effect of the composition of the present application on HBV markers in primary hepatocytes (PHH)

A primary hepatocyte (PHH) cell line infected with type D hepatitis B virus (GenBank: U95551) was used in this example. According to the results of the HBx reporter cell line screening described in example 1, BSG29, BSG31, BSG34, and BSG35 were located in the conserved region of the genome and exhibited the best inhibitory effect. Therefore, these four sgRNAs were used to formulate the pharmaceutical composition provided in the present application and perform epigenetic editing in primary hepatocytes to validate their inhibitory effect on HBV gene expression.

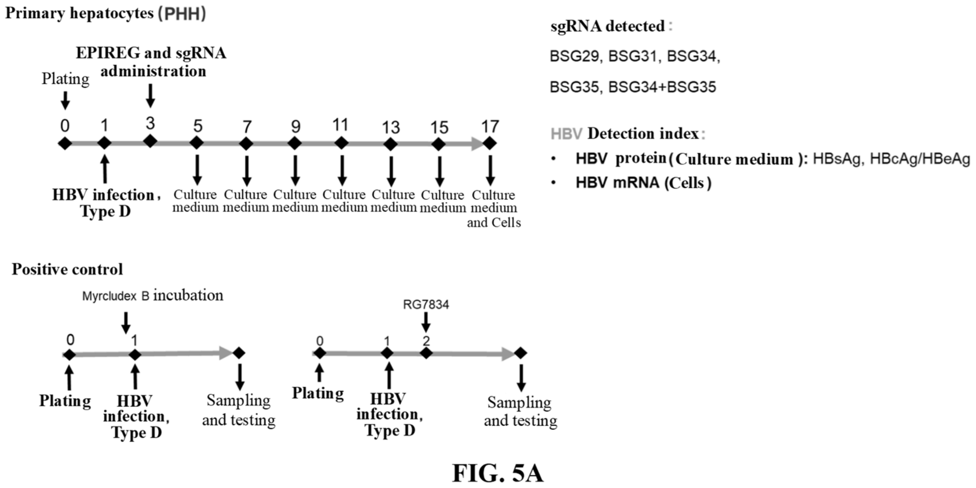

The experimental scheme is shown in FIG. 5A. Two days after primary hepatocytes were infected with HBV, lipid nanoparticles (LNPs) (LNP preparation reference: https://doi.org/10.1038/s41586-021-03534-y) were used to deliver a composition containing mRNA encoding EPIREG (SEQ ID NO: 1191) and each sgRNA (BSG29, BSG31, BSG34, BSG35) into primary hepatocytes (administration dose was 2.5 ug/ml, the mass ratio of EPIREG to sgRNA was 1:1). The culture supernatant was collected every two days after administration to detect the expression levels of HBV surface antigen (HBsAg) and core antigen (HBcAg) secreted by the cells. Cell samples were collected until 14 days after administration, DNA and RNA were extracted from the cells, and the levels of HBV mRNA and changes in the cccDNA of the HBV gene were detected. The two groups of positive controls used Myrcludex B (WuXi AppTec, C9023GE070-1/PE0723) and RG7834 (WuXi AppTec, ET25747-14-P1), respectively. Myrcludex B could inhibit HBV infection of hepatocytes, and RG7834 could inhibit the synthesis of viral proteins. RG7834 was stopped on the day 13 of the experiment to observe the rebound of viral antigen proteins. (Specific detection methods were performed according to the following kit instructions: HBsAg ELISA kit, Antu-CL 0310; HBeAg ELISA kit, Antu-CL 0312; RNeasy 96 Kit (12), QIAGEN # 74182)

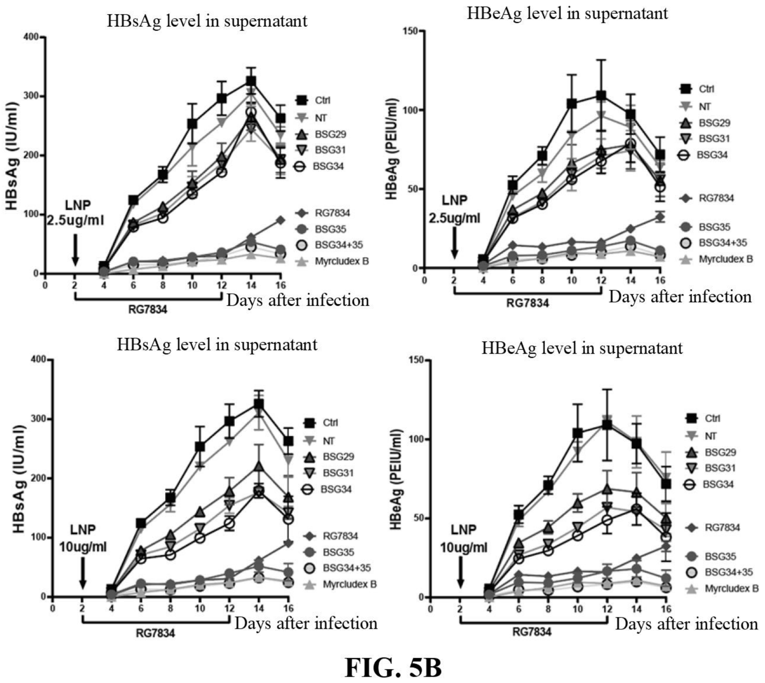

The experimental results 2 days, 4 days, 6 days, 8 days and 10 days after administration are summarized in FIG. 5B. After delivering the composition containing BSG29, BSG31, BSG34, BSG35, and BSG34+BSG35 (the mass ratio of EPIREG to sgRNA in BSG34+BSG35 was 1:0.5:0.5) to HBV-infected primary hepatocytes, the expression levels of HBsAg and HBeAg/HBcAg in the supernatant exhibited different degrees of decrease, among which BSG35 had the best effect, and could simultaneously reduce the expression levels of HBsAg and HBeAg/HBcAg by about 80%. Moreover, the combination of two sgRNAs (BSG34+BSG35) could further enhance the inhibitory effect and further reduce the expression levels of HBsAg and HBeAg/HBcAg. The expression levels of HBsAg and HBeAg/HBcAg in the control group continued to rise with the increase in the number of days of infection, and reached peak values around the day 14 and day 12 after infection, respectively. In the BSG35 experimental group, the expression levels of HBsAg and HBeAg/HBcAg were basically stable on the day 4 after administration. The inhibitory effect of the BSG35 experimental group was close to that of the two reference inhibitors Myrcludex B and RG7834. Moreover, after discontinuation of RG7834, a clear rebound (increasing) trend in the expression of HBsAg and HBeAg/HBcAg was observed. In the BSG35 experimental group, no subsequent administration was performed 4 h after the drug treatment on the day of administration, and the level of viral antigen protein remained stable at a low level throughout the experimental period. The results of this section demonstrate that epigenetic editing could significantly reduce the expression of viral proteins in HBV-infected PHHs, and epigenetic editing using a single sgRNA could simultaneously regulate the expression of multiple viral proteins.

### Example 3

### Construction and functional screening of sgRNA lentiviral library

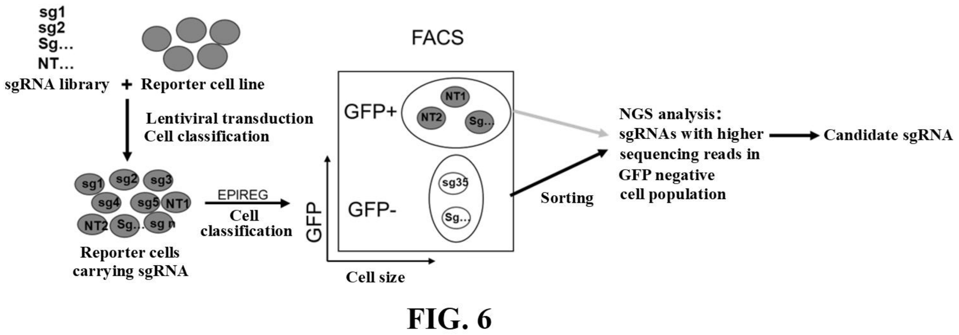

In this example, the type D HBV X protein (HBx), core antigen (HBcAg), and surface antigen (HBsAg) reporter cell lines were used for library screening. First, a sgRNA cell library of type D HBV gene was constructed using a reporter cell line according to the method described in example 1. The sgRNA library was packaged into lentivirus to obtain a sgRNA lentiviral library (carrying red fluorescence), the reporter cell line was infected with the lentiviral library, and the red fluorescence was sorted to obtain the sgRNA cell library. Each cell in this sorted cell library integrated a single sgRNA in the library while the cell itself carried a reporter sequence (carrying green fluorescence). The EPIREG tool described in example 2 (the amino acid sequence of the tool was SEQ ID NO: 1194) (carrying blue fluorescence) was transfected and delivered into a cell library carrying both sgRNA and reporter sequences. Red/green/blue fluorescent triple-positive cells were sorted using a flow cytometer and cultured long-term. The EPIREG transfected into the cells could work together with the sgRNA to silence GFP in the reporter. On the day 14 after transfection, the GFP-negative cell population in the cell library was sorted, the genomic DNA of the negative cell population was extracted for NGS sequencing. The sgRNAs with higher sequencing reads in the negative cell population were compared using the untreated cell library as a control reference, so as to obtain the candidate sgRNAs that could play an editing role corresponding to each reporter, respectively (the schematic diagram of this screening scheme is shown in FIG. 6, and the analysis method is referenced to the literature DOI: 10.1186/s13059-014-0554-4). The functionally validated sgRNAs for each reporter cell line are summarized in the following table (arranged by the starting position of the target sequence of each sgRNA).

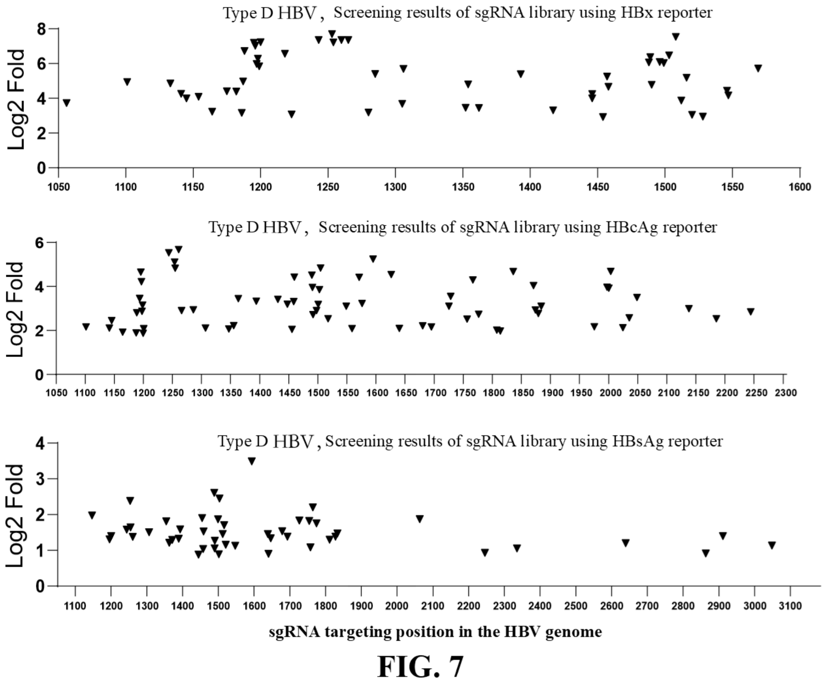

The information of sgRNA obtained by HBx reporter screening (as shown in the upper panel of FIG. 7) is summarized as follows:

| **SEQ ID NO:** | **Complementary nucleotide sequence of the target sequence** | **Target sequence location in the genome** | **Log₂ FC** |
|---|---|---|---|
| 650 | GATTGAATACATGCATACAA | 1056-1075 | 3.726 |
| 305 | CACAGAAAGGCCTTGTAAGT | 1101-1120 | 4.9316 |
| 762 | GGGCAACGGGGTAAAGGTTC | 1133-1152 | 4.8522 |
| 1132 | TTTACCCCGTTGCCCGGCAA | 1141-1160 | 4.252 |
| 553 | CTGGCCGTTGCCGGGCAACG | 1145-1164 | 3.9905 |
| 735 | GGCACAGACCTGGCCGTTGC | 1154-1173 | 4.08 |
| 657 | GCAAACACTTGGCACAGACC | 1164-1183 | 3.2226 |
| 788 | GGGTTGCGTCAGCAAACACT | 1175-1194 | 4.4031 |
| 1146 | TTTGCTGACGCAACCCCCAC | 1182-1201 | 4.3908 |
| 539 | CTGACGCAACCCCCACTGGC TGACGCAACCCCCACTGGCT | 1186-1205 | 3.1536 |
| 1008 | | 1187-1206 | 4.9717 |
| 612 | GACGCAACCCCCACTGGCTG GACCAAGCCCCAGCCAGTGG | 1188-1207 | 6.7125 |
| 601 | | 1195-1214 | 7.1876 |
| 1006 | TGACCAAGCCCCAGCCAGTG | 1196-1215 | 7.0191 |
| 249 | ATGACCAAGCCCCAGCCAGT | 1197-1216 | 5.9752 |
| 357 | CATGACCAAGCCCCAGCCAG | 1198-1217 | 6.2753 |
| 327 | CACTGGCTGGGGCTTGGTCA | 1199-1218 | 5.8386 |
| 127 | ACTGGCTGGGGCTTGGTCAT | 1200-1219 | 7.231 |
| 257 | ATGGGCCATCAGCGCGTGCG | 1218-1237 | 6.5564 |
| 204 | AGGTTCCACGCACGCGCTGA | 1223-1242 | 3.0712 |
| 641 | GATCGGCAGAGGAGCCGAAA | 1243-1262 | 7.351 |
| 955 | TCCTCTGCCGATCCATACTG | 1253-1272 | 7.6855 |
| 944 | TCCGCAGTATGGATCGGCAG | 1254-1273 | 7.2163 |
| 182 | AGGAGTTCCGCAGTATGGAT | 1260-1279 | 7.3504 |
| 496 | CGGCTAGGAGTTCCGCAGTA | 1265-1284 | 7.3529 |
| 1033 | TGCGAGCAAAACAAGCGGCT | 1280-1299 | 3.1795 |
| 451 | CCTGCTGCGAGCAAAACAAG | 1285-1304 | 5.3958 |
| 197 | AGGTCTGGAGCAAACATTAT | 1305-1324 | 3.6817 |
| 792 | GGTCTGGAGCAAACATTATC | 1306-1325 | 5.6925 |
| 254 | ATGGATACGATGTATATTTG | 1352-1371 | 3.4564 |
| 658 | GCAAATATACATCGTATCCA | 1354-1373 | 4.7952 |
| 88 | ACATCGTATCCATGGCTGCT | 1362-1381 | 3.4448 |
| 506 | CGTCCCGCGCAGGATCCAGT | 1393-1412 | 5.3827 |
| 484 | CGCCGACGGGACGTAAACAA | 1417-1436 | 3.3079 |
| 450 | CCTGCGGACGACCCTTCTCG | 1447-1466 | 4.242 |
| 958 | TCCTGCGGACGACCCTTCTC | 1446-1465 | 3.9999 |
| 467 | CGACCCTTCTCGGGGTCGCT | 1454-1473 | 2.9245 |
| 825 | GTCCCAAGCGACCCCGAGAA | 1457-1476 | 5.2457 |
| 211 | AGTCCCAAGCGACCCCGAGA | 1458-1477 | 4.6655 |
| 491 | CGGAACGGCAGACGGAGAAG | 1488-1507 | 6.0674 |
| 973 | TCGGAACGGCAGACGGAGAA | 1489-1508 | 6.3619 |
| 834 | GTCGGAACGGCAGACGGAGA | 1490-1509 | 4.7704 |
| 1065 | TGGTCGGTCGGAACGGCAGA | 1496-1515 | 6.0793 |
| 985 | TCTGCCGTTCCGACCGACCA | 1499-1518 | 6.0276 |
| 483 | CGCCCCGTGGTCGGTCGGAA | 1503-1522 | 6.4685 |
| 200 | AGGTGCGCCCCGTGGTCGGT | 1508-1527 | 7.535 |
| 150 | AGAGAGGTGCGCCCCGTGGT | 1512-1531 | 3.8671 |
| 810 | GTAAAGAGAGGTGCGCCCCG GGGGCGCACCTCTCTTTACG | 1516-1535 | 5.1825 |
| 774 | | 1520-1539 | 3.0494 |
| 498 | CGGGGAGTCCGCGTAAAGAG | 1528-1547 | 2.939 |
| 335 | CAGATGAGAAGGCACAGACG | 1546-1565 | 4.44 |
| 669 | GCAGATGAGAAGGCACAGAC | 1547-1566 | 4.1676 |
| 591 | GAAGCGAAGTGCACACGGTC | 1569-1588 | 5.7107 |

| | | | |
|---|---|---|---|
| Note: Log ₂ FC in FC refers to fold change, which is a value obtained by taking the logarithm of base 2 for the ratio of expression levels between two samples (groups), and is used to express the differential expression level between the two samples (groups). For example, the default screening criterion for differentially expressed genes is usually a Log₂ FC absolute value greater than 1. | | | |

The information of sgRNA obtained by HBcAg reporter screening (as shown in the middle panel of FIG. 7) is summarized as follows:

| **SEQ ID NO:** | **Complementary nucleotide sequence of the target sequence** | **Target sequence location in the genome** | **Log₂ FC** |
|---|---|---|---|
| 305 | CACAGAAAGGCCTTGTAAGT | 1101-1120 | 2.1583 |
| 1132 | TTTACCCCGTTGCCCGGCAA | 1141-1160 | 2.1043 |
| 553 | CTGGCCGTTGCCGGGCAACG | 1145-1164 | 2.4462 |
| 657 | GCAAACACTTGGCACAGACC | 1164-1183 | 1.9192 |
| 1008 | TGACGCAACCCCCACTGGCT | 1187-1206 | 1.8835 |
| 612 | GACGCAACCCCCACTGGCTG | 1188-1207 | 2.8035 |
| 39 | AACCCCCACTGGCTGGGGCT | 1193-1212 | 3.4538 |
| 601 | GACCAAGCCCCAGCCAGTGG | 1195-1214 | 4.6508 |
| 1006 | TGACCAAGCCCCAGCCAGTG | 1196-1215 | 4.2194 |
| 249 | ATGACCAAGCCCCAGCCAGT | 1197-1216 | 2.8648 |
| 357 | CATGACCAAGCCCCAGCCAG | 1198-1217 | 3.1523 |
| 327 | CACTGGCTGGGGCTTGGTCA | 1199-1218 | 1.8664 |
| 127 | ACTGGCTGGGGCTTGGTCAT | 1200-1219 | 2.0792 |
| 641 | GATCGGCAGAGGAGCCGAAA | 1243-1262 | 5.5293 |
| 955 | TCCTCTGCCGATCCATACTG | 1253-1272 | 5.1075 |
| 944 | TCCGCAGTATGGATCGGCAG | 1254-1273 | 4.8272 |
| 182 | AGGAGTTCCGCAGTATGGAT | 1260-1279 | 5.6721 |
| 496 | CGGCTAGGAGTTCCGCAGTA | 1265-1284 | 2.9031 |
| 451 | CCTGCTGCGAGCAAAACAAG | 1285-1304 | 2.9406 |
| 792 | GGTCTGGAGCAAACATTATC | 1306-1325 | 2.103 |
| 113 | ACGATGTATATTTGCGGGAG | 1346-1365 | 2.0632 |
| 658 | GCAAATATACATCGTATCCA | 1354-1373 | 2.2171 |
| 88 | ACATCGTATCCATGGCTGCT | 1362-1381 | 3.4446 |
| 506 | CGTCCCGCGCAGGATCCAGT | 1393-1412 | 3.3238 |
| 427 | CCGCAGGATTCAGCGCCGAC | 1430-1449 | 3.4136 |
| 958 | TCCTGCGGACGACCCTTCTC | 1446-1465 | 3.1878 |
| 467 | CGACCCTTCTCGGGGTCGCT GTCCCAAGCGACCCCGAGAA | 1454-1473 | 2.0481 |
| 825 | | 1457-1476 | 3.3064 |
| 211 | AGTCCCAAGCGACCCCGAGA | 1458-1477 | 4.4175 |
| 491 | CGGAACGGCAGACGGAGAAG | 1488-1507 | 4.5168 |
| 973 | TCGGAACGGCAGACGGAGAA | 1489-1508 | 3.9552 |
| 834 | GTCGGAACGGCAGACGGAGA | 1490-1509 | 2.7177 |
| 1065 | TGGTCGGTCGGAACGGCAGA | 1496-1515 | 2.8985 |
| 985 | TCTGCCGTTCCGACCGACCA | 1499-1518 | 3.1824 |
| 1028 | TGCCGTTCCGACCGACCACG | 1501-1520 | 3.8548 |
| 483 | CGCCCCGTGGTCGGTCGGAA | 1503-1522 | 4.8386 |
| 810 | GTAAAGAGAGGTGCGCCCCG GCAGATGAGAAGGCACAGAC | 1516-1535 | 2.5258 |
| 669 | | 1547-1566 | 3.0945 |
| 81 | ACACGGTCCGGCAGATGAGA | 1557-1576 | 2.0776 |
| 591 | GAAGCGAAGTGCACACGGTC | 1569-1588 | 4.4134 |
| 628 | GAGGTGAAGCGAAGTGCACA | 1574-1593 | 3.2225 |
| 791 | GGTCTCCATGCGACGTGCAG | 1593-1612 | 5.2397 |
| 481 | CGCCCACCGAATGTTGCCCA | 1624-1643 | 4.5414 |
| 1025 | TGCCCAAGGTCTTACATAAG | 1638-1657 | 2.0852 |
| 62 | AATGTCAACGACCGACCTTG | 1678-1697 | 2.2035 |
| 215 | AGTCTTTGAAGTATGCCTCA | 1693-1712 | 2.1589 |
| 872 | TAAAGACTGGGAGGAGTTGG | 1723-1742 | 3.0933 |
| 145 | AGACTGGGAGGAGTTGGGGG | 1726-1745 | 3.5473 |
| 161 | AGATTAAAGGTCTTTGTACT | 1754-1773 | 2.5156 |
| 998 | TCTTTGTACTAGGAGGCTGT | 1764-1783 | 4.2906 |
| 180 | AGGAGGCTGTAGGCATAAAT | 1774-1793 | 2.7289 |
| 841 | GTGAAAAAGTTGCATGGTGC | 1805-1824 | 2.0112 |
| 668 | GCAGAGGTGAAAAAGTTGCA | 1811-1830 | 1.9744 |
| 598 | GACATGAACAAGAGATGATT | 1833-1852 | 4.6761 |
| 372 | CCACCCAAGGCACAGCTTGG | 1868-1887 | 4.0382 |
| 50 | AAGCCACCCAAGGCACAGCT | 1871-1890 | 2.927 |
| 560 | CTGTGCCTTGGGTGGCTTTG | 1876-1895 | 2.7695 |
| 379 | CCATGCCCCAAAGCCACCCA | 1881-1900 | 3.0989 |
| 981 | TCTAGAAGATCTCGTACTGA | 1972-1991 | 2.1673 |
| 225 | ATACCGCCTCAGCTCTGTAT | 1994-2013 | 3.966 |
| 1104 | TTCCCGATACAGAGCTGAGG | 1997-2016 | 3.9205 |
| 750 | GGCTTCCCGATACAGAGCTG | 2000-2019 | 4.6799 |
| 74 | ACAATGCTCAGGAGACTCTA | 2021-2040 | 2.1202 |
| 1068 | TGGTGAGGTGAACAATGCTC | 2032-2051 | 2.5736 |
| 311 | CACCTCACCATACTGCACTC | 2045-2064 | 3.495 |
| 120 | ACTACTAGGTCTCTAGATGC | 2134-2153 | 2.9841 |
| 70 | ACAAGAGTTGCCTGAACTTT | 2181-2200 | 2.5329 |
| 139 | AGACACCAAATACTCTATAA | 2240-2259 | 2.8391 |

The information of sgRNA obtained by HBsAg reporter screening (as shown in the lower panel of FIG. 7) is summarized as follows:

| **SEQ ID NO:** | **Complementary nucleotide sequence of the target sequence** | **Target sequence location in the genome** | **Log₂ FC** |
|---|---|---|---|
| 410 | CCCGTTGCCCGGCAACGGCC | 1146-1165 | 1.9735 |
| 601 | GACCAAGCCCCAGCCAGTGG | 1195-1214 | 1.3073 |
| 127 | ACTGGCTGGGGCTTGGTCAT | 1200-1219 | 1.3983 |
| 641 | GATCGGCAGAGGAGCCGAAA | 1243-1262 | 1.58 |
| 955 | TCCTCTGCCGATCCATACTG | 1253-1272 | 2.3817 |
| 944 | TCCGCAGTATGGATCGGCAG | 1254-1273 | 1.6439 |
| 182 | AGGAGTTCCGCAGTATGGAT | 1260-1279 | 1.3776 |
| 792 | GGTCTGGAGCAAACATTATC | 1306-1325 | 1.5074 |
| 658 | GCAAATATACATCGTATCCA | 1354-1373 | 1.8084 |
| 88 | ACATCGTATCCATGGCTGCT | 1362-1381 | 1.2095 |
| 671 | GCAGCACAGCCTAGCAGCCA | 1371-1390 | 1.2918 |
| 698 | GCTGCCAACTGGATCCTGCG | 1389-1408 | 1.3233 |
| 506 | CGTCCCGCGCAGGATCCAGT | 1393-1412 | 1.5863 |
| 242 | ATCCTGCGGACGACCCTTCT | 1444-1463 | 0.88088 |
| 467 | CGACCCTTCTCGGGGTCGCT | 1454-1473 | 1.8988 |
| 825 | GTCCCAAGCGACCCCGAGAA | 1457-1476 | 1.0352 |
| 211 | AGTCCCAAGCGACCCCGAGA | 1458-1477 | 1.5263 |
| 491 | CGGAACGGCAGACGGAGAAG | 1488-1507 | 2.6046 |
| 973 | TCGGAACGGCAGACGGAGAA | 1489-1508 | 1.056 |
| 834 | GTCGGAACGGCAGACGGAGA | 1490-1509 | 1.2666 |
| 985 | TCTGCCGTTCCGACCGACCA | 1499-1518 | 1.8695 |
| 1028 | TGCCGTTCCGACCGACCACG | 1501-1520 | 0.89219 |
| 483 | CGCCCCGTGGTCGGTCGGAA | 1503-1522 | 2.4519 |
| 150 | AGAGAGGTGCGCCCCGTGGT | 1512-1531 | 1.453 |
| 810 | GTAAAGAGAGGTGCGCCCCG | 1516-1535 | 1.7059 |
| 774 | GGGGCGCACCTCTCTTTACG | 1520-1539 | 1.1527 |
| 669 | GCAGATGAGAAGGCACAGAC | 1547-1566 | 1.1259 |
| 791 | GGTCTCCATGCGACGTGCAG | 1593-1612 | 3.4863 |
| 1025 | TGCCCAAGGTCTTACATAAG GTCCTCTTATGTAAGACCTT | 1638-1657 | 1.4524 |
| 829 | | 1640-1659 | 0.90289 |
| 839 | GTCTTACATAAGAGGACTCT AATGTCAACGACCGACCTTG | 1646-1665 | 1.3376 |
| 62 | | 1678-1697 | 1.5364 |
| 215 | AGTCTTTGAAGTATGCCTCA | 1693-1712 | 1.3799 |
| 145 | AGACTGGGAGGAGTTGGGGG | 1726-1745 | 1.8314 |
| 161 | AGATTAAAGGTCTTTGTACT | 1754-1773 | 1.8238 |
| 1084 | TTAAAGGTCTTTGTACTAGG | 1757-1776 | 1.0831 |
| 998 | TCTTTGTACTAGGAGGCTGT | 1764-1783 | 2.1989 |
| 180 | AGGAGGCTGTAGGCATAAAT | 1774-1793 | 1.7547 |
| 668 | GCAGAGGTGAAAAAGTTGCA | 1811-1830 | 1.297 |
| 27 | AACAAGAGATGATTAGGCAG | 1827-1846 | 1.3821 |
| 598 | GACATGAACAAGAGATGATT | 1833-1852 | 1.4722 |
| 930 | TCAGGCAAGCAATTCTTTGC | 2063-2082 | 1.8744 |
| 227 | ATAGAGTATTTGGTGTCTTT | 2245-2264 | 0.92755 |
| 121 | ACTACTGTTGTTAGACGACG | 2335-2354 | 1.0553 |
| 112 | ACCTTTGGATAAAACCTAGC | 2639-2658 | 1.203 |
| 593 | GAATCCCAGAGGATTGCTGG | 2863-2882 | 0.91237 |
| 270 | ATTTGCTGTGTTTGCTCTGA | 2911-2930 | 1.397 |
| 1072 | TGTAGTATGCCCTGAGCCTG | 3048-3067 | 1.1325 |

### Example 4

### Candidate sgRNA effect validation experiment for library screening

### Effect validation in primary hepatocytes (PHH)

A primary hepatocyte (PHH) cell line (WuXi AppTec, M00995-P) infected with type D hepatitis B virus (GenBank: U95551) was used in this validation experiment. Based on the screening results of the three libraries in Example 3 and combined with factors such as conservation, the 20 sgRNAs shown in the table below were selected to prepare the pharmaceutical composition provided in the present application and perform epigenetic editing in the primary hepatocytes to validate the inhibitory effect of the screened candidate sgRNAs on HBV gene expression.

The 20 sgRNA sequences used for validation in this example are as follows:

| **Experiment number** | **SEQ ID NO:** | **Complementary nucleotide sequence of the target sequence** | **Target sequence location in the genome** |
|---|---|---|---|
| D113(DSG4 5) | 601 | GACCAAGCCCCAGCCAGTGG | 1195-1214 |
| D111 | 249 | ATGACCAAGCCCCAGCCAGT | 1197-1216 |
| D108 | 127 | ACTGGCTGGGGCTTGGTCAT | 1200-1219 |
| D101(DSG4 2) | 955 | TCCTCTGCCGATCCATACTG | 1253-1272 |
| D100 | 944 | TCCGCAGTATGGATCGGCAG | 1254-1273 |
| D99(BSG35) | 182 | AGGAGTTCCGCAGTATGGAT | 1260-1279 |
| D97 | 496 | CGGCTAGGAGTTCCGCAGTA | 1265-1284 |
| D63 | 506 | CGTCCCGCGCAGGATCCAGT | 1393-1412 |
| D14(BSG38) | 669 | GCAGATGAGAAGGCACAGAC | 1547-1566 |
| D1 | 791 | GGTCTCCATGCGACGTGCAG | 1593-1612 |
| D16 | 1025 | TGCCCAAGGTCTTACATAAG | 1638-1657 |
| D33 | 215 | AGTCTTTGAAGTATGCCTCA | 1693-1712 |
| D45 | 145 | AGACTGGGAGGAGTTGGGGG | 1726-1745 |
| D58 | 998 | TCTTTGTACTAGGAGGCTGT | 1764-1783 |
| D61 | 180 | AGGAGGCTGTAGGCATAAAT | 1774-1793 |
| D69 | 668 | GCAGAGGTGAAAAAGTTGCA | 1811-1830 |
| D75 | 598 | GACATGAACAAGAGATGATT | 1833-1852 |
| D199 | 121 | ACTACTGTTGTTAGACGACG | 2335-2354 |
| D340 | 593 | GAATCCCAGAGGATTGCTGG | 2863-2882 |
| D409 | 1072 | TGTAGTATGCCCTGAGCCTG | 3048-3067 |

The experimental scheme is shown in FIG. 5A. Two days after primary hepatocytes were infected with HBV, lipid nanoparticles (LNPs) (LNP preparation reference: https://doi.org/10.1038/s41586-021-03534-y) were used to deliver the composition comprising the mRNA encoding EPIREG (sequence: SEQ ID NO: 1191) and each of the above-mentioned sgRNAs into primary hepatocytes (administration dose was 2.5 ug/ml, the mass ratio of EPIREG to sgRNA was 1:1). Myrcludex B (provided by WuXi AppTec, C9023GE070-1/PE0723) was used as a positive control. Myrcludex B could inhibit HBV infection of hepatocytes. The supernatant of the culture medium was collected every two days after administration in each group, and the expression levels of HBV surface antigen (HBsAg), core antigen (HBcAg/HBeAg) and HBV DNA secreted by the cells were detected until 14 days after infection. Cell samples were collected, DNA and RNA in the cells were extracted, and the level of HBV mRNA was detected. (Specific detection methods were performed according to the following kit instructions: HBsAg ELISA kit, Antu-CL 0310; HBeAg ELISA kit, Antu-CL 0312; RNeasy 96 Kit (12), QIAGEN # 74182)

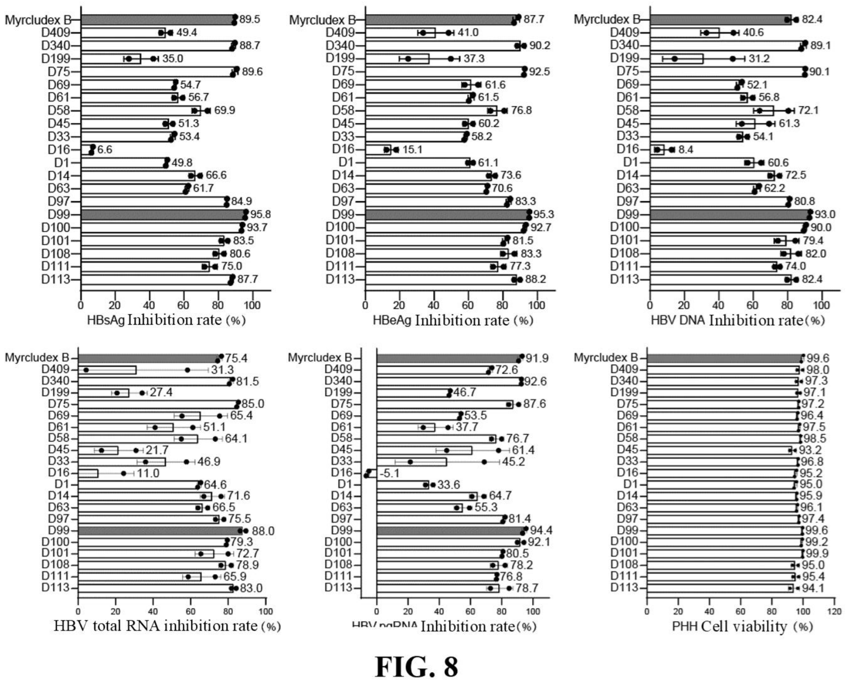

The experimental results 14 days after infection are summarized in FIG. 8. After delivering the composition of 20 sgRNA and EPIREG to HBV-infected primary hepatocytes, the expression levels of HBsAg, HBeAg/HBcAg and HBV DNA in the supernatant exhibited different degrees of reduction. Among them, 17 sgRNAs could reduce the expression of HBV virus antigen and DNA by more than 50% (except D409, D199, D16), and the inhibition rate of 9 sgRNAs could reach nearly 80% or above (D340, D75, D97, D99, D100, D101, D108, D111, and D113), among which D99 (i.e., BSG35) showed the highest inhibition level; EPIREG exhibited the same efficiency and trend in the inhibition on HBV total RNA and pgRNA as HBV antigens and DNA, indicating that this epigenetic editing tool effectively inhibited HBV transcription; at the same time, the composition of 20 sgRNAs and EPIREG did not affect the viability of PHH cells, indicating the safety of this epigenetic editing tool. In summary, through validation in PHH cell lines, most of the candidate sgRNAs obtained from library screening exhibited significant inhibitory effects on HBV.

### Knockdown effect of the composition on HBV markers in transgenic HBV mice

Transgenic HBV mice (Beijing Vitalstar Biotechnology Co.,Ltd., C57BL/6-HBV, subsequent feeding and testing were completed by Beijing Vitalstar) were used in this validation experiment. The genome of this strain of mice was inserted with a 1.28-fold length HBV genome (type A, GenBank: AF305422.1). Based on all the screening results, the sgRNA numbered D99 (BSG35) was located in the conserved region of the genome and exhibited the best inhibitory effect. Therefore, this sgRNA was used to prepare the pharmaceutical composition provided in the present application and perform epigenetic editing in transgenic HBV mice to validate the inhibitory effect of this drug on the expression of integrated HBV genes in an in vivo model.

The composition comprising the mRNA encoding EPIREG (sequence: SEQ ID NO: 1191) and sgRNA numbered D99 (BSG35) (mass ratio of mRNA to sgRNA of 1: 1) was delivered to transgenic HBV mice (at a dose of 10 mg/kg) by means of tail vein injection of lipid nanoparticles (LNPs) (LNP preparation reference: https://doi.org/10.1038/s41586-021-03534-y). PBS (200 ul) was injected as a negative control, and the antisense oligonucleotide (ASO) drug Bepirovirsen (GSK3228836, dose of 200 mg/kg) was used as a positive control, which could target all HBV RNAs and thus reduce the expression of HBV antigens. After administration, blood was collected regularly in each group to detect the levels of HBV surface antigen (HBsAg) and core antigen (HBcAg/HBeAg) secreted in serum, and the level of HBV DNA was detected by qPCR. (Hepatitis B virus e antigen quantification kit: Maccura, article No. IM4403003; Hepatitis B virus surface antigen quantification kit: Maccura, article No. IM4403001; Hepatitis B virus nucleic acid quantification kit: Sansure, 2015340008; the detection method was operated according to the kit instructions).

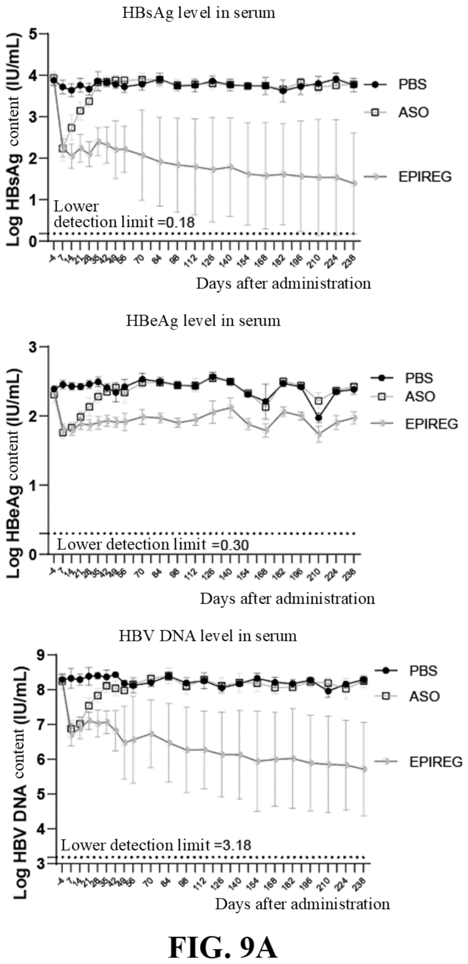

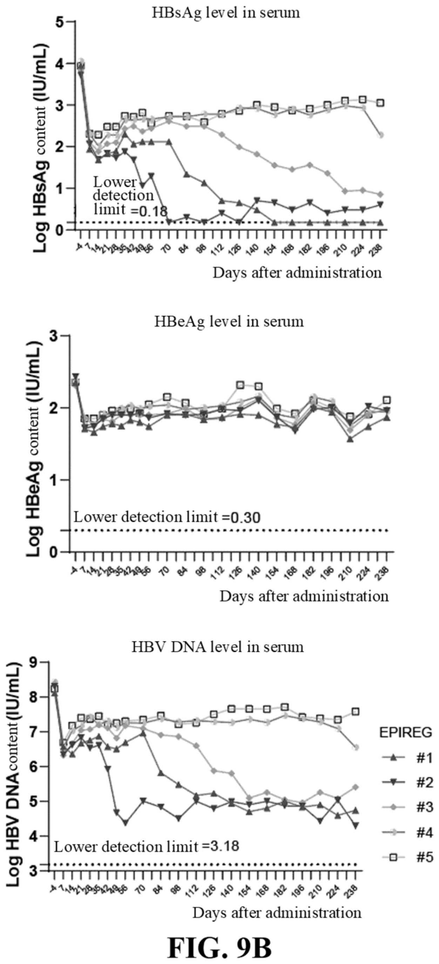

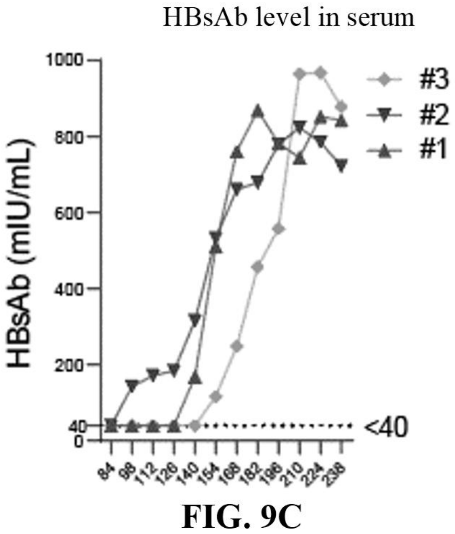

The experimental results after administration are summarized in FIGs 9A-9C. After a single injection of EPIREG, the expression levels of HBsAg, HBeAg, and HBV DNA in the serum of transgenic HBV mice reduced rapidly within one week, with the reduction rate being close to or better than that of the ASO group. However, HBV markers in the ASO group gradually returned to their original levels, while HBV markers in the EPIREG group were stably and persistently inhibited, with HBsAg and HBV DNA continuing to reduce approximately 2 months after drug administration (FIG. 9A). Analysis of the HBV marker expression curves of each mouse in the EPIREG group (FIG. 9B) showed that after a single injection of EPIREG, HBsAg and HBV DNA in the three mice spontaneously reduced further in about 2 months, with HBsAg reduced to the detection limit. In addition, the gradual expression of HBsAb was detectable in the serum of these three mice (FIG. 9C). This result demonstrates that in a transgenic HBV mouse model, the epigenetic editing composition comprising mRNA encoding EPIREG and the sgRNA provided in the present application could significantly and persistently silence the expression of the integrated HBV genome in vivo, and due to the long-term silencing of HBV, the mice could gradually restore their immune response to the surface antigen HBsAg, thereby further reducing the expression of HBV markers.

### Knockdown effect of the composition on HBV markers in AAV-HBV mice

This validation experiment used an AAV-HBV mouse model (modeling, subsequent feeding and detection were completed by Beijing Vitalstar Biotechnology Co.,Ltd.). Mice were infected with adenovirus-associated viruses (AAV) carrying a 1.3-fold length HBV genome (type D, GenBank: U95551) to simulate the expression of episomal HBV genome in vivo. Based on all the screening results, the sgRNA numbered D99 (BSG35) was located in the conserved region of the genome and exhibited the best inhibitory effect. Therefore, this sgRNA was used to prepare the pharmaceutical composition provided in the present application and perform epigenetic editing in AAV-HBV mice to validate the inhibitory effect of this drug on the expression of episomal HBV genes in an in vivo model.

Two titers (1E10 and 1E11) of AAV (AMV-002, Beijing FivePlus Gene Technology Co., Ltd.) carrying a 1.3-fold length HBV genome were delivered to mice via tail vein injection, achieving varying degrees of stable expression of the HBV genome in vivo. Subsequently, on the day of the first dose (D0) and day 15 (D15), a composition comprising mRNA encoding EPIREG (sequence: SEQ ID NO: 1191) and sgRNA numbered D99 (BSG35) (mass ratio of mRNA to sgRNA of 1:1) was delivered to AAV-HBV mice via tail vein injection of lipid nanoparticles (LNPs) (LNP preparation reference: https://doi.org/10.1038/s41586-021-03534-y) (both injections were at a dose of 5 mg/kg). PBS (200 ul) was injected as a negative control. After administration, blood was collected regularly in each group to detect the levels of HBV surface antigen (HBsAg) and core antigen (HBcAg/HBeAg) secreted in serum, and the level of HBV DNA was detected by qPCR (all detection kits were as described in the previous part of this example).

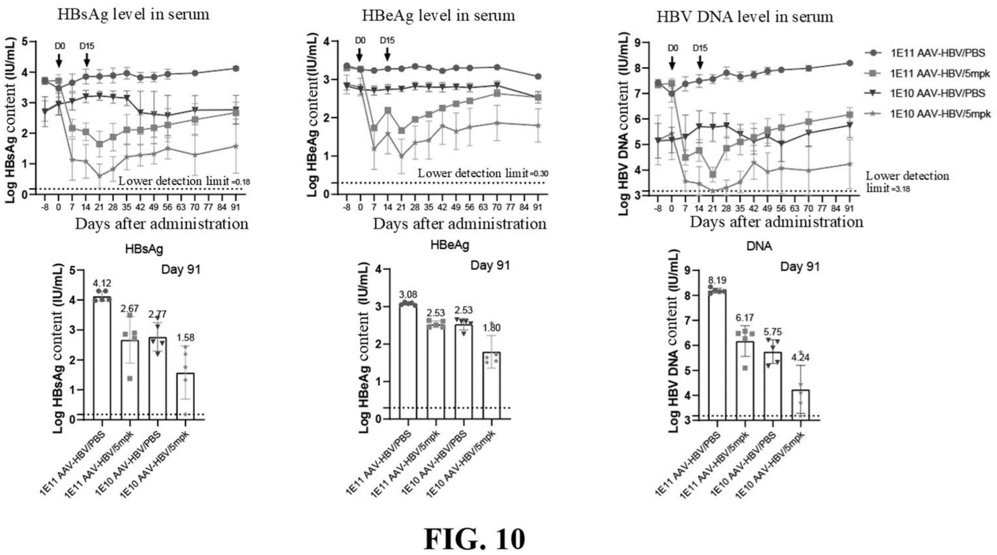

The experimental results after administration are summarized in FIG. 10. After AAV-HBV mice received two injections of EPIREG, the expression levels of HBsAg, HBeAg, and HBV DNA in the serum reduced rapidly. The 1E10 AAV group had low viral expression levels, and viral markers could be reduced to near the detection limit in a short period of time after administration. The 1E11 AAV group had high viral expression levels, and a more significant reduction in viral markers could be achieved after administration. Ultimately, this epigenetic editing tool could stably and persistently reduce HBV markers in both the 1E10 and 1E11 AAV groups. This result demonstrates that this epiediting could significantly and persistently silence expression of the episomal HBV genome in vivo in an AAV-HBV mouse model.

## Claims

1. A composition comprising:
(1) a fusion molecule or a nucleic acid sequence encoding the fusion molecule, wherein the fusion molecule comprises at least one DNA binding protein and at least one gene expression modulator, and
(2) at least one single guide RNA (sgRNA), or a nucleic acid sequence encoding the sgRNA,
wherein the sgRNA is complementary to a target DNA sequence in the vicinity of a hepatitis B virus (HBV) gene and/or within a HBV gene regulatory element, the HBV gene is a type B HBV gene comprising a nucleotide sequence as set forth in SEQ ID NO: 1, a type C HBV gene comprising a nucleotide sequence as set forth in SEQ ID NO: 2, or a type D HBV gene comprising a nucleotide sequence as set forth in SEQ ID NO: 3, the HBV gene regulatory element comprises a transcription initiation site, a core promoter, a promoter, an enhancer, a silencer, an insulator element, a boundary element, and/or a locus control region, and the target DNA sequence is located between nucleotide at position 1056 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

2. The composition according to claim 1, wherein the target DNA sequence is located between nucleotide at position 1056 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

3. The composition according to claim 1 or 2, wherein the target DNA sequence is located between nucleotide at position 1060 and nucleotide at position 1079, between nucleotide at position 1149 and nucleotide at position 1612, between nucleotide at position 1693 and nucleotide at position 1852, and/or between nucleotide at position 2863 and nucleotide at position 2882 of the HBV gene.

4. The composition according to any one of claims 1-3, wherein the target DNA sequence is located in one or more of following regions of the type B HBV gene: between nucleotide at position 1149 and nucleotide at position 1190, between nucleotide at position 1210 and nucleotide at position 1310, between nucleotide at position 1350 and nucleotide at position 1400, between nucleotide at position 1420 and nucleotide at position 1450, and between nucleotide at position 1470 and nucleotide at position 1592.

5. The composition according to any one of claims 1-3, wherein the target DNA sequence is located in one or more of following regions of the type C HBV gene: between nucleotide at position 1150 and nucleotide at position 1180, between nucleotide at position 1200 and nucleotide at position 1310, between nucleotide at position 1350 and nucleotide at position 1390, between nucleotide at position 1420 and nucleotide at position 1460, and between nucleotide at position 1480 and nucleotide at position 1593.

6. The composition according to any one of claims 1-3, wherein the target DNA sequence is located in one or more of following regions of the type D HBV gene: between nucleotide at position 1056 and nucleotide at position 1079, between nucleotide at position 1101 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2882, between nucleotide at position 2911 and nucleotide at position 2930, and between nucleotide at position 3048 and nucleotide at position 3067.

7. The composition according to any one of claims 1-3 and 6, wherein the target DNA sequence is located in one or more of following regions of the type D HBV gene: between nucleotide at position 1060 and nucleotide at position 1079, between nucleotide at position 1160 and nucleotide at position 1310, between nucleotide at position 1253 and nucleotide at position 1284, between nucleotide at position 1370 and nucleotide at position 1470, between nucleotide at position 1490 and nucleotide at position 1612, between nucleotide at position 1693 and nucleotide at position 1852, and between nucleotide at position 2863 and nucleotide at position 2882.

8. The composition according to any one of claims 1-7, wherein the sgRNA comprises a nucleotide sequence as set forth in any one of SEQ ID NOs: 4-1165.

9. The composition according to any one of claims 1-7, wherein the sgRNA comprises a partial sequence of the nucleotide sequence as set forth in any one of SEQ ID NOs: 4-1165, and the partial sequence has a length of 15-20 base pairs.

10. The composition according to any one of claims 1-9, wherein the at least one DNA binding protein is a CRISPR enzyme, a zinc finger nuclease (ZNF), a transcription activator-like effector nuclease (TALEN), a homing endonuclease, a dCas9-FokI nuclease, or a MegaTal nuclease.

11. The composition according to claim 10, wherein the CRISPR enzyme is a class 2 Cas protein and/or a mutant thereof.

12. The composition according to claim 10 or 11, wherein the CRISPR enzyme is one or more of the following Cas proteins: class 2 type II-A Cas protein, class 2 type II-B Cas protein, class 2 type II-C Cas protein, class 2 type V-A Cas protein, class 2 type V-B Cas protein, class 2 type V-C Cas protein, class 2 type V-U Cas protein, and mutants of the above.

13. The composition according to any one of claims 10-12, wherein the CRISPR enzyme is a Cas9 protein and/or a mutant thereof.

14. The composition according to any one of claims 1-13, wherein the at least one DNA binding protein is dCas9.

15. The composition according to claim 14, wherein the dCas9 comprises Staphylococcus aureus dCas9, Streptococcus pyogenes dCas9, Campylobacter jejuni dCas9, Corynebacterium diphtheria dCas9, Eubacterium ventriosum dCas9, Streptococcus pasteurianus dCas9, Lactobacillus farciminis dCas9, Sphaerochaeta globus dCas9, Azospirillum (e.g., strain B510) dCas9, Gluconacetobacter diazotrophicus dCas9, Neisseria cinerea dCas9, Roseburia intestinalis dCas9, Parvibaculum lavamentivorans dCas9, Nitratifractor salsuginis (e.g., strain DSM 16511) dCas9, Campylobacter lari (e.g., strain CF89-12) dCas9, Streptococcus thermophilus (e.g., strain LMD-9) dCas9.

16. The composition according to claim 14 or 15, wherein the dCas9 comprises an amino acid sequence as set forth in SEQ ID NOs: 1170-1187.

17. The composition according to any one of claims 1-16, wherein the at least one gene expression modulator provides a modification of at least one nucleotide in the vicinity of the HBV gene and/or within the HBV gene regulatory element.

18. The composition according to any one of claims 1-17, wherein the at least one gene expression modulator comprises one or more selected from a DNA methyltransferase, a DNA hydroxymethylase, a DNA demethylase, a histone methyltransferase, a histone demethylase, a histone acetyltransferase, a histone deacetylase, a phosphatase, a kinase, a transcriptional activator, a transcriptional repressor, a portion of the above, and any combination of the above.

19. The composition according to claim 17, wherein the modification of the at least one nucleotide is DNA methylation.

20. The composition according to any one of claims 1-19, wherein the at least one gene expression modulator comprises one or more selected from a DNA methyltransferase (DNMT), a zinc finger protein-based transcription factor, a portion of the above, and any combination of the above.

21. The composition according to any one of claims 1-20, wherein the at least one gene expression modulator comprises a DNA methyltransferase (DNMT) or a portion thereof, and a zinc finger protein-based transcription factor or a portion thereof.

22. The composition according to claims 18, 20 and 21, wherein the DNA methyltransferase is DNMT3A, DNMT3B, DNMT3L, DNMT1 or DNMT2.

23. The composition according to claim 22, wherein the DNMT3A comprises an amino acid sequence as set forth in SEQ ID NO: 1166 and the DNMT3L comprises an amino acid sequence as set forth in SEQ ID NO: 1167 or 1195.

24. The composition according to claim 20 or 21, wherein the zinc finger protein-based transcription factor is a Krüppel-associated inhibitor (KRAB) or a KRAB domain derived from ZIM3 (ZIM3 KRAB).

25. The composition according to claim 24, wherein the zinc finger protein-based transcription factor comprises an amino acid sequence as set forth in SEQ ID NO: 1168 or 1196.

26. The composition according to any one of claims 18 and 20-25, wherein the DNA methyltransferase is selected from DNMT3A and DNMT3L and a combination thereof, and the zinc finger protein-based transcription factor is KRAB or ZIM3 KRAB.

27. The composition according to any one of claims 1-26, wherein the fusion molecule comprises the at least one gene expression modulator fused to the C-terminus, the N-terminus or both termini of the at least one DNA binding protein.

28. The composition according to any one of claims 1-27, wherein the at least one gene expression modulator is directly fused to the at least one DNA binding protein.

29. The composition according to any one of claims 1-27, wherein the at least one gene expression modulator is indirectly fused to the at least one DNA binding protein via a non-regulatory agent, a second modulator or a linker.

30. The composition according to any one of claims 1-29, wherein the fusion molecule comprises domains DNMT3A-DNMT3L-dCas9-KRAB or domains DNMT3A-DNMT3L-ZIM3 KRAB-dCas9, wherein "-" means that the individual domains of the fusion molecule are directly and/or indirectly linked, and the individual domains are linked in order from N-terminus to C-terminus.

31. The composition according to any one of claims 1-30, wherein the fusion molecule comprises an amino acid sequence as set forth in SEQ ID NO: 1169 or 1194.

32. The composition according to any one of claims 1-31, wherein the fusion molecule further comprises at least one nuclear localization sequence (NLS).

33. The composition according to claim 32, wherein the at least one nuclear localization sequence is directly or indirectly fused to the C-terminus, the N-terminus or both termini of the at least one DNA binding protein.

34. The composition according to any one of claims 1-33, wherein the nucleic acid sequence encoding the fusion molecule is deoxyribonucleic acid (DNA) or messenger ribonucleic acid (mRNA).

35. The composition according to any one of claims 1-34, wherein the fusion molecule is packaged in liposomes or lipid nanoparticles.

36. The composition according to any one of claims 1-35, wherein the fusion molecule and the sgRNA are packaged in liposomes or lipid nanoparticles.

37. The composition according to any one of claims 1-36, wherein the fusion molecule and the sgRNA are packaged in the same liposome or lipid nanoparticle, or in different liposomes or lipid nanoparticles.

38. The composition according to any one of claims 1-37, wherein the liposome or the lipid nanoparticle comprises an ionizable lipid (20%-70%, molar ratio), a PEGylated lipid (0%-30%, molar ratio), a supporting lipid (30%-50%, molar ratio), and cholesterol (10%-50%, molar ratio).

39. The composition according to claim 38, wherein the ionizable lipid is selected from a pH-responsive ionizable lipid, a thermo-responsive ionizable lipid and a photo-responsive ionizable lipid.

40. The composition according to any one of claims 1-34, wherein the fusion molecule is packaged in AAV vectors.

41. The composition according to any one of claims 1-34 and 40, wherein the fusion molecule and the sgRNA are packaged in AAV vectors.

42. The composition according to any one of claims 1-34, 40 and 41, wherein the fusion molecule and the sgRNA are packaged in the same AAV vector or different AAV vectors.

43. The composition according to any one of claims 1-42, wherein the composition is a pharmaceutical composition comprising a pharmaceutically acceptable carrier.

44. A single guide RNA (sgRNA), wherein the sgRNA comprises a sequence complementary to a target DNA sequence, and the target DNA sequence is located in the vicinity of a hepatitis B virus (HBV) gene and/or within an HBV gene regulatory element; the HBV gene is a type B HBV gene comprising a nucleotide sequence as set forth in SEQ ID NO: 1, a type C HBV gene comprising a nucleotide sequence as set forth in SEQ ID NO: 2, or a type D HBV gene comprising a nucleotide sequence as set forth in SEQ ID NO: 3; the HBV gene regulatory element comprises a transcription initiation site, a core promoter, a promoter, an enhancer, a silencer, an insulator element, a boundary element, and/or a locus control region, and the target DNA sequence is located between nucleotide at position 1056 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

45. The sgRNA according to claim 44, wherein the target DNA sequence is located between nucleotide at position 1056 and nucleotide at position 1900, between nucleotide at position 1972 and nucleotide at position 2082, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2930, and/or between nucleotide at position 3048 and nucleotide at position 3067 of the HBV gene.

46. The sgRNA according to claim 44 or 45, wherein the target DNA sequence is located between nucleotide at position 1060 and nucleotide at position 1079, between nucleotide at position 1149 and nucleotide at position 1612, between nucleotide at position 1693 and nucleotide at position 1852, and/or between nucleotide at position 2863 and nucleotide at position 2882 of the HBV gene.

47. The sgRNA according to any one of claims 44-46, wherein the target DNA sequence is located in one or more of following regions of the type B HBV gene: between nucleotide at position 1149 and nucleotide at position 1190, between nucleotide at position 1210 and nucleotide at position 1310, between nucleotide at position 1350 and nucleotide at position 1400, between nucleotide at position 1420 and nucleotide at position 1450, and between nucleotide at position 1470 and nucleotide at position 1592.

48. The sgRNA according to any one of claims 44-46, wherein the target DNA sequence is located in one or more of following regions of the type C HBV gene: between nucleotide at position 1150 and nucleotide at position 1180, between nucleotide at position 1200 and nucleotide at position 1310, between nucleotide at position 1350 and nucleotide at position 1390, between nucleotide at position 1420 and nucleotide at position 1460, and between nucleotide at position 1480 and nucleotide at position 1593.

49. The composition according to any one of claims 44-46, wherein the target DNA sequence is located in one or more of following regions of the type D HBV gene: between nucleotide at position 1056 and nucleotide at position 1079, between nucleotide at position 1101 and nucleotide at position 1900, between nucleotide at position 1972 and nucleotide at position 2085, between nucleotide at position 2134 and nucleotide at position 2264, between nucleotide at position 2335 and nucleotide at position 2354, between nucleotide at position 2639 and nucleotide at position 2658, between nucleotide at position 2863 and nucleotide at position 2882, between nucleotide at position 2911 and nucleotide at position 2930, and between nucleotide at position 3048 and nucleotide at position 3067.

50. The sgRNA according to any one of claims 44-46 and 49, wherein the target DNA sequence is located in one or more of following regions of the type D HBV gene: between nucleotide at position 1060 and nucleotide at position 1079, between nucleotide at position 1160 and nucleotide at position 1310, between nucleotide at position 1253 and nucleotide at position 1284, between nucleotide at position 1370 and nucleotide at position 1470, between nucleotide at position 1490 and nucleotide at position 1612, between nucleotide at position 1693 and nucleotide at position 1852, and between nucleotide at position 2863 and nucleotide at position 2882.

51. The sgRNA according to any one of claims 44-50, wherein the sgRNA comprises a nucleotide sequence as set forth in any one of SEQ ID NOs: 4-1165.

52. The sgRNA according to any one of claims 44-50, wherein the sgRNA comprises a partial sequence of a nucleotide sequence as set forth in any one of SEQ ID NOs: 4-1165, and the partial sequence has a length of 15 to 20 base pairs.

53. A nucleic acid molecule encoding the sgRNA according to any one of claims 44-52.

54. A method for reducing or eliminating an expression of a hepatitis B virus (HBV) gene product in a cell, wherein the method comprises a step of introducing the composition according to any one of claims 1-43 into the cell, thereby reducing or eliminating the expression of the HBV gene product in the cell.

55. A method for reducing or eliminating an expression of a hepatitis B virus (HBV) gene product in a subject in vivo, wherein the method comprises a step of introducing the composition according to any one of claims 1-43 into a cell of the subject, thereby reducing or eliminating the expression of the HBV gene product in the subject.

56. A method for treating a hepatitis B virus (HBV) infection-related disease in a subject or alleviating a symptom of a HBV infection-related disease in a subject, wherein the method comprises a step of introducing an effective amount of the composition according to any one of claims 1-43 into a cell of the subject.

57. The method according to claim 55 or 56, wherein the subject is a mammal, such as a human, a monkey, a mouse, a rat, a rabbit, a pig, a horse, a cat, and a dog.

58. The method according to any one of claims 55-57, wherein the method comprises administering the composition to the subject once or more than once.

59. The method according to any one of claims 55-58, wherein the method comprises administering the composition to the subject at least twice.

60. The method according to any one of claims 55-59, wherein an interval between administering the composition more than once or administering the composition at least twice is 1 day, 2 days, 3 days, 4 days, 5 days, 6 days, 7 days, 8 days, 9 days, 10 days, 11 days, 12 days, 13 days, 14 days or 15 days.

61. The method according to claim 56, wherein the HBV infection-related disease comprises hepatitis, cirrhosis, liver fibrosis, and hepatocellular carcinoma caused by HBV infection.

62. The composition according to any one of claims 1-43 for use in treating a hepatitis B virus (HBV) infection-related disease in a subject or alleviating a symptom of an HBV infection-related disease in a subject.

63. A kit, wherein the kit comprises the composition according to any one of claims 1-43 and a container for placing the composition and/or an instruction for use.
